# EUROPEAN PATENT APPLICATION

(11) **EP 1 227 152 A1**
(43) Date of publication of application: **31.07.2002**
(21) Application number: 01102050.0
(22) Date of filing: 30.01.2001
(51) Int. Cl.: C12N 15/31, C12N 1/20, C07K 14/195, C07K 16/12, C12Q 1/68, A61K 39/02, G01N 33/53, G06F 19/00, C12R 1/01

(54) **Bacterial strain and genome of bifidobacterium**

(71) Applicant: Société des Produits Nestlé S.A., 1800 Vevey (CH)
(72) Inventor: The designation of the inventor has not yet been filed
(74) Representative: Becker Kurig Straus

(57) **Abstract**

The present invention pertains to a novel microorganism of the genus Bifidobacterium longum, in particular to its genomic sequence and the nucleotide sequences encoding polypeptides of Bifidobacterium NCC2705 (CNCM I-2618), which are secreted or specific or which are involved in the metabolism, in the replication process, and to polypeptides encoded by such sequences as well as to vectors including the said sequences and cells or non-human animals transformed with these nucleotide sequences and vectors, respectively. The invention also relates to transcriptional gene products of the Bifidobacteruim genome and to methods of detecting these nucleic acids or polypeptides. The invention eventually comprises a data carrier comprising the nucleotide sequence and/or polypeptide sequence of NCC2705 and also pertains to food and pharmaceutical compositions containing said microorganism for the prevention and/or treatment of diarrhea brought about by rotaviruses and pathogenic bacteria containg said Bifidobcterium.

## Description

The present invention pertains to a novel microorganism of the genus Bifidobacterium longum, in particular to its genomic and plasmid sequence and nucleotide sequences encoding polypeptides of said Bifidobacterium, to vectors including the said sequences and cells or non-human animals transformed with these nucleotide sequences and vectors, respectively. The invention also relates to transcriptional and translational products of the Bifidobacterium genome and to methods of detecting these nucleic acids or polypeptides, respectively. The invention also relates to a data carrier comprising nucleotide sequences and/or polypeptide sequences of NCC2705. In addition, the present invention pertains to the Bifidobacterium longum strain NCC2705 and also to food and pharmaceutical compositions containg said Bifidobcterium or active components thereof for the prevention and/or treatment of diarrhea brought about by rotaviruses and pathogenic bacteria.

Organisms that produce lactic acid as a major metabolic component have been known since decades. These bacteria are normally found in milk or in milk processing factories, respectively, living or decaying plants but also in the intestine of man and animals. These microorganisms, summarized under the term "lactic acid bacteria", represent a rather inhomogeneous group and comprise the genera Lactococcus, Lactobacillus, Streptococcus, Bifidobacterium, Pediococcus etc..

Lactic acid bacteria have been utilized by mankind as fermenting agents for the preservation of food taking benefit of a low pH and the action of products generated during the fermentative activity thereof to inhibit the growth of spoilage bacteria. In addition, lactic acid bacteria have also been used for preparing a variety of different foodstuff such as cheese, yogurt and other fermented dairy products from milk.

Quite recently lactic acid bacteria have attracted a great deal of attention in that some strains have been found to exhibit valuable properties to man and animals upon ingestion. In particular, specific strains of the genus Lactobacillus or Bifidobacterium have been found to pass the gastro-intestinal tract in a viable and live form without getting destroyed in the upper part thereof, especially by the impact of the low pH prevailing in the stomach and be able to colonize the intestinal mucosa, with their temporary or sustained maintenance in the gut being presumed to bring about numerous positive effects on the health of the living beings. These strains are generically termed probiotics.

EP 0 768 375 discloses such a specific strain of the genus Bifidobacterium, that is capable to become implanted in the intestinal flora and may adhere to intestinal cells. This Bifidobacterium is reported to assist in immunomodulation, being capable to competitively exclude adhesion of pathogenic bacteria to intestinal cells, thus supporting the maintenance of the individual's health.

In view of the valuable properties particular strains of lactic acid bacteria may provide, there is a desire in the art for additional lactic acid bacterial strains that are beneficial to the well being of man and/or animal. In addition, a more detailed information is desired relating to the biology of these strains, in particular as regards the interaction with the hosts, the phenomena of passing different environmental conditions in the gut as well as having the capability to adhere to the intestine's mucosa and eventually the involvement in the enhancement of the immune system and defense against pathogens, which information will allow a better understanding of these mechanisms.

Consequently, a problem of the present invention is to provide substantial data about bacterial strains that exhibit properties beneficial for man and/or animals.

In view of said problem, a subject of the present invention is the nucleotide sequence having the sequence SEQ. ID. No. 1 of the lactic acid bacterium Bifidobacterium longum NCC2705 genome and/or the nucleotide sequence SEQ ID. No 1099 of the plasmid contained tehrein. The invention is, however, not limited to the sequences indicated in SEQ. ID. No. 1 and SEQ ID. NO. 1099, respectively, but encompasses genomes and nucleotides encoding polypeptides of strain variants, polymorphisms, allelic variants, and mutants thereof.

In the figures:
Fig. 1 shows a graph, indicating the capability of Bifidobacterium longum NCC 2705 to adhere to human intestinal cells in culture; as a comparison another Bifidobacterium strain BL28 Ca1 was used;
Fig. 2 shows the pathogen sensitivity of pathogenic bacteria towards Bifidobacterium longum NCC2705;
Fig. 3 shows the activity of the cell lines NCC2705 and B1 28 against S. typhimurium SL1344 infecting Caco-2 cells;
Fig. 4 shows the rate of survival of mice infected with Salmonella typhimurium SL 1344 and treated with the Bifidobacterium NCC2705.

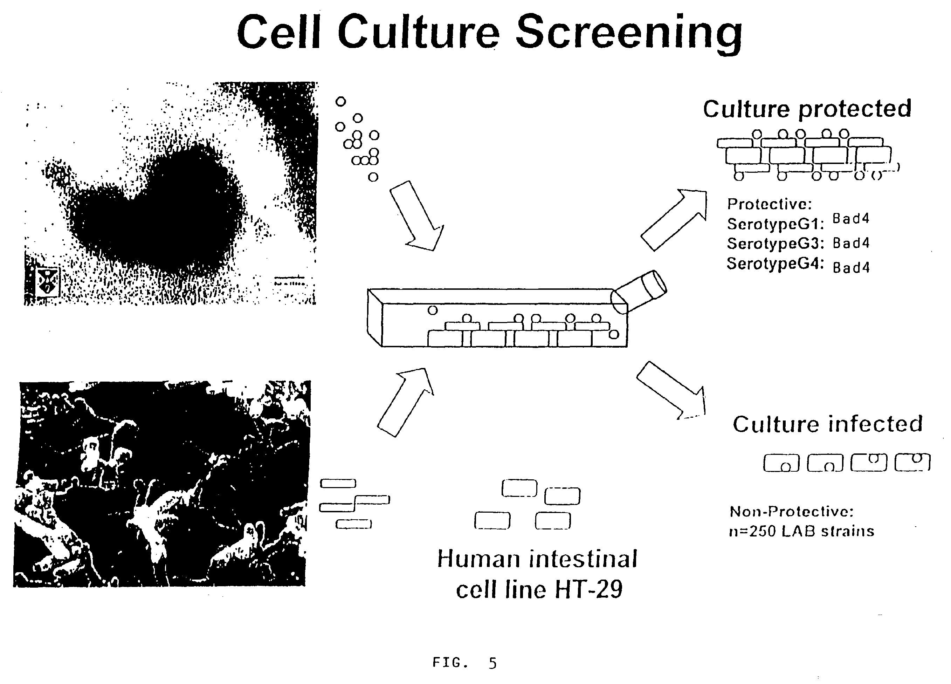

Fig. 5 shows a scheme illustrating the cell culture screening for assessing rotaviral protective properties of the bacterial strain NCC 2705.

The present invention is based on whole-genome sequencing of the genome of the Bifidobacterium longum strain NCC 2705, that has been deposited with the Institute Pasteur according to the Budapest Treaty on January 29^{th}, 2001 receiving the deposit no. CNCM I- I-2618.

In a first aspect the present invention relates to nucleotide sequences selected from the group comprising (a) the nucleotide sequence of SEQ. ID. No. 1; (b) a nucleotide sequence exhibiting at least 90% identity with the sequence of SEQ. ID. No. 1; or (c) a nucleotide sequence that is homologous or hybridizes to SEQ ID. No. 1 under stringent conditions.

In another aspect the invention relates to nucleotide sequences selected from the group comprising (a) the nucleotide sequence of SEQ. ID. No. 1099; (b) a nucleotide sequence exhibiting at least 90% identity with the sequence of SEQ. ID. No. 1099; or (c) a nucleotide sequence that is homologous or hybridizes to SEQ ID. No. 1099 under stringent conditions.

The terms genome or genomic sequence shall be understood to mean the sequence of the chromosome of Bifodobacterium longum. The term plasmid shall be understood to designate any extrachromosomal piece of DNA contained in the Bifidobacterium of the present invention. Nucleotide sequence, polynucleotide or nucleic acid are understood to desigante a double-stranded DNA, a single-stranded DNA or transcriptional products of of the said DNAs at various length including oligonuclotides of about 10 to 100 nucleotides in length.

A homologous nucleotide sequence according to the present invention is understood to mean a nucleotide sequence having a percentage identity with the bases of the nucleotide sequence SEQ. ID. No. 1 or SEQ. ID. No. 1099 of at least 80%, preferably 90% and more preferably 95%, 96%, 97%, 98% or 99 %. The said homologous may comprise, e.g., the sequences corresponding to the genomic sequence or to the sequences of its representative fragments of a bacterium belonging to the Bifidobacterium species, preferably the Bifodobacterium longum species, as well as the sequences corresponding to the genomic sequence or to the sequences of its representative fragments of a bacterium belonging to the variants of the species Bifidobacterium. In the present invention, the terms species and genus are mutually interchangeable.

These homologous sequences may thus correspond to variations linked to mutations within the same species or between species and may correspond in particular to truncations, substitutions, deletions and/or additions of at least one nucleotide. The said homologous sequences may also correspond to variations linked to the degeneracy of the genetic code or to a bias in the genetic code which is specific to the family, to the species or to the variant and which are likely to be present in Bifidobacterium.

Protein and/or nucleic acid sequence homologies may be evaluated using any of the variety of sequence comparison algorithms and programs known in the art. Such algorithms and programs include, but are by no means limited to, TBLASTN, BLASTP, FASTA, TFASTA, and CLUSTALW (see e.g. Pearson and Lipman, 1988, Proc. Natl. Acad. Sci. USA 85 (8): 2444-2448; Altschul et al., 1990, J. Mol. Biol. 215 (3) : 403-410; Thompson et al., 1994, Nucleic Acids Res. 22 (2): 4673-4680; Higgins et al., 1996, Methods Enzymol. 266: 383-402; Altschul et al., 1990, J. Mol. Biol. 215 (3): 403 - 410; Altschul et al., 1993, Nature Genetics 3: 266-272).

In a particularly preferred embodiment, protein and nucleic acid sequence homologies are evaluated using the Basic Local Alignment Search Tool ("BLAST") which is well known in the art (supra). In particular, four specific BLAST programs have been used to perform the following task:

| | |
|---|---|
| (1) BLASTP | Compares an amino acid query sequence against a protein sequence database |
| | |
| (2) BLASTN | Compares a nucleotide query sequence against a nucleotide sequence database |
| | |
| (3) BLASTX | Compares a nucleotide query sequence translated in all reading frames against a protein sequence database |
| | |
| (4) TBLASTN | Compares a protein query sequence against a nucleotide sequence database dynamically translated in all reading frames |

Among these representative fragments, those capable of hybridizing under stringent conditions with a nucleotide sequence according to the invention are preferred. Hybridization under stringent conditions means that the temperature and ionic strength conditions are chosen such that they allow hybridization to be maintained between two complementary DNA fragments. Such conditions of high stringency may e.g. be achieved by carrying out the hybridisation at a preferred temperature of 65 °C in the presence of SSC buffer, e.g. 1 x SSC corresponding to 0.15 M NaCl and 0.05 M Na-citrate. The washing steps may be, for example, the following: 2 x SSC, 0.1% SDS at room temperature followed by three washes with 1 x SSC, 0.1% SDS; 0.5 x SSC, 0.1% SDS; 0.1 x SSC, 0.1% SDS at 68 C for 15 minutes.

The nucleotide sequence SEQ. ID. No. 1 and SEQ. ID. No 1099, respectively, has been obtained by sequencing the genome of and the plasmid contained in Bifidobacterium longum NCC2705 by the method of directed sequencing after fluorescent automated sequencing of the inserts of clones and assembling of these sequences of nucleotide fragments (inserts) by means of softwares. To this end, fragments of the genome were created, ligated into suitable vectors for amplification and propagation and the corresponding fragments were sequenced. Overlaps and the final arrangement of the fragments, the nucleotide sequence thereof, were assessed by the aid of appropiate softwares.

The present invention is further directed to nucleic acid molecules comprising open reading frames (ORFs) encoding Bifodobacterium longum proteins. Therefore, according to another aspect the present invention relates to a polynucleotide having a nucleotide sequence of an open reading frame (ORF) of a Bifidobacterium longum genome comprising, (a) a nucleotide sequence chosen from any one of Seq ID. No. 2 to SEQ. ID. NO. 1098; or (b) a nucleotide sequence exhibiting at least 95 %, preferably 96 %, 97 % 98 % and most preferably 99 % identity with any one of SEQ ID. NO. 2 to SEQ. ID. NO. 1098; or (c) a polynucleotide which hybridizes to any one of SEQ. ID. NO. 2 SEQ. ID. NO. 1098 under conditions of high stringency.

These nucleic acid molecules may be obtained, by e.g. specific amplification of the corresponding sequence using the polymerase chain reaction. Due to the sequence information provided herein the skilled person may design and synthesize any suitable primer nucleotide arid amplify a fragment of interest using the polymerase chain reaction. Therefore, the present invention also comprises nucleotide sequences selected from sequence SEQ. ID. NO. 1 which can be used as a primer for the amplification of nucleic acid sequences. Other techniques for amplifying the target nucleic acid may of course be also be used, such as e.g. the TAS (Transcription-based Amplification System) technique, the 3SR (Self-Sustained Sequence Replication) technique, the NASBA (Nucleic Acid Sequence Based Amplification) technique, the SDA (Strand Displacement Amplification) technique or the TMA (Transcription Mediated Amplification) technique etc..

The (poly)nucleotides of the invention may also be used as probes and techniques for amplifying or modifying a nucleic acid serving as a probe, such as e.g the LCR (Ligase Chain Reaction) technique, the RCR (Repair Chain Reaction) technique, the CPR (Cycling Probe Reaction) technique or the Q-beta-replicase amplification technique may well be applied.

The present invention, therefore, envisages both hybridization (detection) probes and primers for detecting a nucleoide sequence (target nucleotide) of the present invention. In the case of the target being a RNA molecule, e.g. a mRNA, said mRNA may be directly detected or transformed to a cDNA prior to detection.

Alternatively, in order to obtain fragments of the nucleic acid represented by SEQ. ID. NO.1 the Bifodobacterium longum genomic DNA may be subjected to digestion with selected restriction enzymes, with the fargments being separated by e.g. electrophoresis or another suitable separation technique. Such techniques are well known in the art and are inter alia disclosed in Sambrook et al. A Laboratory Manual, Cold Spring Harbor, 1992. Such fragments may easily be obtained by isolating the genomic DNA of Bifodobacterium longum NCC2705 (CNCM I-2618) and performing the necessary steps.

In an alternative form the nucleic acids may also be obtained by chemical synthesis when they are not too large in size according to methods well known to a person skilled in the art.

Modified nucleotide sequences shall be understood to mean any nucleotide sequence obtained by mutagenesis according to techniques well known to a skilled person and exhibiting modifications in relation to the normal sequences, for example mutations in the regulatory and/or promoter sequences for the expression of a polypeptide, in particular leading to a modification of the level of expression of the said polypeptide or to a modulation of the replicative cycle. Modified nucleotide sequence will also be understood to mean any nucleotide sequence encoding a modified polypeptide as defined below.

The subject of the present invention also includes Bifidobacterium longum nucleotide sequences characterized in that they are selected from a nucleotide sequence having an open reading frame as identified by SEQ. ID. NO. 2 to SEQ. ID. NO. 1098, shown in the table I below.

**Table I**

| Seq.ID | Code | Start | Stop | Function | Best Blast Hit ID | E-val |
|---|---|---|---|---|---|---|
| | | | | | | |
| SEQ. ID. No.2 | ORF3 | 785 | 2455 | hsp60 heat shock protein | TREMBLNEW.AF240579_1 | 0 |
| SEQ.ID.No.3 | ORF7 | 3674 | 4447 | phosphate response regulator protein phoP | PIR:H70705 | 7,00E-71 |
| SEQ.ID.No.4 | ORF10 | 4622 | 6469 | two-component sensor histidine kinase | PIR:A70706 | 7,00E-51 |
| SEQ.ID.No.5 | ORF13 | 6542 | 6928 | cold shock protein | PIR:G70816 | 1,00E-25 |
| SEQ. ID. No.6 | ORF14 | 6940 | 8124 | Unknown | PIR:B70780 | 1,00E-35 |
| SEQ. ID. No.7 | ORF23 | 9354 | 11960 | ATP-binding proteinase | PIR:T36384 | 0 |
| SEQ. ID. No.8 | ORF24 | 13462 | 12146 | cytosine deaminase | TREMBLNEW:AE004481_2 | 9,00E-95 |
| SEQ.ID.No.9 | ORF28 | 15297 | 16670 | membrane transport protein | TREMBLNEW:SC2G58_25 | 6,00E-88 |
| SEQ. ID. No.10 | ORF29 | 16759 | 17514 | Creatinase | PIR:T44251 | 2,00E-60 |
| SEQ.ID.No.11 | ORF31 | 17689 | 19479 | 5'-nucleotidase | PIR:F75511 | 4,00E-20 |
| SEQ. ID. No.12 | ORF36 | 21148 | 19691 | Unknown | PIR:B70506 | 2,00E-44 |
| SEQ. ID. No.13 | ORF38 | 21248 | 22645 | histidyl-tRNA synthetase | PIRNEW:A82586 | 4,00E-68 |
| SEQ. ID. No.14 | ORF42 | 22594 | 24480 | Aspartate--tRNA ligase | PIR:C70724 | 0 |
| SEQ. ID. No.15 | ORF47 | 26476 | 27288 | glutamate uptake system ATP-binding protein - | PIR:T35147 | 1,00E-102 |
| SEQ. ID. No.16 | ORF48 | 27362 | 28159 | glutamate-binding periplasmic protein | PIR:T35146 | 5,00E-72 |
| SEQ. ID. No.17 | ORF49 | 28162 | 28836 | glutamate transport protein gluC | PIR:T35145 | 2,00E-47 |
| SEQ. ID. No.18 | ORF50 | 28845 | 29942 | glutamate transport protein gluD | PIR:T35144 | 5,00E-40 |
| SEQ.ID.No.19 | ORF51 | 30034 | 31572 | acid phosphatase | PIR:T28698 | 3,00E-50 |
| SEQ. ID. No.20 | ORF57 | 32653 | 33576 | Unknown | PIR:D75557 | 6,00E-46 |
| SEQ.ID.No.21 | ORF60 | 33713 | 36277 | helicase-like protein | PIR:T17732 | 5,00E-42 |
| SEQ.ID.No.22 | ORF64 | 36363 | 37739 | ATP/GTP binding protein | TREMBLNEW:SC9C5_30 | 1,00E-119 |
| SEQ.ID.No.23 | ORF65 | 37800 | 38519 | sensory response regulator | PIR:H70592 | 5,00E49 |
| SEQ.ID.No.24 | ORF67 | 38519 | 40213 | two-component sensory histidine kinase | PIR:G70592 | 1,00E-68 |
| SEQ.ID.No.25 | ORF74 | 42259 | 43239 | Periplasmic sugar-binding protein | PIR:S56453 | 2,00E-31 |
| SEQ.ID.No.26 | ORF75 | 43383 | 44921 | nbose ABC transporter, ATP-binding protein | PIRNEW:A82497 | 1,00E-100 |
| SEQ. ID. No.27 | ORF76 | 44926 | 45993 | carbohydrate transport protein | PIR:S56456 | 2,00E-44 |
| SEQ.ID.No.28 | ORF78 | 45993 | 47012 | high affinity sugar transport protein rbsC | PIR:S56457 | 4,00E-30 |
| SEQ. ID. No.29 | ORF79 | 48695 | 47241 | Permease | TREMBL:OOE250422_3 | 1,00E-54 |
| SEQ. ID. No.30 | ORF83 | 49084 | 49977 | 2-hydroxyacid dehydrogenase family | PIRNEW:T50667 | 2,00E-30 |
| SEQ. ID. No.31 | ORF90 | 54950 | 52521 | methyl coenzyme M reductase system, component A2 | PIR:D69159 | 7,00E-56 |
| SEQ. ID. No.32 | ORF99 | 58071 | 58835 | polyphosphate glucokinase | PIR:E75472 | 4,00E-53 |
| SEQ. ID. No.33 | ORF101 | 59063 | 60319 | aminotransferase | PIR:T37220 | 4,00E-22 |
| SEQ.ID.No.34 | ORF103 | 60331 | 61152 | ABC transporter, ATP-binding protein | PIR:B75270 | 3,00E-23 |
| SEQ. ID. No.35 | ORF113 | 67189 | 69948 | DNA ligase | PIR:T35810 | 1,00E-131 |
| SEQ.ID.No.36 | ORF114 | 70127 | 71239 | Unknovn (ATP-binding protein) | TREMBLNEW:SCP8_15 | 2,00E-79 |
| SEQ. ID. No.37 | ORF119 | 72964 | 73776 | sugar transport protein | TREMBL:SCE59_3 | 1,00E-45 |
| SEQ. ID. No.38 | ORF129 | 77028 | 77984 | 3-hydroxyacyl-CoA dehydrogenase | PIR:A69252 | 2,00E-41 |
| SEQ. ID. No.39 | ORF131 | 78166 | 79272 | Unknown | TREMBL:SCF41_25 | 3,00E-43 |
| SEQ.ID.No.40 | ORF137 | 80448 | 81182 | Unknown | PIR:A69760 | 3,00E-25 |
| SEQ. ID. No.41 | ORF139 | 82012 | 81215 | Unknown | PIR:S76205 | 2,00E-36 |
| SEQ.ID.No.42 | ORF141 | 82797 | 82012 | ABC-type transport system ATP-binding protein | PIRNEW:F82198 | 5,00E-20 |
| SEQ. ID. No.43 | ORF145 | 82902 | 83465 | translation elongation factor | PIR:B70658 | 4,00E-67 |
| SEQ.ID.No.44 | ORF146 | 83523 | 84092 | transcription termination protein | PIR:A70658 | 5,00E-21 |
| SEQ. ID. No.45 | ORF147 | 83803 | 85506 | carbamoyl-phosphate synthetase | PIR:D70959 | 1,00E-120 |
| SEQ.ID.No.46 | ORF148 | 85511 | 88891 | Carbamoyl-phosphate synthase large chain - | PIR:A70990 | 0 |
| SEQ. ID. No.47 | ORF149 | 88894 | 89811 | orotidine-5'-phosphate decarboxylase | PIR:B49930 | 6,00E-40 |
| SEQ.ID.No.48 | ORF151 | 89994 | 90581 | guanylate kinase | PIR:C70899 | 6,00E-44 |
| SEQ. ID. No.49 | ORF152 | 91293 | 90589 | DNA polymerase III | PIR:C72360 | 4,00E-22 |
| SEQ. ID. No.50 | ORF156 | 91440 | 93692 | translation elongation factor EF-G homolog | TREMBL:BFTETAQ3_2 | 3,00E-64 |
| SEQ. ID. No.51 | ORF160 | 94496 | 95239 | amino acid ABC transporter, permease protein | PIR:D71849 | 4,00E-53 |
| SEQ. ID. No.52 | ORF162 | 95229 | 95900 | amino-acid ABC transporter integral membrane protein | PIR:G81391 | 5,00E-49 |
| SEQ. ID. No.53 | ORF165 | 95896 | 96684 | amino acid ABC transporter, ATP-binding protein | PIR:F71849 | 7,00E-64 |
| SEQ. ID. No.54 | ORF166 | 96753 | 97652 | amino acid binding protein | TREMBL:U60994_4 | 1,00E-72 |
| SEQ. ID. No.55 | ORF168 | 97891 | 99126 | cystathionine beta-lyase | PIRNEW:T47232 | 8,00E-95 |
| SEQ. ID. No.56 | ORF170 | 101042 | 99774 | transposase | TREMBL:REU010061_1 | 4,00E-78 |
| SEQ. ID. No.57 | ORF176 | 102563 | 103459 | Unknown | TREMBL:AC008261_3 | 2,00E-46 |
| SEQ. ID. No.58 | ORF180 | 105297 | 104506 | glutamate racemase | PIR:C69978 | 3,00E-39 |
| SEQ. ID. No.59 | ORF182 | 105456 | 106295 | Diaminopimelate epimerase | PIR:T35113 | 5,00E-30 |
| SEQ. ID. No.60 | ORF186 | 107179 | 108069 | Unknown | TREMBLNEW:SCP8_24 | 8,00E-37 |
| SEQ. ID. No.61 | ORF191 | 110307 | 111878 | ATP-dependent DNA helicase | TREMBLNEW:SC23B6_12 | 3,00E-82 |
| SEQ. ID. No.62 | ORF194 | 113696 | 111954 | Unknown | TREMBLNEW:SC23B6_23 | 5,00E-35 |
| SEQ.ID.No.63 | ORF197 | 113917 | 114795 | Unklovn | PIR:T36157 | 1,00E42 |
| SEQ.ID.No.64 | ORF199 | 114914 | 115327 | Unknown | PIR:T44716 | 1,00E-24 |
| SEQ.ID.No.65 | ORF202 | 117499 | 118686 | 1-deoxy-D-xylulose 5-phosphate reductoisomerase | PIR:A70923 | 1,00E-103 |
| SEQ.ID.No.66 | ORF203 | 118698 | 119906 | peptidoglycan acetylation | PIR:T35407 | 1,00E-135 |
| SEQ.ID.No.67 | ORF205 | 121792 | 122508 | Unknown | TREMBL:SCC121_13 | 7,00E-47 |
| SEQ.ID.No.68 | ORF206 | 122454 | 123299 | undecaprenyl phosphate synthetase | PIR:H70585 | 5,00E-77 |
| SEQ.ID.No.69 | ORF211 | 125854 | 124787 | Unknown | PIR:C72369 | 1,00E-25 |
| SEQ.ID.No.70 | ORF212 | 126895 | 126116 | ABC transporter, permease protein | PIR:D72369 | 6,00E-26 |
| SEQ. ID. No.71 | ORF214 | 128647 | 127094 | sucrose hydrolase | PIR:S52162 | 3,00E-91 |
| SEQ. ID. No.72 | ORF216 | 130160 | 128661 | sucrose transport protein | PIR:GRECST | 1,00E-92 |
| SEQ. ID. No.73 | ORF217 | 131250 | 130213 | transcription regulator, LacI family | PIR:F72282 | 3,00E-31 |
| SEQ. ID. No.74 | ORF219 | 132691 | 131297 | Serpin | SWISSPROT:NEUS_MOUSE | 5,00E-30 |
| SEQ. ID. No.75 | ORF225 | 135167 | 133791 | membrane transport protein | PIR:G64937 | 3,00E-48 |
| SEQ. ID. No.76 | ORF229 | 137121 | 136123 | phosphomethylpyrimidine kinase | PIR:T35647 | 3,00E-48 |
| SEQ. ID. No.77 | ORF232 | 140189 | 137439 | thiamin biosynthesis protein thiA | PIR:D69722 | 0 |
| SEQ. ID. No.78 | ORF235 | 141216 | 140275 | hydoxyethylthiazole kinase | TREMBLNEW:AP001297_22 | 1,00E-28 |
| SEQ. ID. No.79 | ORF238 | 141652 | 143121 | glycyl-tRNA synthetase | PIR:D70585 | 0 |
| SEQ.ID.No.80 | ORF239 | 143269 | 144510 | transcription regulator | TREMBL:5CC82_3 | 1,00E-100 |
| SEQ.ID.No.81 | ORF240 | 144625 | 145833 | ftsZ protein | PIR:JE0282 | 1,00E-92 |
| SEQ.ID.No.82 | ORF246 | 148841 | 149800 | ribosomal large subunit pseudouridine synthase | PIR:T34943 | 1,00E-67 |
| SEQ. ID. No.83 | ORF254 | 152161 | 155727 | DNA-directed DNA polymerase III alpha chain | PIR:T35093 | 0 |
| SEQ. ID. No.84 | ORF257 | 156459 | 158333 | protoporphyrinogen oxidase | TREMBL:SP09352_2 | 1,00E-175 |
| SEQ. ID. No.85 | ORF290 | 172284 | 171292 | transposase | PIR:T14971 | 5,00E-33 |
| SEQ. ID. No.86 | ORF292 | 173573 | 172074 | transposase | TREMBLNEW:AP001520_222 | 4,00E-52 |
| SEQ. ID. No.87 | ORF297 | 176631 | 174295 | phage infection protein | PIR:E69115 | 6,00E-65 |
| SEQ. ID. No.88 | ORF299 | 179366 | 176631 | phage infection protein | PIR:E69115 | 1,00E-65 |
| SEQ. ID. No.89 | ORF303 | 180792 | 179875 | NADPH-flavin oxidoreductase | PIR:S39698 | 1,00E-21 |
| SEQ. ID. No.90 | ORF305 | 181144 | 181788 | Unknown | TREMBL:SC9G1_8 | 3,00E-24 |
| SEQ. ID. No.91 | ORF307 | 182199 | 183524 | Sugar-binding protein | TREMBL:CTPULSA _3 | 2,00E-35 |
| SEQ. ID. No.92 | ORF310 | 184801 | 183788 | Transcriptional regulators of the LacI family | TREMBL:SCF43_17 | 2,00E-30 |
| SEQ. ID. No.93 | ORF311 | 185082 | 186035 | sugar transport system pemxase | PIR:S37704 | 2,00E-47 |
| SEQ. ID. No.94 | ORF313 | 186038 | 186877 | ABC transporter sugar permease | PIR:S37705 | 3,00E-50 |
| SEQ. ID. No.95 | ORF318 | 187883 | 190756 | arabinosidase | TREMBL:BF55187_1 | 4,00E-41 |
| SEQ. ID. No.96 | ORF323 | 192387 | 191209 | transposase | TREMBLNEW:TFU66426_1 | 1,00E-64 |
| SEQ. ID. No.97 | ORF327 | 192648 | 194147 | transposase | TREMBLNEW:AP001520_222 | 3,00E-53 |
| SEQ. ID. No.98 | ORF330 | 193937 | 194929 | probable transposase | PIR:T14971 | 5,00E-33 |
| SEQ. ID. No.99 | ORF333 | 195262 | 196671 | Unknown | PIR:C71089 | 4,00E-76 |
| SEQ. ID. No.100 | ORF334 | 198212 | 196740 | aromatic amino acid transport protein | PIR:QRECAA | 4,00E-96 |
| SEQ. ID. No. 101 | ORF335 | 199003 | 198437 | transcriptional regulator (TetR/AcrR family) | TREMBLNEW:AP001509_157 | 2,000-23 |
| SEQ. ID. No. 102 | ORF337 | 199147 | 199845 | ABC transporter, ATP-binding protein | PIR:B69477 | 7,00E-61 |
| SEQ. ID. No.103 | ORF345 | 203871 | 205394 | amino acid permease | PIR:B69580 | 2,00E-82 |
| SEQ. ID. No.104 | ORF348 | 205839 | 210554 | Unknown | TREMBL:SC6D11_2 | 3,00E-88 |
| SEQ. ID. No.105 | ORF351 | 210769 | 214602 | Unknown | TREMBLNEW:AB013390_9 | 5,00E-38 |
| SEQ. ID. No.106 | ORF358 | 215664 | 215104 | DNA-damage-inducible protein | SWISSPROT:DIND_ECOLI | 1,00E-24 |
| SEQ. ID. No.107 | ORF363 | 216808 | 218676 | ABC transporter (ATP-binding protein) | TREMBL:SC7A8_2 | 1,00E-115 |
| SEQ. ID. No.108 | ORF365 | 218894 | 220720 | ABC transporter | TREMBL:SC7A8_3 | 1,00E-136 |
| SEQ. ID. No.109 | ORF372 | 223915 | 225009 | Unknown | TREMBL:SCE87_4 | 2,00E-63 |
| SEQ. ID. No.110 | ORF377 | 226371 | 228233 | methionine--tRNA ligase | PIR:D71091 | 1,00E-113 |
| SEQ. ID. No.111 | ORF382 | 232054 | 230531 | aminopeptidase | TREMBL:LHPEPC_1 | 4,00E-47 |
| SEQ. ID. No.112 | ORF384 | 232279 | 234504 | Unknown | PIR:F72395 | 1,00E-98 |
| SEQ. ID. No.113 | ORF389 | 236829 | 235675 | lacI-family transcriptional regulator | TREMBLNEW:SCG22_12 | 4,00E-34 |
| SEQ. ID. No.114 | ORF393 | 237203 | 238609 | alpha galactosidase | TREMBLNEW:AP001513_143 | 1,00E-95 |
| SEQ. ID. No.115 | ORF396 | 239413 | 241272 | ABC transporter | TREMBLNEW:AP001511_14 | 1,00E-105 |
| SEQ.ID. No.116 | ORF397 | 241286 | 243091 | ABC transporter (ATP-binding protein) | PIR:S72638 | 1,00E-142 |
| SEQ.ID. No.117 | ORF399 | 243351 | 245819 | alpha-L-arabinofuranosidase I | PIR:T36818 | 1,00E-108 |
| SEQ.ID. No.118 | ORF404 | 247917 | 246175 | arabinan endo-1,5-alpha-L-arabinosidase homolog | PIR:E70076 | 3,00E-44 |
| SEQ. ID. No.119 | ORF406 | 249669 | 248077 | arabinan endo-1,5-alpha-L-arabinosidase | PIR:E70076 | 4,000-60 |
| SEQ. ID. No.120 | ORF413 | 252032 | 253087 | transcription regulator, LacI family | TREMBLNEW:SCG22_12 | 6,00E-66 |
| SEQ. ID. No.121 | ORF419 | 254388 | 255443 | alpha-L-arabinofuranosidase II | TREMBLNEW:AP001513_140 | 1,00E-108 |
| SEQ. ID. No.122 | ORF424 | 257319 | 258269 | ABC transporter sugar permease | PIRNEW:D82557 | 1,00045 |
| SEQ. ID. No.123 | ORF427 | 258269 | 259192 | membrane transport protein | PIR:A56641 | 1,00048 |
| SEQ. ID. No.124 | ORF433 | 261148 | 262104 | Unknown | TREMBL:SCE87_2 | 2,00E-57 |
| SEQ. ID. No.125 | ORF436 | 262540 | 264747 | potassium uptake protein | PIRNEW:F82623 | 4,00E-78 |
| SEQ. ID. No.126 | ORF456 | 274558 | 276960 | Unknown | PIR:T00092 | 2,00E-67 |
| SEQ.ID. No.127 | ORF463 | 279189 | 278236 | glycosyltransferase homolog | TREMBL:AF033015_6 | 4,00E-75 |
| SEQ.ID. No.128 | ORF465 | 282221 | 280941 | polysaccharide ABC transporter | PIR:T00089 | 1,00E-102 |
| SEQ. ID. No.129 | ORF466 | 283047 | 282226 | ABC-type transport protein | PIR:T00088 | 2,00E-59 |
| SEQ. ID. No.130 | ORF469 | 284425 | 284814 | glycerol-3-phosphate cytidylyltransferase | PIR:A49757 | 1,00E-44 |
| SEQ.ID. No.131 | ORF472 | 288030 | 286135 | Unknown | PIR:D70888 | 4.00E-21 |
| SEQ.ID. No.132 | ORF475 | 288829 | 289782 | Glycosyltransferase-type protein | TREMBL:AF033015_6 | 6,00E-76 |
| SEQ. ID. No.133 | ORF476 | 289796 | 291790 | transferase | PIR:D70888 | 2,00E-95 |
| SEQ. ID. No. 134 | ORF477 | 293831 | 291894 | Unknown | PIR:D70888 | 4,00E-95 |
| SEQ. ID. No.135 | ORF479 | 294815 | 294189 | Unknown | PIR:T00092 | 3,00E-22 |
| SEQ. ID. No.136 | ORF482 | 294973 | 295959 | transposase | TREMBLNEW:TFU66426_1 | 7,00E-69 |
| SEQ. ID. No.137 | ORF484 | 296743 | 295985 | probable transposase | PIR:T14971 | 7,00E-37 |
| SEQ. ID. No.138 | ORF489 | 298223 | 297207 | transposase | TREMBLNEW:AP001520_222 | 5,00E-57 |
| SEQ. ID. No.139 | ORF496 | 300685 | 300095 | transposase | TREMBL:AF029727_1 | 1,00E-28 |
| SEQ.ID. No.140 | ORF503 | 304082 | 303180 | glucose-1-phosphate thymidylyltransferase | TREMBL:AB030032_7 | 1,00E-108 |
| SEQ.ID. No.141 | ORF504 | 305615 | 304176 | dTDP-4-keto-6-deoxyglucose-3,5-epimerase | TREMBL:SMD182_5 | 9,00E-58 |
| SEQ.ID. No.142 | ORF506 | 306672 | 305632 | dTDP-glucose-4,6-dehydratase | TREMBL:AF105113_5 | 1,00E-124 |
| SEQ.ID. No.143 | ORF508 | 308254 | 306818 | polysaccharide biosynthesis (cps-like) | TREMBL:A76918_12 | 2,00E-58 |
| SEQ.ID. No.144 | ORF509 | 310121 | 308379 | 2-succinyl-6-hydroxy-24-cyclohexadiene-1-carboxylate synthase | PIR:G69656 | 5,00E-24 |
| SEQ.ID. No.145 | ORF510 | 310888 | 310151 | dehydrogenase-like | PIR:S39737 | 3,00E-25 |
| SEQ.ID. No.146 | ORF514 | 314448 | 313510 | glycosyltransferase | TREMBL:AF026471_9 | 1,00E-105 |
| SEQ.ID. No.147 | ORF515 | 315657 | 314467 | putative rhamnosyl transferase | TREMBL:AF026471_8 | 1,00E-138 |
| SEQ.ID. No.148 | ORF516 | 317259 | 315718 | glycosyl transferase | TREMBL:SCF62_7 | 6,00E-47 |
| SEQ.ID. No.149 | ORF529 | 321876 | 321079 | transposase | PIR:B60340 | 5,00E-25 |
| SEQ. ID. No.150 | ORF542 | 326223 | 325735 | transposase | PIR:B60340 | 7,00E-24 |
| SEQ. ID. No.151 | ORF546 | 327618 | 329354 | Undecaprenyl-phosphate glucose 1-phosphate transferase | TREMBL:SCF62_7 | 4,00E-59 |
| SEQ. ID. No.152 | ORF553 | 331662 | 333176 | multidrug resistance protein | PIR:F69763 | 2,00E-73 |
| SEQ. ID. No.153 | ORF554 | 334646 | 333282 | transmembrane protein | TREMBL:MBO5699_1 | 1,00E-100 |
| SEQ. ID. No.154 | ORF557 | 336993 | 336313 | Unknown | PIR:A70672 | 7,00E-34 |
| SEQ. ID. No.155 | ORF558 | 337926 | 337060 | oxidoreductase | PIR:T34993 | 4,00E-68 |
| SEQ.ID. No.156 | ORF561 | 341255 | 338565 | arabinogalactanendo-1,4-beta-galactosidase | TREMBLNEW:AP001514_29 | 1,00E-141 |
| SEQ. ID. No.157 | ORF563 | 342506 | 341454 | transcription regulator, LacI family | PIR:F72282 | 2,00E-28 |
| SEQ. ID. No.158 | ORF564 | 344823 | 342553 | beta-galactosidase | TREMBL:SC6D11_3 | 1,00E-164 |
| SEQ.ID. No.159 | ORF566 | 345876 | 344872 | ABC transporter sugar permease | TREMBL:SC6D11_5 | 1,00E-113 |
| SEQ. ID. No.160 | ORF567 | 346841 | 345882 | ABC transporter sugar permease | TREMBL:SC6D11_6 | 6,00E-80 |
| SEQ. ID. No.161 | ORF570 | 348409 | 347063 | solute-binding lipoprotein | TREMBL:SC6D11_4 | 3,00E-88 |
| SEQ. ID. No.162 | ORF574 | 353922 | 349792 | ABC transporter | TREMBL:HI32749_4 | 6,00E-95 |
| SEQ.ID. No.163 | ORF578 | 354795 | 356615 | long chain fatty acid coA ligase - | PIR:T35513 | 1,00E-127 |
| SEQ.ID. No.164 | ORF581 | 357435 | 356803 | ABC transporter, ATP-binding protein | TREMBL:AF140784_2 | 6,00E-61 |
| SEQ. ID. No.165 | ORF582 | 358671 | 357451 | vexl(vexp1) homolog | TREMBL:AF140784_1 | 2,00E-30 |
| SEQ. ID. No.166 | ORF585 | 360038 | 358671 | ABC transporter | TREMBL:AF140784_3 | 3,00E-56 |
| SEQ. ID. No.167 | ORF591 | 362034 | 362747 | Unknown | PIR:T36850 | 4,00E-26 |
| SEQ. ID. No.168 | ORF593 | 364451 | 362937 | L-arabinose isomerase | TREMBL:AB036736_2 | 1,00E-135 |
| SEQ. ID. No.169 | ORF594 | 365375 | 364686 | L-ribulose-phosphate 4-epimerase | TREMBL:AB036736_1 | 1,00E47 |
| SEQ. ID. No.170 | ORF597 | 367106 | 365463 | xylulose kinase | TREMBL:PSP249910_1 | 2,00E-22 |
| SEQ. ID. No.171 | ORF598 | 368415 | 367264 | Transcriptional regulators of the LacI family | TREMBL:SC6D11_7 | 1,00E-39 |
| SEQ. ID. No.172 | ORF600 | 369342 | 368506 | ribonuclease HII | PIR:T35186 | 4,00E-26 |
| SEQ. ID. No.173 | ORF603 | 370319 | 369465 | signal peptidase I | TREMBL:SLTK24SIP_1 | 2,00E-33 |
| SEQ. ID. No.174 | ORF606 | 373183 | 371486 | Gglucose-6-phosphate isomerase | PIR:H70715 | 0 |
| SEQ. ID. No.175 | ORF611 | 376269 | 374992 | Antibiotic resistance factor | TREMBLNEW:SPN277485_1 | 1,00E-47 |
| SEQ. ID. No.176 | ORF613 | 377629 | 376322 | pepdidoglycan biosynthesis | TREMBL:SSU66883_4 | 2,00E-38 |
| SEQ. ID. No.177 | ORF615 | 379262 | 377706 | beta-lactam resistance factor | TREMBLNEW:SPN277485_1 | 3,00E-59 |
| SEQ. ID. No.178 | ORF620 | 380176 | 379421 | Unknown | PIR:T36125 | 2,00E-46 |
| SEQ.ID. No.179 | ORF621 | 380996 | 380223 | ribonuclease PH | PIR:T36127 | 6,00E-82 |
| SEQ. ID. No.180 | ORF623 | 382637 | 381282 | Unknown | PIR:T36140 | 1,00E-124 |
| SEQ. ID. No.181 | ORF627 | 384144 | 385151 | Ion Channel | TREMBL:LLU60828_1 | 1,00E-105 |
| SEQ. ID. No.182 | ORF634 | 387400 | 387876 | Unknown | PIR:T35654 | 4,00E-23 |
| SEQ. ID. No.183 | ORF636 | 388205 | 389035 | ribonucleas | PIR:T44706 | 2,00E-44 |
| SEQ.ID. No.184 | ORF638 | 389199 | 391163 | Acetolactate synthase | PIR:T35828 | 0 |
| SEQ.ID. No.185 | ORF639 | 391183 | 391734 | acetolactate synthase | PIR:JC7166 | 5,00E-52 |
| SEQ.ID. No.186 | ORF643 | 394929 | 392656 | ABC transporter | PIR:G70817 | 9,00E-91 |
| SEQ. ID. No.187 | ORF644 | 395867 | 394980 | amino transferase | PIR:S75988 | 2,00E-21 |
| SEQ. ID. No.188 | ORF645 | 395810 | 397561 | cysteine-tRNA ligase | PIR:B70607 | 1,00E-110 |
| SEQ. ID. No.189 | ORF647 | 398700 | 400445 | signal recognition particle protein (ffh) | PIR:T34771 | 1,00E-159 |
| SEQ.ID. No.190 | ORF650 | 401867 | 402325 | 305 ribosomal protein S16 | PIR:H70927 | 2,00E-22 |
| SEQ. ID. No.191 | ORF652 | 402605 | 403225 | Unknown | PIR:T34778 | 3,00E-20 |
| SEQ. ID. No.192 | ORF653 | 404403 | 403354 | inosine-uridine preferring nucleoside hydrolase | PIR:B75524 | 2,00E-40 |
| SEQ. ID. No.193 | ORF655 | 405364 | 404504 | oxidoreductase | PIR:B75341 | 2,00E-32 |
| SEQ. ID. No. 194 | ORF661 | 408342 | 407767 | Unknown | TREMBL:LMU66186_1 | 1,00E-36 |
| SEQ. ID. No.195 | ORF663 | 409416 | 408640 | phosphate ABC transporter | PIR:D70810 | 6,00E-98 |
| SEQ. ID. No.196 | ORF664 | 410434 | 409472 | phosphate transport system permease protein A-1 - | PIR:B70584 | 3,00E-49 |
| SEQ.ID. No.197 | ORF666 | 411420 | 410470 | phosphate transport system permease protein | TREMBLNEW:SCD84_8 | 4,00E-56 |
| SEQ. ID. No.198 | ORF669 | 412764 | 411634 | Phosphate-binding protein | TREMBL:ML15182_25 | 3,00E-41 |
| SEQ. ID. No.199 | ORF671 | 413774 | 413007 | two-component response regulator | TREMBL:MBY13627_3 | 3.00E-75 |
| SEQ.ID. No.200 | ORF672 | 415238 | 413910 | two-component sensor histidine kinase | PIR:E70744 | 9,00E-44 |
| SEQ. ID. No.201 | ORF676 | 417556 | 416327 | 2-dehydro-3-deoxyphosphoheptonate aldolase | PIR:140837 | 1,00E-92 |
| SEQ. ID. No.202 | ORF677 | 418818 | 417688 | 2-dehydro-3-deoxyphosphoheptonate aldolase | PIR:ADECHF | 2,00E-96 |
| SEQ. ID. No.203 | ORF678 | 420453 | 419113 | cysteine aminopeptidase | PIR:S52865 | 3,00E-88 |
| SEQ. ID. No.204 | ORF679 | 422145 | 420571 | Unknown | TREMBL:SC8F4_ | 1,00E-21 |
| SEQ. ID. No.205 | ORF681 | 422970 | 422296 | pyroglutamyl-peptidase I | PIR:S23432 | 4,00E-26 |
| SEQ. ID. No.206 | ORF683 | 423855 | 422983 | CDP-ribitol pyrophosphorylase | PIR:S60902 | 9,00E-32 |
| SEQ. ID. No.207 | ORF684 | 424915 | 423938 | Unknown | PIR:T34951 | 2,00E-33 |
| SEQ. ID. No.208 | ORF686 | 426819 | 424996 | protease | TREMBLNEW:AP001511_163 | 2,00E-65 |
| SEQ. ID. No.209 | ORF689 | 426773 | 427525 | tRNA (guanine-N1)-methyltransferase | PIR:E70927 | 8,00E-53 |
| SEQ. ID. No.210 | ORF690 | 428172 | 427555 | methylase | PIR:C70671 | 1,00E-26 |
| SEQ. ID. No.211 | ORF691 | 431233 | 428393 | ATP-dependent DNA helicase | PIR:T35650 | 1,00E-108 |
| SEQ. ID. No.212 | ORF696 | 433135 | 434409 | Unknown | PIR:B70078 | 7,00E-86 |
| SEQ.ID. No.213 | ORF704 | 435955 | 436485 | methylated-DNA-protein-cysteine methyltransferase | PIR:T34664 | 9,00E-21 |
| SEQ.ID. No.214 | ORF709 | 439060 | 440586 | L-aspartase | TREMBL:AF181498_4 | 1,00E-124 |
| SEQ.ID. No.215 | ORF713 | 444121 | 442847 | polyamine ABC-transporter ATP-binding protein | PIR:T35802 | 2,00E-52 |
| SEQ.ID. No.216 | ORF718 | 446161 | 444980 | ABC transport system permease | PIR:E81344 | 2,00E-27 |
| SEQ.ID. No.217 | ORF720 | 447105 | 445942 | Unknown | TREMBLNEW:AE004747_11 | 4,00E-29 |
| SEQ.ID. No.218 | ORF721 | 448644 | 447361 | D-alanine:D-alanine ligase | PIR:B70673 | 6,00E-75 |
| SEQ.ID. No.219 | ORF723 | 449733 | 448735 | glycerol-3-phosphate dehydrogenase | PIR:T35643 | 4,00E-79 |
| SEQ.ID. No.220 | ORF737 | 455903 | 456889 | peptidylprolyl isomerase | TREMBL:SCI41_22 | 2,00E-25 |
| SEQ.ID. No.221 | ORF740 | 457747 | 456941 | Unknown | PIR:E70769 | 1,00E-60 |
| SEQ.ID. No.222 | ORF742 | 459561 | 458092 | H+-transporting ATP synthase beta chain - | PIR:S37547 | 0 |
| SEQ.ID. No.223 | ORF744 | 460493 | 459573 | ATP synthase gamma subunit | TREMBLNEW:SC26G5_16 | 1,00E-62 |
| SEQ.ID. No.224 | ORF747 | 462128 | 460500 | H+-transporting ATP synthase | TREMBLNEW:SC26G5_15 | 0 |
| SEQ.ID. No.225 | ORF748 | 463040 | 462207 | ATP synthase delta chain | TREMBLNEW:SC26G5_14 | 4,00E-24 |
| SEQ.ID. No.226 | ORF751 | 464793 | 463984 | H+-transporting ATP synthase | PIR:S37541 | 1,00E-21 |
| SEQ.ID. No.227 | ORF755 | 466289 | 465258 | homoserine O-succinyltransferase | TREMBLNEW:AP001515_14 | 2,00E-88 |
| SEQ.ID. No.228 | ORF774 | 472562 | 473587 | Bacteriophage portal protein | TREMBL:BPH6589_4 | 1,00E-22 |
| SEQ.ID. No.229 | ORF780 | 477113 | 479179 | Unknown | TREMBL:LLHORF02_9 | 2,00E-27 |
| SEQ.ID. No.230 | ORF793 | 483906 | 483079 | integrase | PIR:B70965 | 2,00E-47 |
| SEQ.ID. No.231 | ORF802 | 486594 | 488831 | alpha-amylase | TREMBL:SC6A11_19 | 1,00E-174 |
| SEQ.ID. No.232 | ORF807 | 489062 | 489553 | inorganic pyrophosphatase | PIR:T36335 | 2,00E48 |
| SEQ. ID. No.233 | ORF809 | 489790 | 490371 | Unknown | PIR:F81434 | 6,00E-27 |
| SEQ. ID. No.234 | ORF812 | 491348 | 492145 | two-component response regulator | PIR:JN0831 | 5,00E-66 |
| SEQ. ID. No.235 | ORF817 | 492207 | 492890 | Endonuclease III | PIR:C70790 | 6,00E-58 |
| SEQ.ID. No.236 | ORF821 | 494520 | 497357 | valine-tRNA ligase | PIR:B71675 | 1,00E-149 |
| SEQ.ID. No.237 | ORF828 | 502513 | 500447 | transcription termination factor Rho | TREMBL:MLRHO_1 | 1,00E-151 |
| SEQ.ID. No.238 | ORF832 | 504332 | 502722 | Unknown | PIR:B71153 | 1,00E-29 |
| SEQ.ID. No.239 | ORF840 | 509762 | 508266 | Glu-tRNA(Gln) amidotransferase chain B | PIR:T35817 | 1,00E-170 |
| SEQ. ID. No.240 | ORF842 | 511329 | 509791 | Glu-tRNA(Gln) amidotransferase chain A - | PIR:T35815 | 1,00E-158 |
| SEQ. ID. No.241 | ORF845 | 512647 | 511802 | Unknown | PIR:G70833 | 6,00E-35 |
| SEQ. ID. No.242 | ORF848 | 514770 | 513370 | Unknown | TREMBL:AF213822_7 | 7,00E-55 |
| SEQ. ID. No.243 | ORF850 | 515115 | 514837 | Unknown | PIR:A70907 | 1,00E-28 |
| SEQ. ID. No.244 | ORF852 | 515227 | 517932 | copper-transporting ATPase | TREMBL:AF129004_1 | 1,00E-113 |
| SEQ. ID. No.245 | ORF853 | 518753 | 518022 | glycerol uptake facilitator protein | PIR:B72254 | 3,00E-47 |
| SEQ. ID. No.246 | ORF855 | 520666 | 518990 | phosphotransferase system enzyme I | TREMBLNEW:AP001517_195 | 1,00E-129 |
| SEQ. ID. No.247 | ORF857 | 521759 | 521316 | ribosomal protein L9 | PIR:T36596 | 2,00E-25 |
| SEQ. ID. No.248 | ORF859 | 522744 | 522091 | single-strand binding protein | PIR:F70913 | 6,00E-39 |
| SEQ. ID. No.249 | ORF861 | 523094 | 522804 | ribosomal protein S6 | PIR:E70913 | 1,00E-21 |
| SEQ. ID. No.250 | ORF864 | 523860 | 524870 | ribose-phosphate pyrophosphokinase | TREMBLNEW:SCE66_2 | 1,00E-104 |
| SEQ. ID. No.251 | ORF865 | 525833 | 524952 | Unknown | PIRNEW:H82646 | 6,00E-29 |
| SEQ. ID. No.252 | ORF868 | 529629 | 525916 | endo-1,4-beta-xylanase | PIR:H69735 | 1,00E-24 |
| SEQ. ID. No.253 | ORF873 | 535559 | 529737 | Unknown | PIR:T17479 | 0 |
| SEQ. ID. No.254 | ORF881 | 538453 | 536261 | Unknown | PIR:F72395 | 1,00E-101 |
| SEQ. ID. No.255 | ORF885 | 539055 | 538240 | Sugar permeases | PIR:T30595 | 1,00E-38 |
| SEQ.ID. No.256 | ORF887 | 540002 | 539079 | sugar transport system permease | PIR:T36087 | 7,00E-30 |
| SEQ.ID. No.257 | ORF892 | 542101 | 543120 | Transcriptional regulators of the LacI family | TREMBL:AF086819_1 | 3,00E-40 |
| SEQ.ID. No.258 | ORF893 | 544958 | 543141 | ABC-type transport protein | PIR:S38903 | 4,00E-79 |
| SEQ.ID. No.259 | ORF897 | 546143 | 545394 | cobyric acid synthase | PIR:T31439 | 2,00E-47 |
| SEQ.ID. No.260 | ORF899 | 547718 | 546249 | UDP-N-acetylmuramyl tripeptide synthetase | PIR:T31440 | 7,00E-28 |
| SEQ. ID. No.261 | ORF904 | 549247 | 547721 | replicative DNA helicase | PIR:T36598 | 1,00E-144 |
| SEQ. ID. No.262 | ORF905 | 549303 | 550895 | DNA-damage-inducible protein f | PIR:T36597 | 5,00E-69 |
| SEQ. ID. No.263 | ORF908 | 552758 | 550935 | protein pII uridylyltransferase | PIR:T34770 | 3,00E-83 |
| SEQ. ID. No.264 | ORF911 | 554501 | 553209 | ammonium transport protein | TREMBL:CAJ10319_2 | 2,00E-90 |
| SEQ. ID. No.265 | ORF914 | 556131 | 554872 | prokaryotic docking protein | PIR:T35664 | 1,00E-88 |
| SEQ. ID. No.266 | ORF915 | 556236 | 557072 | transcription regulator | TREMBLNEW:SCE66_8 | 1,00E-56 |
| SEQ.ID. No.267 | ORF917 | 557099 | 558130 | glycosyl transferase | TREMBL:SPAJ6986_9 | 2,00E-30 |
| SEQ. ID. No.268 | ORF918 | 559943 | 558210 | dipeptidase | TREMBL:LHDIPEP_1 | 2,00E-90 |
| SEQ. ID. No.269 | ORF921 | 560036 | 561700 | Glucose-6-phosphate 1-dehydrogenase | PIR:T36009 | 0 |
| SEQ.ID. No.270 | ORF922 | 561700 | 562722 | Unknown | PIR:T35159 | 1,00E-57 |
| SEQ.ID. No.271 | ORF925 | 562862 | 563791 | glucose-6-phosphate 1-dehydrogenase | PIR:T36011 | 9,00E-35 |
| SEQ.ID. No.272 | ORF927 | 565552 | 564101 | 6-phosphogluconate dehydrogenase | PIR:D70664 | 1,00E-167 |
| SEQ.ID. No.273 | ORF931 | 567372 | 569999 | PHAGE INFECTION PROTEIN. | SWISSPROT:PIP_LACLA | 3,00E-35 |
| SEQ.ID. No.274 | ORF934 | 569999 | 572158 | phage infection protein | PIR:E69115 | 2,00E-59 |
| SEQ.ID. No.275 | ORF941 | 574357 | 573563 | ABC transport system ATP-binding protein | PIR:D81263 | 2,00E-65 |
| SEQ.ID. No.276 | ORF945 | 576934 | 575633 | ABC transport system permease protein | PIR:T47014 | 2,00E-61 |
| SEQ. ID. No.277 | ORF949 | 579066 | 578386 | Unknown | PIR:A40182 | 9,00E-36 |
| SEQ. ID. No.278 | ORF951 | 581052 | 579244 | Unknown | PIR:B71257 | 2,00E-39 |
| SEQ. ID. No.279 | ORF956 | 582828 | 582190 | Unknown | PIR:E70063 | 4,00E-32 |
| SEQ.ID. No.280 | ORF959 | 582996 | 583352 | Unknown | PIR:D81250 | 5,00E-29 |
| SEQ.ID. No.281 | ORF965 | 586420 | 585539 | Transposase | TREMBL:ECIS1397_2 | 6,00E-46 |
| SEQ.ID. No.282 | ORF972 | 593430 | 587533 | Unknown | PIR:T29435 | 1,00E-142 |
| SEQ. ID. No.283 | ORF975 | 593599 | 594780 | transposase | TREMBLNEW:AB032203_2 | 1,00E48 |
| SEQ. ID. No.284 | ORF983 | 598200 | 596683 | glutamyl-tRNA synthase | PIR:T45422 | 1,00E-163 |
| SEQ. ID. No.285 | ORF987 | 600974 | 600060 | Unknown | PIRNEW:G82131 | 8,00E-25 |
| SEQ. ID. No.286 | ORF991 | 602769 | 602113 | Unknown | PIR:H70544 | 7,00E-31 |
| SEQ. ID. No.287 | ORF994 | 603031 | 604473 | Unknown | PIR:T35894 | 2,00E-25 |
| SEQ. ID. No.288 | ORF998 | 607523 | 606009 | formate tetrahydrofolate ligase | PIRNEW:E81981 | 5,00E-41 |
| SEQ. ID. No.289 | ORF1000 | 609284 | 607686 | dipeptidase | TREMBL:LHDIPEP_1 | 1,00E-105 |
| SEQ.ID. No.290 | ORF1004 | 610300 | 611799 | transposase | TREMBLNEW:AP001520_222 | 4,00E-52 |
| SEQ. ID. No.291 | ORF1007 | 611589 | 612581 | probable transposase | PIR:T14971 | 5,00E-33 |
| SEQ. ID. No.292 | ORF1010 | 613847 | 612690 | DNA polymerase | PIR:T36661 | 6,00E-57 |
| SEQ. ID. No.293 | ORF1013 | 614425 | 613847 | thymidylate kinase | PIR:T36663 | 8,00E-47 |
| SEQ. ID. No.294 | ORF1015 | 617834 | 614745 | DNA topoisomerase I | PIR:T36664 | 0 |
| SEQ. ID. No.295 | ORF1022 | 619472 | 621883 | penicillin-binding protein | PIR:T36588 | 2,00E-72 |
| SEQ. ID. No.296 | ORF1026 | 623874 | 621961 | Alpha-isopropyl malate synthase | PIR:G70794 | 0 |
| SEQ. ID. No.297 | ORF1032 | 626934 | 628025 | aspartate-semialdehyde dehydrogenase | PIR:A44846 | 3,00E-71 |
| SEQ. ID. No.298 | ORF1034 | 628652 | 628113 | aspartokinase | PIR:T35383 | 3,00E-39 |
| SEQ. ID. No.299 | ORF1035 | 629496 | 628735 | aspartate kinase alpha chain | PIR:S42422 | 2,00E-86 |
| SEQ.ID. No.300 | ORF1037 | 631140 | 629860 | Unknown | TREMBL:AF179376_1 | 4,00E-67 |
| SEQ.ID. No.301 | ORF1040 | 632029 | 633159 | fimbria-associated protein | TREMBL:AF019629_2 | 5,00E-41 |
| SEQ.ID. No.302 | ORF1041 | 633761 | 633162 | recombination protein | PIR:T35386 | 3,00E-70 |
| SEQ. ID. No.303 | ORF1042 | 636699 | 633790 | DNA polymerase III subunit | TREMBL:SCD25_3 | 1,00E-115 |
| SEQ. ID. No.304 | ORF1045 | 638822 | 637695 | Unknown | PIR:H70861 | 3,00E-21 |
| SEQ. ID. No.305 | ORF1052 | 642575 | 641511 | secretory protein | PIR:T36677 | 1,00E41 |
| SEQ. ID. No.306 | ORF1056 | 644480 | 643758 | Unknown function (dedA like protein) | PIR:XMECAD | 1,00E-29 |
| SEQ. ID. No.307 | ORF1059 | 646218 | 645418 | 3-oxoacyl-[acyl-carrier-protein] reductase | TREMBLNEW:AP001515_225 | 2,00E-41 |
| SEQ.ID. No.308 | ORF1064 | 650268 | 648919 | xanthine permease | PIR:C75614 | 1,00E-44 |
| SEQ. ID. No.309 | ORF1067 | 651128 | 650544 | heat shock transcription regulator | PIR:S70209 | 6,00E-20 |
| SEQ.ID. No.310 | ORF1068 | 652162 | 651146 | heat shock chaperone protein DNAJ-like | PIR:S76622 | 2,00E-26 |
| SEQ.ID. No.311 | ORF1072 | 653053 | 652397 | heat shock protein grpE | PIR:PN0643 | 8,00E-23 |
| SEQ.ID. No.312 | ORF1073 | 654930 | 653053 | dnaK-type molecular chaperone (Heat shock protein) | PIR:JN0830 | 0 |
| SEQ. ID. No.313 | ORF1077 | 657994 | 655916 | alpha-xylosidase | TREMBLNEW:SSO251975_1 | 2,00E-41 |
| SEQ. ID. No.314 | ORF1082 | 659278 | 658565 | transmembrane transport protein | PIR:T35672 | 4,00E-21 |
| SEQ. ID. No.315 | ORF1085 | 659548 | 660555 | transcriptional regulatory protein, LacI-family | TREMBL:SCF43_17 | 2,00E-27 |
| SEQ. ID. No.316 | ORF1090 | 661813 | 664047 | 4-alpha-glucanotransferase | PIR:G70928 | 1,00E-137 |
| SEQ.ID. No.317 | ORF1091 | 664182 | 665213 | transcriptional regulatory protein, LacI-family | TREMBL:SCF43_17 | 9,00E-27 |
| SEQ.ID. No.318 | ORF1094 | 665376 | 667187 | alpha-1,4-glucosidase | TREMBL:AF105219_2 | 1,00E-141 |
| SEQ.ID. No.319 | ORF1096 | 668331 | 667273 | ketol-acidreductoisomerase | PIR:D70855 | 6,00E-96 |
| SEQ.ID. No.320 | ORF1097 | 669803 | 668754 | ketol-acid reductoisomerase | TREMBLNEW:SC9A4_16 | 4,00E-98 |
| SEQ.ID. No.321 | ORF1098 | 671520 | 670003 | transmembrane transport protein | PIR:S47743 | 1,00E-114 |
| SEQ. ID. No.322 | ORF1101 | 673038 | 671998 | SUCROSE OPERON REGULATORY PROTEIN | SWISSPROT:SCRR_STRMU | 1,00E-37 |
| SEQ. ID. No.323 | ORF1107 | 676470 | 674947 | sucP-like protein | TREMBL:AF065245_3 | 1,00E-149 |
| SEQ. ID. No.324 | ORF1108 | 676805 | 677851 | transposase | TREMBL:AP000342_11 | 1,00E-50 |
| SEQ. ID. No.325 | ORF1124 | 683780 | 682749 | transcription regulator, LacI family | TREMBLNEW:SCG22_12 | 1,00E-40 |
| SEQ. ID. No. 326 | ORF1126 | 685751 | 683955 | alpha-L-arabinofuranosidase | PIR:S55274 | 1,00E-158 |
| SEQ. ID. No.327 | ORF1127 | 687709 | 685919 | potassium uptake protein | TREMBLNEW:AE004526_1 | 3,00E-52 |
| SEQ. ID. No.328 | ORF1130 | 688994 | 687912 | Transcriptional regulators of the Lad family | SWISSPROT:SCRR_STRMU | 9,00E-34 |
| SEQ. ID. No.329 | ORF1138 | 692525 | 690945 | Unknown | PIRNEW:C82449 | 1,00E-35 |
| SEQ. ID. No.330 | ORF1141 | 693835 | 692552 | adenylosuccinate synthetase | PIR:T36519 | 1,00E-165 |
| SEQ. ID. No.331 | ORF1143 | 694051 | 695115 | fructose-bisphosphate | TREMBL:MLCB4_29 | 1,00E-115 |
| SEQ. ID. No.332 | ORF1145 | 696249 | 695332 | heat shock protein | PIR:F70549 | 1,00E-64 |
| SEQ. ID. No.333 | ORF1147 | 697893 | 696445 | ferredoxin/ferredoxin--NADP reductase | TREMBL:SCF15_2 | 1,00E-1 10 |
| SEQ. ID. No.334 | ORF1148 | 698024 | 698863 | Unknown | PIR:H70908 | 5,00E-22 |
| SEQ. ID. No.335 | ORF1150 | 701365 | 699209 | cation-transporting ATPase | PIR:T36946 | 1,00E-96 |
| SEQ. ID. No.336 | ORF1157 | 703804 | 701780 | periplasmic serine proteinase | PIR:F72359 | 9,00E43 |
| SEQ. ID. No.337 | ORF1162 | 704221 | 705531 | queuine tRNA-ribosyltransferase | PIR:B69722 | 7,00E-84 |
| SEQ. ID. No.338 | ORF1168 | 706971 | 707651 | hemolysin III | TREMBL:AB003158_1 | 8,00E-23 |
| SEQ. ID. No.339 | ORF1172 | 708786 | 710264 | restriction endonuclease | PIR:JQ0759 | 2,00E45 |
| SEQ. ID. No.340 | ORF1173 | 711545 | 710277 | methyltransferase | PIR:JQ0760 | 2,00E-83 |
| SEQ. ID. No.341 | ORF1178 | 715545 | 714565 | peptide methionine sulfoxide reductase | PIR:G81243 | 1,00E-86 |
| SEQ.ID. No.342 | ORF1182 | 718646 | 716076 | Unknown | PIR:C70619 | 1,00E-23 |
| SEQ. ID. No.343 | ORF1188 | 719290 | 718646 | Unknown | PIR:S28812 | 6,00E-26 |
| SEQ.ID. No.344 | ORF1192 | 721991 | 720885 | Unknown | PIR:D64900 | 6,00E-42 |
| SEQ. ID. No.345 | ORF1198 | 723768 | 723052 | Unknown | PIR:S66056 | 1,00E-35 |
| SEQ. ID. No.346 | ORF1208 | 727984 | 730425 | X-Pro dipeptidyl-peptidase | TREMBLNEW:SC9E12_7 | 1,00E-127 |
| SEQ. ID. No.347 | ORF1210 | 730613 | 731311 | Unknown | PIR:T36712 | 7,00E-23 |
| SEQ. ID. No.348 | ORF1212 | 731344 | 731871 | Unknown | PIR:T36713 | 2,00E-20 |
| SEQ. ID. No.349 | ORF1213 | 731879 | 733570 | phosphoprotein phosphatase | PIR:H70699 | 2,00E-53 |
| SEQ. ID. No.350 | ORF1215 | 733570 | 735126 | FtsW cell division protein | PIR:T36715 | 6,00E-75 |
| SEQ. ID. No.351 | ORF1217 | 735126 | 736589 | penicillin-binding protein | PIR:F70699 | 2,00E-87 |
| SEQ. ID. No.352 | ORF1220 | 736589 | 737536 | serine/threonine protein kinase | PIR:E70699 | 1,00E-55 |
| SEQ. ID. No.353 | ORF1221 | 737536 | 739605 | probable serine/threonine protein kinase | PIR:T36717 | 1,00E-107 |
| SEQ. ID. No.354 | ORF1223 | 740478 | 739837 | glutamine amidotransferase | PIR:T36720 | 8,00E-62 |
| SEQ. ID. No.355 | ORF1225 | 741767 | 740532 | Unknown | PIR:T36718 | 8,00E-28 |
| SEQ. ID. No.356 | ORF1228 | 742564 | 741767 | Unknown | PIR:T36722 | 1,00E-28 |
| SEQ. ID. No.357 | ORF1234 | 744637 | 743795 | Unknown | PIR:A70840 | 9,00E-24 |
| SEQ. ID. No.358 | ORF1238 | 747835 | 745313 | Phosphorylase | PIR:S77252 | 0 |
| SEQ. ID. No.359 | ORF1242 | 749016 | 750104 | tryptophanyl-tRNA synthetase | PIR:E75438 | 1,00E-58 |
| SEQ. ID. No.360 | ORF1244 | 752072 | 750288 | Unknown | PIR:D75261 | 5,00E-36 |
| SEQ. ID. No.361 | ORF1247 | 753762 | 752125 | sodium/proline symporter | PIR:H69670 | 1,00E-91 |
| SEQ. ID. No.362 | ORF1256 | 756161 | 758911 | PHOSPHOENOLPYRUVATE CARBOXYLASE | SWISSPROT:CAPP ANASP | 1,00E-100 |
| SEQ. ID. No.363 | ORF1266 | 763670 | 762024 | ATP-dependent DNA helicase | PIR:F75302 | 3,00E-34 |
| SEQ. ID. No.364 | ORF1269 | 764892 | 763888 | transcription regulator, LacI family | TREMBLNEW:SCG22_12 | 3,00E-23 |
| SEQ. ID. No.365 | ORF1272 | 765438 | 766721 | permease | TREMBLNEW:AP001515_262 | 5,00E-65 |
| SEQ. ID. No.366 | ORF1274 | 768757 | 766844 | THIOREDOXIN REDUCTASE | SWISSPROT:TRXB_EUBAC | 6,00E-78 |
| SEQ.ID. No.367 | ORF1275 | 769489 | 768929 | alkyl hydrogen peroxide reductase | TREMBL:AF016233_2 | 9,00E-66 |
| SEQ. ID. No.368 | ORF1277 | 769695 | 770375 | icfA like carbonic anhydrase icfA | PIR:E70804 | 1,00E-36 |
| SEQ. ID. No.369 | ORF1280 | 770716 | 771978 | hemolysin | PIR:S76248 | 5,00E-52 |
| SEQ. ID. No.370 | ORF1281 | 772078 | 772554 | Unknown | TREMBL:AF068267_1 | 4,000-30 |
| SEQ. ID. No.371 | ORF1291 | 775634 | 776737 | Unknown | PIR:164018 | 1,00E-38 |
| SEQ. ID. No.372 | ORF1298 | 781148 | 779817 | aspartate transaminase | PIR:E71009 | 8,00E-68 |
| SEQ. ID. No.373 | ORF1300 | 781354 | 781785 | transcriptional regulatory protein | PIR:D70981 | 1,00E-29 |
| SEQ. ID. No.374 | ORF1302 | 782107 | 783450 | NADP-specific glutamate dehydrogenase | TREMBLNEW:AP001514_107 | 1,00E-162 |
| SEQ. ID. No.375 | ORF1304 | 785251 | 783833 | Unknown | PIR:D71089 | 3,00E-67 |
| SEQ. ID. No.376 | ORF1309 | 789370 | 786722 | DNA topoisomerase chain | PIR:T10970 | 0 |
| SEQ.ID. No.377 | ORF1310 | 791528 | 789441 | DNA GYRASE SUBUNIT B | SWISSPROT:GYRB_STRCO | 0 |
| SEQ.ID. No.378 | ORF1314 | 793363 | 792179 | DNA repair and genetic recombination protein | PIR:T10967 | 7,00E-60 |
| SEQ. ID. No.379 | ORF1315 | 794566 | 793445 | DNA-directed DNA polymerase III beta chain | PIR:B41870 | 2,00E-88 |
| SEQ. ID. No.380 | ORF1318 | 796804 | 795305 | replication initiation protein dnaA | PIR:IQMCL | 1,00E-106 |
| SEQ. ID. No.381 | ORF1323 | 797960 | 798964 | SpoIIJ like protein | PIR:T36570 | 9,00E-33 |
| SEQ. ID. No.382 | ORF1325 | 799091 | 799621 | Unknown | PIR:T36571 | 2,00E-40 |
| SEQ. ID. No.383 | ORF1327 | 799776 | 800438 | GLUCOSE INHIBITED DIVISION PROTEIN B | TREMBL:AF187159_6 | 3,00E-33 |
| SEQ.ID. No.384 | ORF1328 | 800652 | 801659 | ATPases involved in chromosome partitioning | TREMBL:AF187159_5 | 1,00E-76 |
| SEQ.ID. No.385 | ORF1329 | 801662 | 803020 | partitioning or sporulation protein | PIR:T36574 | 2,00E-68 |
| SEQ.ID. No.386 | ORF1331 | 804354 | 803332 | THIOREDOXIN REDUCTASE | SWISSPROT:TRXB_MYCSM | 1,00E-82 |
| SEQ. ID. No.387 | ORF1337 | 808341 | 806617 | Unknown | PIR:T36581 | 4,00E-44 |
| SEQ. ID. No.388 | ORF1342 | 812056 | 810608 | MutT/nudix family protein | PIR:E70600 | 1,00E-28 |
| SEQ. ID. No.389 | ORF1344 | 811984 | 813396 | RNA nucleotidyltransferase | PIR:T36583 | 1,00E-162 |
| SEQ.ID. No.390 | ORF1347 | 815110 | 814163 | Unknown | PIR:F70603 | 3,00E-26 |
| SEQ. ID. No.391 | ORF1350 | 816033 | 815110 | dimethyladenosine transferase | PIR:E70603 | 7,00E-62 |
| SEQ. ID. No.392 | ORF1358 | 821476 | 819239 | serine/threonine protein kinase | TREMBL:SCL11_7 | 7,00E-36 |
| SEQ. ID. No.393 | ORF1374 | 826539 | 826943 | involved in RIBONUCLEOTIDE REDUCTASE FUNCTION | PIR:E70648 | 1,00E-33 |
| SEQ. ID. No.394 | ORF1376 | 827062 | 829254 | RIBONUCLEOSIDE-DIPHOSPHATE REDUCTASE ALPHA | SWISSPROT:RIR1_MYCTU | 0 |
| SEQ.ID. No.395 | ORF1378 | 829569 | 830558 | ribonucleotide reductase subunit R2F | PIR:C70861 | 1,00E-145 |
| SEQ.ID. No.396 | ORF1379 | 830640 | 831797 | glycosyl trsnsferase (cps like) | PIR:T44648 | 1,00E-29 |
| SEQ.ID. No.397 | ORF1381 | 832160 | 833284 | ABC-type sugar transport protein | TREMBL:SRMSIK_1 | 1,00E-127 |
| SEQ.ID. No.398 | ORF1390 | 841887 | 843461 | Unknown | PIR:S52348 | 1,00E-20 |
| SEQ.ID. No.399 | ORF1392 | 843648 | 844631 | fimbria-associated protein | TREMBL:AP019629_2 | 1,00E-41 |
| SEQ.ID. No.400 | ORF1399 | 845955 | 847364 | Unknown | TREMBLNEW:SC5F8_10 | 1,00E-108 |
| SEQ.ID. No.401 | ORF1400 | 848496 | 847477 | RNA methyltransferase | TREMBLNEW:SCD8A_9 | 2,00E-51 |
| SEQ.ID. No.402 | ORF1401 | 848068 | 849849 | permease | TREMBLNEW:SC2D46_22 | 8,00E-50 |
| SEQ.ID. No.403 | ORF1403 | 849865 | 850758 | beta-1,4-D-xylanase | PIR:S16567 | 8,00E-35 |
| SEQ.ID. No.404 | ORF1405 | 850944 | 853928 | cation-transporting ATPase | TREMBLNEW:AP001515_249 | 1,00E-178 |
| SEQ. ID. No.405 | ORF1407 | 854346 | 855527 | transposase | TREMBLNEW:AB032203_2 | 1,00E-48 |
| SEQ. ID. No.406 | ORF1410 | 855686 | 856453 | cyclase involved in histidine biosynthetic pathway | PIR:T35077 | 2,00E-96 |
| SEQ. ID. No.407 | ORF1413 | 856856 | 858619 | anthranilate synthase component I | SWISSPROT:TAPE_ARTGO | 1,00E-141 |
| SEQ.ID. No.408 | ORF1415 | 858938 | 860536 | ABC transporter ATP binding protein | TREMBL:SCI8_25 | 1,00E-179 |
| SEQ. ID. No.409 | ORF1418 | 861361 | 861726 | Unknown | TREMBL:SCD25_30 | 9,00E-23 |
| SEQ. ID. No.410 | ORF1421 | 864063 | 862453 | methyl coenzyme M reductase system, component A2 | PIR:D69159 | 3,00E-67 |
| SEQ. ID. No.411 | ORF1427 | 866861 | 865593 | transposase | TREMBL:REU010061_1 | 4,00E-78 |
| SEQ. ID. No.412 | ORF1442 | 875122 | 878154 | Excinuclease ABC chain A | PIR:S04781 | 0 |
| SEQ. ID. No.413 | ORF1443 | 878307 | 880670 | excinuclease abc subunit c | PIR:A70903 | 3,00E-92 |
| SEQ. ID. No.414 | ORF1445 | 880782 | 881750 | shikimate 5-dehydrogenase | PIR:D70660 | 1,00E-25 |
| SEQ. ID. No.415 | ORF1447 | 881753 | 882736 | Unknown | PIR:T36025 | 3,00E-80 |
| SEQ. ID. No.416 | ORF1450 | 882938 | 883885 | Unknown | PIR:D70903 | 3,00E-65 |
| SEQ.ID. No.417 | ORF1453 | 883937 | 885259 | phosphoglycerate kinase | PIR:T36019 | 1,00E-135 |
| SEQ.ID. No.418 | ORF1454 | 885319 | 886119 | triose phosphate isomerase | PIR:A70916 | 8,00E-68 |
| SEQ.ID. No.419 | ORF1457 | 886536 | 887483 | lactate dehydrogenase | PIR:T44580 | 1,00E-67 |
| SEQ.ID. No.420 | ORF1463 | 888457 | 889998 | aminotransferase | TREMBL:HI32714_11 | 1,00E-151 |
| SEQ.ID. No.421 | ORF1467 | 892749 | 891415 | branched-chain amino acid transport system II carrier protein | SWISSPROT:BRNQ_BACSU | 1,00E-69 |
| SEQ.ID. No.422 | ORF1469 | 893981 | 892881 | transaldolase | PIR:C70917 | 1,00E-108 |
| SEQ.ID. No.423 | ORF1470 | 896210 | 894105 | transketolase | PIR:T35162 | 0 |
| SEQ.ID. No.424 | ORF1473 | 896585 | 897700 | heat-inducible transcriptional repressor | TREMBL:SCC77_22 | 7,00E-65 |
| SEQ.ID. No.425 | ORF1476 | 897759 | 898901 | heat-shock protein DnaJ | TREMBL:SCC77_21 | 8,00E-69 |
| SEQ.ID. No.426 | ORF1481 | 899886 | 900767 | bacitracin resistance like protein | TREMBLNEW:SC2G61_8 | 3,00E-55 |
| SEQ. ID. No.427 | ORF1482 | 901919 | 900930 | Unknown | PIR:S72871 | 3,00E-35 |
| SEQ. ID. No.428 | ORF1486 | 902580 | 904610 | threonine-tRNA synthetase | TREMBL:SCL2_21 | 0 |
| SEQ. ID. No.429 | ORF1488 | 904753 | 905334 | Histidine triad protein (hit) | TREMBL:SCL2_20 | 1,00E-49 |
| SEQ. ID. No.430 | ORF1490 | 905476 | 906228 | Unknown | PIR:F70500 | 3,00E-84 |
| SEQ. ID. No.431 | ORF1491 | 906237 | 906818 | probable crossover junction endodeoxyribonuclease ruvC | PIR:A70727 | 7,00E-32 |
| SEQ. ID. No.432 | ORF1493 | 906879 | 907502 | holliday junction DNA helicase | TREMBL:SCL2_9 | 7,00E-26 |
| SEQ.ID. ID. No.433 | ORF1494 | 907505 | 908566 | holliday junction DNA helicase | TREMBL:SCL2_8 | 1,00E-113 |
| SEQ. ID. No.434 | ORF1497 | 909132 | 909710 | Adenine/guaninephosphoribosyltransferases | TREMBL:ECU82664_67 | 3,00E-32 |
| SEQ.ID. No.435 | ORF1499 | 909810 | 911009 | succinyl-CoA synthetase beta subunit | PIR:T45434 | 3,00E-92 |
| SEQ.ID. No.436 | ORF1501 | 911012 | 911920 | succinyl-CoA synthetase alpha subunit | PIR:T45435 | 3,00E-98 |
| SEQ.ID. No.437 | ORF1506 | 913270 | 914904 | 5'-phosphoribosyl-5-aminoimidazole-4-carboxamide formyltransfer ase | TREMBL:AB003159_3 | 1,00E-166 |
| SEQ.ID. No.438 | ORF1512 | 916418 | 917185 | pseudouridylate synthase | TREMBL:MLCB1351_3 | 7,00E-64 |
| SEQ.ID. No.439 | ORF1514 | 917185 | 919311 | GTP-binding protein | PIR:H70504 | 1,00E-143 |
| SEQ.ID. No.440 | ORF1515 | 919669 | 921195 | UDP-glucose pyrophosphorylase | TREMBL:AF203909_1 | 5,00E-57 |
| SEQ. ID. No.441 | ORF1523 | 923592 | 926180 | helicase | TREMBL:SCI41_14 | 1,00E-148 |
| SEQ.ID. No.442 | ORF1529 | 929078 | 928449 | hypothetical protein Rv1830 - Mycobacterium | PIR:G70721 | 3,00E-34 |
| SEQ. ID. No.443 | ORF1530 | 929622 | 929182 | Unknown | TREMBLNEW:SC1A8A_4 | 2,00E-28 |
| SEQ. ID. No.444 | ORF1531 | 930450 | 929632 | Unknown | PIR:B70721 | 4,00E-23 |
| SEQ.ID. No.445 | ORF1535 | 932460 | 931768 | CDP-diacylglycerol--glycerol-3-phosphate 3-phosphatidyl-transferase | TREMBLNEW:SC1A8A_9 | 4,00E-21 |
| SEQ.ID. No.446 | ORF1536 | 933247 | 932399 | ATP-phosphoribosyl transferase | PIR:D70513 | 2,00E-73 |
| SEQ.ID. No.447 | ORF1540 | 934253 | 933588 | ribulose-phosphate 3-epimerase | PIR:E70901 | 2,00E-60 |
| SEQ.ID. No.448 | ORF1541 | 935276 | 934332 | prolipoprotein diacylglyceryl transferase | TREMBLNEW:SC8E7_19 | 1,00E-55 |
| SEQ.ID. No.449 | ORF1545 | 936264 | 935392 | tryptophan synthase | TREMBL:AF057042_2 | 2,00E-63 |
| SEQ. ID. No.450 | ORF1546 | 938369 | 936285 | tryptophan synthase beta chain | PIR:T35066 | 1,00E-134 |
| SEQ. ID. No.451 | ORF1549 | 939747 | 938899 | Endonuclease IV | TREMBLNEW:AP001511_275 | 3,00E-49 |
| SEQ. ID. No.452 | ORF1548 | 939871 | 941268 | histidine permease | PIR:A69751 | 1,00E-137 |
| SEQ. ID. No.453 | ORF1559 | 945113 | 944658 | Unknown | PIR:T35570 | 8,00E-34 |
| SEQ. ID. No.454 | ORF1575 | 952215 | 951598 | MUTATOR MUTT PROTEIN | TREMBLNEW:AP001517_211 | 7,00E-33 |
| SEQ. ID. No.455 | ORF1580 | 954360 | 955931 | histidine permease | PIR:A69751 | 1,00E-151 |
| SEQ. ID. No.456 | ORF1581 | 956788 | 956141 | signal peptidase I | TREMBL:SLTK24SIP_1 | 6,00E-37 |
| SEQ. ID. No.457 | ORF1587 | 959688 | 960875 | aspartate aminotransferase | PIR:H69826 | 2,00E-65 |
| SEQ. ID. No.458 | ORF1590 | 960918 | 962678 | Unknown | PIR:T35571 | 5,00E-30 |
| SEQ. ID. No.459 | ORF1595 | 963514 | 964434 | Unknown | TREMBL:SA17221_1 | 4,00E-81 |
| SEQ. ID. No.460 | ORF1599 | 966884 | 966192 | Orotate phosphoribosyltransferase | TREMBLNEW:AE004945_10 | 8,00E45 |
| SEQ. ID. No.461 | ORF1601 | 968170 | 966896 | dihydroorotase dehydrogenase | PIR:B70304 | 1,00E-57 |
| SEQ. ID. No.462 | ORF1604 | 968691 | 967870 | dihydroorotate dehydrogenase | PIR:G39845 | 5,00E-23 |
| SEQ. ID. No.463 | ORF1605 | 969780 | 968830 | orotidine 5'-phosphate decarboxylase | PIR:G75302 | 3,00E-38 |
| SEQ. ID. No.464 | ORF1607 | 971294 | 969801 | pyrC DIHYDROOROTASE | SWISSPROT:PYRC_LACPL | 4,00E-56 |
| SEQ. ID. No.465 | ORF1611 | 972690 | 971713 | aspartate carbamoyltransferase, catalytic chain | PIR:C75042 | 1,00E-79 |
| SEQ. ID. No.466 | ORF1613 | 976044 | 972817 | glutamate-ammonia-ligase adenylyltransferase | PIR:A70776 | 1,00E-171 |
| SEQ. ID. No.467 | ORF1615 | 977221 | 976103 | bile salt hydrolase | TREMBLNEW:AF148138_1 | 0 |
| SEQ. ID. No.468 | ORF1617 | 978041 | 977190 | 5,10-methylenetetrahydrofolate reductase | PIR:D81326 | 3,00E-56 |
| SEQ. ID. No.469 | ORF1618 | 980403 | 978103 | 5-methyltetrahydropteroyltriglutamate-- homocystei methyltransferase | PIR:C81326 | 0 |
| SEQ. ID. No.470 | ORF1622 | 982368 | 981301 | SAM-dependent methyltransferase | TREMBL:SCI41_34 | 3,00E-63 |
| SEQ. ID. No.471 | ORF1627 | 984644 | 983910 | transcription regulator | TREMBLNEW:AP001516_108 | 4,00E-38 |
| SEQ. ID. No.472 | ORF1631 | 985704 | 986210 | Unknown | TREMBLNEW:AE004789_1 | 1,00E-33 |
| SEQ. ID. No.473 | ORF1646 | 992784 | 993620 | Unknown | TREMBLNEW:AF164956_27 | 2,00E-22 |
| SEQ. ID. No.474 | ORF1656 | 997180 | 996596 | bacterioferritin comigratory protein | PIR:F70870 | 4,00E-38 |
| SEQ.ID. No.475 | ORF1662 | 1000036 | 999293 | ABC-type transport system | PIR:D70860 | 5,00E-45 |
| SEQ. ID. No.476 | ORF1664 | 1001554 | 1000307 | hexosyltransferase | TREMBL:SCM11_17 | 1,00E-105 |
| SEQ.ID. No.477 | ORF1670 | 1005950 | 1004313 | peptide transport system secreted peptide-binding protein | PIR:C70789 | 1,00E-124 |
| SEQ. ID. No.478 | ORF1676 | 1009609 | 1008077 | glutamate synthase | PIR:T34868 | 1,00E-151 |
| SEQ. ID. No.479 | ORF1678 | 1014182 | 1009614 | glutamate synthase large chain | PIR:T34869 | 0 |
| SEQ. ID. No.480 | ORF1687 | 1018227 | 1017184 | Transcriptional regulators of the Lad family | TREMBL:SC6D11_7 | 1,00E-45 |
| SEQ. ID. No.481 | ORF1696 | 1020864 | 1021502 | Unknown | PIR:F69433 | 7,00E-20 |
| SEQ. ID. No.482 | ORF1697 | 1022739 | 1021558 | transposase | TREMBLNEW:AB032203_2 | 1,00E-48 |
| SEQ. ID. No.483 | ORF1705 | 1025143 | 1023926 | Unknown | TREMBLNEW:AE004537_6 | 2,00E-94 |
| SEQ. ID. No.484 | ORF1707 | 1025240 | 1026337 | transcriptional regulator; LacI-family | TREMBL:AB016845_1 | 3,00E-36 |
| SEQ.ID. No.485 | ORF1708 | 1027829 | 1026423 | oxygen-independent coproporphyrinogen III oxidase | TREMBL:SCC77_26 | 1,00E-77 |
| SEQ.ID. No.486 | ORF1711 | 1029709 | 1027832 | lepA"; product: "GTP-binding protein (elongation factor family) | PIR:G70683 | 0 |
| SEQ.ID. No.487 | ORF1715 | 1031159 | 1030371 | Unknown | PIR:T34761 | 1,00E-33 |
| SEQ. ID. No.488 | ORF1717 | 1032306 | 1031410 | beta-lactamase regulatory protein | SWISSPROT:YBLI_STRCI | 8,00E-26 |
| SEQ.ID. No.489 | ORF1719 | 1033627 | 1032503 | branched-chain amino acid aminotransferase | PIRNEW:A82612 | 1,00E-103 |
| SEQ.ID. No.490 | ORF1721 | 1034472 | 1033855 | ribosomal protein 125 | TREMBLNEW:SCE66_3 | 2,00E-33 |
| SEQ.ID. No.491 | ORF1725 | 1037559 | 1036138 | NAD(P)+ transhydrogenase (B-specific) | TREMBL:RR05294_3 | 2,00E-90 |
| SEQ.ID. No.492 | ORF1728 | 1039043 | 1037883 | NAD(P) transhydrogenase | PIR:F70618 | 3,00E-56 |
| SEQ.ID. No.493 | ORF1730 | 1039384 | 1041492 | long-chain-fatty-acid-CoA ligase | PIR:E70937 | 1,00E-115 |
| SEQ.ID. No.494 | ORF1731 | 1042669 | 1041551 | GTP-binding protein | PIR:C70586 | 2,00E-77 |
| SEQ.ID. No.495 | ORF1733 | 1044047 | 1042617 | Unknown | PIR:E70586 | 8,00E-54 |
| SEQ.ID. No.496 | ORF1737 | 1044803 | 1044126 | Unknown | TREMBL:SCC117_6 | 2,00E-24 |
| SEQ.ID. No.497 | ORF1739 | 1045836 | 1044664 | Phosphate starvation-inducible protein PhoH | TREMBL:SCC117_5 | 2,00E-83 |
| SEQ. ID. No.498 | ORF1742 | 1047039 | 1046245 | Unknown | TREMBL:SCC77_19 | 3,00E-28 |
| SEQ. ID. No.499 | ORF1743 | 1047283 | 1048158 | rRNA methylase | TREMBL:SCL11_8 | 2,00E-60 |
| SEQ. ID. No.500 | ORF1745 | 1048362 | 1049603 | glucose-1-phosphate adenylyltransferase - | PIR:C70610 | 1,00E-122 |
| SEQ. ID. No.501 | ORF1746 | 1050289 | 1049699 | Unknown | PIR:E70872 | 3,00E-26 |
| SEQ. ID. No.502 | ORF1747 | 1050835 | 1050299 | Unknown Function. NifU-related protein | PIR:T35992 | 1,00E-29 |
| SEQ. ID. No.503 | ORF1749 | 1052136 | 1050865 | Aminotransferase | PIR:C70872 | 2,00E-95 |
| SEQ. ID. No.504 | ORF1751 | 1053054 | 1052278 | ABC-type transport system ATP-binding chain - | PIR:T35994 | 1,00E-100 |
| SEQ. ID. No.505 | ORF1753 | 1054315 | 1053083 | Unknown | PIR:T35996 | 4,00E-77 |
| SEQ.ID. No.506 | ORF1754 | 1055820 | 1054324 | ABC transporter membrane protein | PIR:T35997 | 0 |
| SEQ. ID. No.507 | ORF1759 | 1059546 | 1057888 | CTP synthetase | PIR:T36879 | 0 |
| SEQ. ID. No.508 | ORF1760 | 1060978 | 1059695 | peptidase V | PIR:B69994 | 1,00E-30 |
| SEQ. ID. No.509 | ORF1763 | 1061638 | 1061195 | 3-dehydroquinate dehydratase | PIR:F75475 | 2,00E-33 |
| SEQ. ID. No.510 | ORF1765 | 1063424 | 1061805 | 3-dehydroquinate synthase | TREMBLNEW:SC9C5_18 | 2,00E-82 |
| SEQ. ID. No.511 | ORF1768 | 1064694 | 1063510 | chorismate synthase | PIR:H70658 | 1,00E-117 |
| SEQ. ID. No.512 | ORF1770 | 1066580 | 1065402 | Unknown | TREMBLNEW:AF170880_2 | 3,00E-30 |
| SEQ.ID. No.513 | ORF1773 | 1069739 | 1067061 | alanine-tRNA ligase | PIR:C70520 | 0 |
| SEQ. ID. No.514 | ORF1779 | 1071663 | 1073519 | acyltransferase | PIR:C69975 | 6,00E-57 |
| SEQ. ID. No.515 | ORF1782 | 1074239 | 1073616 | ribosomal protein | PIR:A37146 | 8,00E-40 |
| SEQ.ID. No.516 | ORF1783 | 1075406 | 1074441 | ABC transporter (ATP-binding protein) | TREMBLNEW:AE004665_5 | 9,00E-33 |
| SEQ.ID. No.517 | ORF1789 | 1079904 | 1077262 | ATP-dependent DNA helicase | TREMBLNEW:SCD63A_8 | 0 |
| SEQ. ID. No.518 | ORF1790 | 1080021 | 1080599 | xanthine phosphoribosyltransferase | TREMBLNEW:AE004942_6 | 1,00E-45 |
| SEQ.ID. No.519 | ORF1792 | 1080653 | 1082014 | xanthine/uracil permease family protein | PIR:E81141 | 6,00E-62 |
| SEQ. ID. No.520 | ORF1800 | 1083887 | 1084822 | Unknown | TREMBLNEW:SC9E12_23 | 3,00E-32 |
| SEQ. ID. No.521 | ORF1803 | 1085474 | 1086085 | nicotinamidase | PIR:B70944 | 4,00E-40 |
| SEQ. ID. No.522 | ORF1811 | 1091637 | 1090564 | ABC transporter (ATP-binding protein) | TREMBLNEW:AP001514_104 | 2,00E-58 |
| SEQ.ID. No.523 | ORF1819 | 1093279 | 1093932 | two-component response regulator | PIR:T36862 | 7,00E-31 |
| SEQ.ID. No.524 | ORF1823 | 1095812 | 1094763 | low-affinity inorganic phosphate transport homolog | PIR:A69855 | 2,00E-44 |
| SEQ. ID. No.525 | ORF1827 | 1098586 | 1096190 | carbon starvation protein | PIR:H70649 | 1,00E-170 |
| SEQ.ID. No.526 | ORF1829 | 1098729 | 1100123 | Unknown | TREMBLNEW:AP001517_49 | 1,00E-34 |
| SEQ.ID. No.527 | ORF1834 | 1103124 | 1100941 | ATP-dependent RNA helicase | TREMBL:SCD25_32 | 1,00E-100 |
| SEQ.ID. No.528 | ORF1836 | 1104994 | 1103507 | uracil transport protein | PIR:D64842 | 2,00E-72 |
| SEQ.ID. No.529 | ORF1843 | 1108353 | 1107193 | Unknown | TREMBL:EFY17797_6 | 1,00E-37 |
| SEQ. ID. No.530 | ORF1850 | 1111209 | 1112906 | serine-threonine protein kinase | PIR:T10009 | 2,00E-22 |
| SEQ.ID. No.531 | ORF1853 | 1114437 | 1112950 | macrolide-efflux pump | TREMBL:LLLPK214_12 | 1,00E-35 |
| SEQ.ID. No.532 | ORF1855 | 1116800 | 1114752 | multidrug resistance protein | PIR:F69763 | 1,00E-80 |
| SEQ.ID. No.533 | ORF1865 | 1122567 | 1121476 | gtp-binding protein | PIR:F70898 | 1,00E-121 |
| SEQ.ID. No.534 | ORF1866 | 1123565 | 1122588 | pyrroline-5-carboxylate reductase | PIR:D71281 | 4,00E-42 |
| SEQ. ID. No.535 | ORF1868 | 1124803 | 1123463 | prolyl aminopeptidase | PIR:JC4623 | 9,00E-95 |
| SEQ. ID. No.536 | ORF1871 | 1125014 | 1127587 | two-component sensor kinase | PIR:T36078 | 3,00E-28 |
| SEQ. ID. No.537 | ORF1874 | 1127666 | 1128409 | two-component response regulator | PIR:T29457 | 5,00E-54 |
| SEQ. ID. No.538 | ORF1875 | 1131146 | 1128507 | Unknown | TREMBLNEW:SCG20A_11 | 4,00E-35 |
| SEQ.ID. No.539 | ORF1880 | 1131979 | 1131191 | ABC transporter, ATP-binding protein | TREMBLNEW:SCG20A_12 | 8,00E-72 |
| SEQ. ID. No.540 | ORF1885 | 1133644 | 1132331 | O-acetylhomoserine | TREMBLNEW:AP001516_32 | 1,00E-126 |
| SEQ.ID. No.541 | ORF1888 | 1134197 | 1135066 | pyridoxal kinase | PIR:G70195 | 8,00E-24 |
| SEQ. ID. No.542 | ORF1891 | 1135867 | 1137399 | Mg(2+) chelatase family protein | PIR:D70926 | 1,00E-117 |
| SEQ.ID. No.543 | ORF1892 | 1137399 | 1139096 | DNA processing chain A (DprA) protein | PIR:C70926 | 4.00E-29 |
| SEQ.ID. No.544 | ORF1895 | 1139156 | 1141018 | succinate dehydrogenase | TREMBLNEW:SC5G8_24 | 1,00E-138 |
| SEQ.ID. No.545 | ORF1896 | 1141117 | 1142079 | fumarate reductase | PIR:F70843 | 7,00E-35 |
| SEQ.ID. No.546 | ORF1899 | 1143828 | 1142890 | Unknown | TREMBLNEW:SCP8_3 | 9,00E-69 |
| SEQ.ID. No.547 | ORF1901 | 1145479 | 1144064 | sodium/proton antiporter | PIR:T36685 | 3,00E-61 |
| SEQ.ID. No.548 | ORF1905 | 1147454 | 1145964 | ATP-dependent clp proteinase | TREMBL:BSCLPXGEN_1 | 1,00E-128 |
| SEQ.ID. No.549 | ORF1907 | 1148202 | 1147504 | ATP-dependent protease | TREMBL:AF071885_2 | 7,00E-67 |
| SEQ. ID. No.550 | ORF1908 | 1148831 | 1148211 | ATP-dependent clp proteinase | PIR:D70865 | 9,00E-58 |
| SEQ.ID. No.551 | ORF1911 | 1150898 | 1149435 | chloride channel protein-related protein | TREMBL:AF179611_12 | 4,00E-26 |
| SEQ.ID. No.552 | ORF1914 | 1152461 | 1151085 | chaperone protein | PIR:E70865 | 5,00E-83 |
| SEQ.ID. No.553 | ORF1915 | 1153817 | 1152519 | Ribonuclease | PIR:T34708 | 9,00E-76 |
| SEQ.ID. No.554 | ORF1918 | 1155396 | 1154518 | pyruvate formate-lyase 1 activating enzyme | TREMBL:AF088897-13 | 8,00E-54 |
| SEQ.ID. No.555 | ORF1920 | 1157920 | 1155509 | formate C-acetyltransferase | TREMBL:AF088897_12 | 0 |
| SEQ.ID. No.556 | ORF1922 | 1160121 | 1158427 | NAD+ synthase (glutamine-hydrolyzing) | PIRNEW:T51756 | 1,00E-128 |
| SEQ.ID. No.557 | ORF1923 | 1161621 | 1160473 | hippurate hydrolase | PIR:E69640 | 5,00E-75 |
| SEQ.ID. No.558 | ORF1928 | 1163598 | 1162396 | ABC transporter (ATP-binding protein) | TREMBLNEW:AP001518_312 | 7,00E-77 |
| SEQ.ID. No.559 | ORF1930 | 1164712 | 1163735 | lipoprotein | PIR:JN0753 | 4,00E-36 |
| SEQ. ID. No.560 | ORF1932 | 1164914 | 1165732 | Unknown | PIR:C69862 | 2,00E-24 |
| SEQ. ID. No.561 | ORF1935 | 1168334 | 1165860 | transketolase I | PIR:S76896 | 0 |
| SEQ. ID. No.562 | ORF1936 | 1168735 | 1170339 | GMP synthetase | PIR:A70735 | 0 |
| SEQ. ID. No.563 | ORF1939 | 1170918 | 1172789 | acyltransferase | PIR:C69975 | 3,00E-57 |
| SEQ. ID. No.564 | ORF1941 | 1172869 | 1173897 | ribose-phosphate pyrophosphokinase | PIR:D70622 | 1,00E-113 |
| SEQ. ID. No.565 | ORF1943 | 1174182 | 1175561 | udp-n-acetylglucosamine pyrophosphorylase - | PIR:E70622 | 1,00E-110 |
| SEQ.ID. No.566 | ORF1944 | 1175568 | 1175978 | Unknown | TREMBL:SCC123_15 | 9,00E-28 |
| SEQ. ID. No.567 | ORF1946 | 1176163 | 1176858 | phosphoglycerate mutase | PIR:A75439 | 2,00E-20 |
| SEQ.ID. No.568 | ORF1949 | 1177491 | 1179188 | phosphate acetyltransferase | PIR:F70628 | 2,00E-95 |
| SEQ. ID. No.569 | ORF1951 | 1179326 | 1180552 | ACETATE KINASE | SWISSPROT:ACKA_CORGL | 1,00E-104 |
| SEQ.ID. No.570 | ORF1952 | 1182093 | 1180729 | 3-phosphoshikimate 1-carboxyvinyltransferase | TREMBLNEW:SC7E4_9 | 2,00E-89 |
| SEQ.ID. No.571 | ORF1957 | 1183623 | 1184213 | resolvase | PIR:A70583 | 2,00E-34 |
| SEQ.ID. No.572 | ORF1959 | 1184203 | 1185522 | transposase | PIR:B70583 | 6,00E-47 |
| SEQ.ID. No.573 | ORF1962 | 1188391 | 1186907 | lactose transport protein | TREMBL:LL47655_2 | 1,00E-132 |
| SEQ.ID. No.574 | ORF1964 | 1188756 | 1191824 | beta-galactosidase | TREMBL:BLO242596_1 | 0 |
| SEQ. ID. No.575 | ORF1967 | 1192641 | 1193192 | 3-methyladenine-DNA glycosylase | TREMBL:BLO242596_2 | 1,00E-108 |
| SEQ. ID. No.576 | ORF1972 | 1197131 | 1194993 | glycosyl hydrolase | TREMBL:SCO001206_2 | 0 |
| SEQ.ID. No.577 | ORF1976 | 1201862 | 1198998 | DNA polymerase I | PIR:C70559 | 0 |
| SEQ.ID. No.578 | ORF1977 | 1202693 | 1201911 | response regulator | PIR:T35758 | 3,00E-50 |
| SEQ.ID. No.579 | ORF1980 | 1205315 | 1203789 | pyruvate kinase | PIR:T35759 | 1,00E-130 |
| SEQ.ID. No.580 | ORF1982 | 1206382 | 1205399 | transmembrane transport protein | PIR:T36036 | 2,00E-72 |
| SEQ.ID. No.581 | ORF1983 | 1208662 | 1206554 | excinuclease ABC chain B | PIR:S03812 | 0 |
| SEQ.ID. No.582 | ORF1984 | 1209291 | 1208677 | Unknown | PIR:T35741 | 7,00E-35 |
| SEQ.ID. No.583 | ORF1986 | 1210940 | 1209468 | ribosomal protein S1 | PIR:T35743 | 0 |
| SEQ.ID. No.584 | ORF1988 | 1211938 | 1211063 | methylenetetrahydrofolate dehydrogenase | TREMBLNEW:SC2A6_9 | 1,00E-85 |
| SEQ.ID. No.585 | ORF1991 | 1213312 | 1214295 | ABC transporter, ATP-binding protein | TREMBLNEW:AP001511_283 | 7,00E-24 |
| SEQ.ID. No.586 | ORF1995 | 1214365 | 1215195 | permease of ABC zinc transporter | TREMBLNEW:AE004962_10 | 4,00E-25 |
| SEQ. ID. No.587 | ORF1997 | 1215774 | 1215253 | Unknown | PIR:C70607 | 3,00E-27 |
| SEQ. ID. No.588 | ORF1999 | 1216476 | 1215886 | transcription factor | PIR:H70803 | 7,00E-46 |
| SEQ. ID. No.589 | ORF2002 | 1216693 | 1218903 | 1,4-alpha-glucan branching enzyme | PIR:B70770 | 0 |
| SEQ. ID. No.590 | ORF2003 | 1218942 | 1219661 | phosphate response regulator protein phoP | TREMBLNEW:SCCB12_7 | 4,00E-79 |
| SEQ. ID. No.591 | ORF2005 | 1219661 | 1220734 | two-component system sensor kinase | TREMBLNEW:SCCB12_6 | 3,00E-45 |
| SEQ. ID. No.592 | ORF2021 | 1228492 | 1229922 | transcription regulator | PIR:T35584 | 2,00E-24 |
| SEQ. ID. No.593 | ORF2025 | 1230170 | 1231918 | cell division-related protein | TREMBLNEW:SCD35_15 | 6,00E-40 |
| SEQ. ID. No.594 | ORF2029 | 1233918 | 1232389 | Sensory transduction histidine kinase | PIR:E70596 | 2,00E-73 |
| SEQ. ID. No.595 | ORF2033 | 1235060 | 1235875 | hemolysin-like protein | TREMBL:AB003158_1 | 5,00E-33 |
| SEQ. ID. No.596 | ORF2037 | 1236412 | 1235936 | transcription elongation factor | PIR:F70894 | 7,00E-22 |
| SEQ. ID. No.597 | ORF2038 | 1236918 | 1236514 | peptidylprolyl isomerase | PIR:546228 | 2,00E-25 |
| SEQ. ID. No.598 | ORF2039 | 1239279 | 1237063 | L-serine dehydratase | PIR:T34749 | 1,00E-128 |
| SEQ. ID. No.599 | ORF2043 | 1240667 | 1239669 | exopolyphosphatase | PIR:E70623 | 1,00E-53 |
| SEQ. ID. No.600 | ORF2045 | 1241296 | 1240733 | Unknown | PIR:D70623 | 1,00E-30 |
| SEQ. ID. No.601 | ORF2048 | 1243293 | 1241998 | phosphopyruvate hydratase | PIR:B69620 | 1,00E-140 |
| SEQ. ID. No.602 | ORF2050 | 1244391 | 1243447 | oxidoreductase | PIR:B75341 | 3,00E-32 |
| SEQ.ID. No.603 | ORF2053 | 1248115 | 1244534 | transcription-repair coupling factor (mfd) | PIR:G70622 | 0 |
| SEQ. ID. No.604 | ORF2055 | 1248704 | 1248108 | peptidyl-trna hydrolase | PIR:A70622 | 2,00E-36 |
| SEQ. ID. No.605 | ORF2061 | 1251010 | 1250516 | phosphinothricin acetyltransferase | PIR:E75427 | 2,00E-21 |
| SEQ.ID. No.606 | ORF2062 | 1254333 | 1251136 | Unknown | TREMBLNEW:SC23B6_28 | 1,00E-160 |
| SEQ. ID. No.607 | ORF2065 | 1256591 | 1255809 | Unknown | TREMBL:SCC123_17 | 3,00E-69 |
| SEQ. ID. No.608 | ORF2068 | 1258578 | 1257169 | gamma-glutamyl phosphate | TREMBL:SCC123_23 | 1,00E-109 |
| SEQ. ID. No.609 | ORF2070 | 1259284 | 1258586 | glycine hydroxymethyltransferase | PIR:C70896 | 9,00E-41 |
| SEQ. ID. No.610 | ORF2071 | 1259507 | 1260994 | threonine synthase | PIR:T39213 | 7,00E-73 |
| SEQ. ID. No.611 | ORF2074 | 1261517 | 1264300 | cation-transporting P-ATPase | PIR:E69000 | 1,00E-144 |
| SEQ.ID. No.612 | ORF2078 | 1266735 | 1265818 | ABC transporter, ATP-binding protein | TREMBLNEW:AP001509_90 | 5,00E-52 |
| SEQ. ID. No.613 | ORF2082 | 1269816 | 1268017 | DNA repair protein | PIR:T36883 | 3,00E-88 |
| SEQ. ID. No.614 | ORF2087 | 1270844 | 1269819 | Unknown | PIR:T36884 | 4,00E-59 |
| SEQ. ID. No.615 | ORF2091 | 1271091 | 1272554 | potassium uptake protein KtrB | TREMBLNEW:AP001509_36 | 2,00E-47 |
| SEQ. ID. No.616 | ORF2094 | 1272191 | 1273267 | potassium uptake protein KtrA | TREMBLNEW:AP001509_35 | 1,00E-30 |
| SEQ. ID. No.617 | ORF2097 | 1274906 | 1274067 | Hemolysin A homolog | PIR:T36885 | 8,00E-47 |
| SEQ. ID. No.618 | ORF2099 | 1276134 | 1275097 | sugar phosphatase | PIR:T36891 | 1,00E-59 |
| SEQ. ID. No.619 | ORF2102 | 1277930 | 1276146 | Unknown | PIR:B70502 | 2,00E-27 |
| SEQ. ID. No.620 | ORF2106 | 1279280 | 1277961 | tyrosyl-tRNA synthetase | TREMBL:SCI8_3 | 1,00E-122 |
| SEQ. ID. No.621 | ORF2113 | 1282087 | 1281281 | molybdopterin biosynthesis protein | PIR:A70415 | 3,00E-75 |
| SEQ. ID. No.622 | ORF2115 | 1283033 | 1282167 | thiamin biosynthesis protein | PIR:B81307 | 2,00E-71 |
| SEQ. ID. No.623 | ORF2119 | 1285060 | 1283591 | argininosuccinate lyase | TREMBL:SCL24_6 | 1,00E-153 |
| SEQ. ID. No.624 | ORF2120 | 1286744 | 1285509 | argininosuccinate synthase | PIR:JC4548 | 1,00E-152 |
| SEQ. ID. No.625 | ORF2122 | 1287429 | 1286830 | arginine repressor | TREMBL:SCL24_12 | 3,00E-28 |
| SEQ. ID. No.626 | ORF2123 | 1288301 | 1287339 | omithine carbamoyltransferase | PIR:C70621 | 6,00E-96 |
| SEQ. ID. No.627 | ORF2125 | 1289640 | 1288348 | acetonitrile aminotransferase | TREMBL:SCL24_13 | 1,00E-75 |
| SEQ. ID. No.628 | ORF2126 | 1290586 | 1289633 | acetylglutamate kinase | PIR:A70621 | 2,00E-84 |
| SEQ. ID. No.629 | ORF2127 | 1291833 | 1290661 | glutamate N-acetyltransferase | PIR:H70620 | 2,00E-85 |
| SEQ. ID. No.630 | ORF2128 | 1292924 | 1291833 | N-acetyl-gamma-glutamyl-phosphate reductase | PIR:T36815 | 3,00E-80 |
| SEQ.ID. No.631 | ORF2133 | 1296349 | 1293743 | phenylalanyl-tRNA synthetase beta chain | PIR:T36829 | 1,00E-171 |
| SEQ. ID. No.632 | ORF2135 | 1297424 | 1296360 | phenylalanyl-tRNA synthetase alpha chain | PIR:T36830 | 1,00E-87 |
| SEQ. ID. No.633 | ORF2137 | 1298362 | 1297481 | rRNA methylase | PIR:T36832 | 1,00E-32 |
| SEQ. ID. No.634 | ORF2141 | 1300779 | 1299379 | cation ABC transporter (ATP-binding protein) | PIR:H69858 | 3,00E-70 |
| SEQ. ID. No.635 | ORF2145 | 1303117 | 1301753 | peptidase | PIR:G70870 | 3,00E-92 |
| SEQ. ID. No.636 | ORF2150 | 1303979 | 1305466 | dihydrolipoamide dehydrogenase | PIR:JC4089 | 1,00E-72 |
| SEQ. ID. No.637 | ORF2154 | 1306655 | 1308088 | glutamine synthetase I | TREMBL:CGGLNA_1 | 0 |
| SEQ. ID. No.638 | ORF2159 | 1310751 | 1310080 | GALACTOSIDE O-ACETYLTRANSFERASE | SWISSPROT:THGA_LACLA | 4,00E-47 |
| SEQ.ID. No.639 | ORF2161 | 1311605 | 1310916 | endonuclease III related protein | PIR:A64479 | 2,00E-30 |
| SEQ.ID. No.640 | ORF2162 | 1312971 | 1311616 | Unknown | TREMBLNEW:AP001514_169 | 7,00E-53 |
| SEQ.ID. No.641 | ORF2166 | 1315626 | 1313032 | excinuclease ABC chain A | TREMBL:SCM10_6 | 1,00E-124 |
| SEQ. ID. No.642 | ORF2186 | 1323905 | 1322865 | alcohol dehydrogenase (Zn-dependent) | PIR:T44975 | 6,00E-81 |
| SEQ.ID. No.643 | ORF2201 | 1330127 | 1328892 | translation elongation factor EF-Tu | PIR:D26956 | 1,00E-159 |
| SEQ. ID. No.644 | ORF2203 | 1332384 | 1330264 | translation elongation factor EF-G | PIR:E70827 | 0 |
| SEQ. ID. No.645 | ORF2207 | 1335090 | 1334032 | sodium-dependent transporter | PIR:E69902 | 4,00E-70 |
| SEQ. ID. No.646 | ORF2211 | 1335424 | 1336674 | low specificity L-threonine aldolase | PIR:T02833 | 3,00E-68 |
| SEQ. ID. No.647 | ORF2215 | 1337984 | 1336827 | polysaccharide biosynthesis protein | PIR:D71867 | 1,00E-60 |
| SEQ.ID. No.648 | ORF2217 | 1338152 | 1339492 | phosphoribosylglycinamide formyltransferase 2 | TREMBLNEW:AE004794_3 | 1,00E-100 |
| SEQ.ID. No.649 | ORF2220 | 1339888 | 1340637 | phosphoribosylaminoimidazole succinocarboxamide synthetase | TREMBLNEW:AP001509_64 | 7,00E-68 |
| SEQ.ID. No.650 | ORF2223 | 1340703 | 1344434 | phosphoribosylformylglycinamidine synthase | PIRNEW:A82272 | 6,00E-45 |
| SEQ.ID. No.651 | ORF2225 | 1344328 | 1345869 | lipase/esterase | PIR:C69464 | 1,00E-28 |
| SEQ.ID. No.652 | ORF2239 | 1350691 | 1351665 | potassium channel, beta subunit | PIR:A75289 | 5,00E-71 |
| SEQ. ID. No.653 | ORF2241 | 1353512 | 1351920 | Unknown | TREMBL:AF188935_69 | 2,00E-30 |
| SEQ. ID. No.654 | ORF2242 | 1353671 | 1355170 | adenosylhomocysteinase | PIR:B72649 | 3,00E-59 |
| SEQ. ID. No.655 | ORF2250 | 1358226 | 1359185 | amino acid ABC transporter, permease protein | PIR:H69278 | 3,00E-23 |
| SEQ. ID. No.656 | ORF2251 | 1359185 | 1359967 | glutamine transporl protein | PIR:H69334 | 7,00E-67 |
| SEQ. ID. No.657 | ORF2253 | 1360374 | 1361882 | amidophosphoribosyltransferase | PIRNEW:T51702 | 0 |
| SEQ. ID. No.658 | ORF2254 | 1361951 | 1363039 | phosphoribosylaminoimidazole synthetase | TREMBLNEW:AP001509_69 | 1,00E-117 |
| SEQ. ID. No.659 | ORF2256 | 1363069 | 1364334 | phosphoribosylamine-glycine ligase | TREMBL:LLJ000883_2 | 1,00E-130 |
| SEQ. ID. No.660 | ORF2258 | 1366348 | 1364714 | aldehyde dehydrogenase (NAD(P)+) | SWISSPROT:DHAP_HUMAN | 1,00E-83 |
| SEQ. ID. No.661 | ORF2260 | 1368544 | 1366472 | Unknown | PIR:T00092 | 2,00E-66 |
| SEQ. ID. No.662 | ORF2263 | 1369728 | 1368808 | Unknown | PIR:D70507 | 1,00E-22 |
| SEQ.ID. No.663 | ORF2265 | 1370680 | 1370243 | metal uptake regulation protein | TREMBL:SCC121_11 | 5,00E-31 |
| SEQ.ID. No.664 | ORF2266 | 1371855 | 1370680 | phospboribosylaminoinldazole carboxylase | TREMBL:LLJ000883_4 | 1,00E-102 |
| SEQ.ID. No.665 | ORF2269 | 1372345 | 1371842 | phosphoribosylaminoimidazole carboxylase | PIR:DEBSPE | 4,00E-52 |
| SEQ.ID. No.666 | ORF2270 | 1373433 | 1372444 | NADPH:quinone oxidoreductase | PIRNEW:T51766 | 1,00E-42 |
| SEQ.ID. No.667 | ORF2271 | 1373472 | 1375793 | 1-deoxyxylulose-5-phosphate synthase | PIR:E70528 | 3,00E-64 |
| SEQ.ID. No.668 | ORF2277 | 1379299 | 1378547 | ABC transporter, ATP-binding protein | PIR:B69377 | 3,00E-56 |
| SEQ. ID. No.669 | ORF2283 | 1382045 | 1380999 | transcriptional regulator | SWISSPROT:Y143_HAEIN | 2,00E-24 |
| SEQ. ID. No.670 | ORF2284 | 1381963 | 1384269 | arabinosidase | TREMBL:BO15178_1 | 3,00E-65 |
| SEQ. ID. No.671 | ORF2288 | 1385925 | 1384741 | Unknown | PIR:B64819 | 7,00E-24 |
| SEQ. ID. No.672 | ORF2292 | 1387407 | 1386478 | Asparaginase | PIR:D64820 | 3,00E-49 |
| SEQ. ID. No.673 | ORF2294 | 1389427 | 1387766 | aminotransferase | PIR:E75208 | 5,00E-67 |
| SEQ.ID. No.674 | ORF2297 | 1390405 | 1389488 | hisH-like amidotransferase | TREMBL:SCL2_12 | 4,00E-38 |
| SEQ.ID. No.675 | ORF2299 | 1391180 | 1390212 | superoxide-inducible protein | PIR:F64173 | 1,00E-113 |
| SEQ.ID. No.676 | ORF2300 | 1393384 | 1391279 | DNA primase | TREMBL:AF027507_3 | 1,00E-125 |
| SEQ.ID. No.677 | ORF2301 | 1394975 | 1393551 | deoxyguanosinetriphosphate triphosphohydrolase | TREMBL:SC7A8_9 | 1,00E-108 |
| SEQ.ID. No.678 | ORF2302 | 1396385 | 1395030 | alanine racemase | PIR:T35574 | 8,00E-73 |
| SEQ. ID. No.679 | ORF2304 | 1396598 | 1398031 | amino acid transporter | PIR:E69825 | 1,00E-145 |
| SEQ.ID. No.680 | ORF2307 | 1399425 | 1399009 | Unknown | PIR:T43793 | 1,00E-24 |
| SEQ.ID. No.681 | ORF2309 | 1401568 | 1399616 | DNA helicase recQ | PIR:F69901 | 1,00E-117 |
| SEQ. ID. No.682 | ORF2312 | 1404774 | 1403593 | CYSTATHIONINE GAMMA-SYNTHASE | SWISSPROT:METB_MYCLE | 1,00E-126 |
| SEQ.ID. No.683 | ORF2313 | 1406092 | 1404869 | cystathionine beta-synthase | TREMBLNEW:SCE25_18 | 1,00E-110 |
| SEQ. ID. No.684 | ORF2316 | 1407402 | 1406422 | oligopeptide transport protein | PIR:S75333 | 1,00E-43 |
| SEQ. ID. No.685 | ORF2318 | 1407988 | 1407200 | oligopeptide transport ATP-binding protein | PIR:G75026 | 3,00E-47 |
| SEQ. ID. No.686 | ORF2319 | 1409343 | 1407988 | PEPTIDE ABC TRANSPORTER PERMEASE | SWISSPROT:Y4TQ_RHISN | 3,00E-32 |
| SEQ. ID. No.687 | ORF2322 | 1409861 | 1408887 | oligopeptide | PIR:G64820 | 5,00E-35 |
| SEQ. ID. No.688 | ORF2324 | 1411545 | 1409929 | probable oligopeptide binding protein | PIR:B71130 | 7,00E-39 |
| SEQ. ID. No.689 | ORF2326 | 1411671 | 1412438 | Unknown | TREMBLNEW:AP001507_86 | 1,00E-46 |
| SEQ.ID. No.690 | ORF2327 | 1413877 | 1412504 | solute-binding lipoprotein | TREMBL:SC6D11_4 | 6,00E-79 |
| SEQ. ID. No.691 | ORF2329 | 1415381 | 1414041 | solute-binding lipoprotein | TREMBL:SC6D11_4 | 1,00E-104 |
| SEQ. ID. No.692 | ORF2333 | 1417065 | 1415716 | solute-binding protein/sugar-binding protein | TREMBL:SC6D11_4 | 2,00E-91 |
| SEQ. ID. No.693 | ORF2334 | 1417225 | 1418922 | alpha-L-arabinofuranosidase | TREMBLNEW:AP001513_134 | 1,00E-124 |
| SEQ. ID. No.694 | ORF2335 | 1420082 | 1419048 | transcription regulator, LacI family | TREMBLNEW:SCG22_12 | 9,00E-61 |
| SEQ. ID. No.695 | ORF2337 | 1422258 | 1420186 | beta-galactosidase | TREMBL:SC6D11_3 | 0 |
| SEQ.ID. ID. No.696 | ORF2341 | 1423483 | 1422476 | ABC transporter sugar permease | TREMBL:SC6D11_5 | 1,00E-116 |
| SEQ. ID. No.697 | ORF2342 | 1424471 | 1423542 | ABC-type sugar transport systems | TREMBL:SC6D11_6 | 1,00E-77 |
| SEQ. ID. No.698 | ORF2343 | 1424688 | 1425695 | transcription regulator, LacI family | TREMBL:SC6D11_7 | 2,00E-68 |
| SEQ. ID. No.699 | ORF2346 | 1426592 | 1427404 | Unknown | SWISSPROT:Y4ME_RHISN | 4,00E-25 |
| SEQ. ID. No.700 | ORF2347 | 1428151 | 1427453 | Unknown | PIR:F70533 | 9,00E-45 |
| SEQ. ID. No.701 | ORF2350 | 1430120 | 1428231 | glucosamine-fructose-6-phosphate aminotransferase - | PIR:T35569 | 0 |
| SEQ.ID. No.702 | ORF2352 | 1431192 | 1430365 | glutamine transport protein | TREMBL:AF104994_3 | 1,00E-75 |
| SEQ.ID. No.703 | ORF2354 | 1432191 | 1431211 | amino acid ABC transporter, peimease protein | TREMBLNEW:SC27G11_21 | 2,00E-50 |
| SEQ.ID. No.704 | ORF2356 | 1433263 | 1432325 | periplasmic amino acid-binding protein | TREMBLNEW:SC27G11_22 | 7,00E-36 |
| SEQ. ID. No.705 | ORF2357 | 1434461 | 1433400 | Periplasmic amino acid binding protein | TREMBLNEW:SC27G11_22 | 2,00E-42 |
| SEQ. ID. No.706 | ORF2358 | 1435112 | 1434516 | SSRA-BINDING PROTEIN | TREMBL:SCE59_25 | 5,00E-45 |
| SEQ. ID. No.707 | ORF2362 | 1437540 | 1436620 | cell division protein (ftsX) | TIEEMBL:SCES9_27 | 3,00E-40 |
| SEQ. ID. No.708 | ORF2363 | 1438721 | 1437555 | cell division ATP-binding protein | TREMBL:SCE59_28 | 6,00E-71 |
| SEQ. ID. No.709 | ORF2364 | 1439854 | 1438733 | translation releasing factor RF-2 | TREMBL:SCE59_31 | 1,00E-126 |
| SEQ. ID. No.710 | ORF2369 | 1442368 | 1441718 | polypeptide deformylase | TREMBL:SCM1_16 | 4,00E41 |
| SEQ.ID. No.711 | ORF2371 | 1443778 | 1442396 | phospho-sugar mutase | PIR:T35565 | 1,00E-117 |
| SEQ. ID. No.712 | ORF2377 | 1448432 | 1445826 | ALANINE AMINOPEPTIDASE | TREMBL:SC8E4A_13 | 0 |
| SEQ.ID. No.713 | ORF2378 | 1450387 | 1448477 | D-ala-D-ala dipeptidase | PIR:T35845 | 0 |
| SEQ.ID. No.714 | ORF2380 | 1451314 | 1450412 | dihydrodipicolinate synthase | PIR:H70879 | 1,00E-64 |
| SEQ.ID. No.715 | ORF2381 | 1452232 | 1451480 | dihydrodipicolinate reductase | PIR:D70882 | 1,00E-57 |
| SEQ. ID. No.716 | ORF2384 | 1453887 | 1452571 | transport protein | PIR:T35894 | 8,00E41 |
| SEQ. ID. No.717 | ORF2388 | 1458334 | 1454102 | ATP-dependent DNA helicase | PIR:G70951 | 2,00E-61 |
| SEQ. ID. No.718 | ORF2399 | 1462569 | 1463987 | probable serine/threonine-specific protein kinase | PIR:A70652 | 8,00E-35 |
| SEQ. ID. No.719 | ORF2409 | 1469810 | 1471522 | methanol dehydrogenase regulatory protein | TREMBLNEW:AP001509_42 | 3,00E-84 |
| SEQ. ID. No.720 | ORF2422 | 1481598 | 1477564 | DNA-directed RNA polymerase | PIR:G70535 | 0 |
| SEQ.ID. No.721 | ORF2423 | 1485329 | 1481769 | DNA-directed RNA polymerase | PIR:F70535 | 0 |
| SEQ.ID. No.722 | ORF2425 | 1487257 | 1486274 | adenine glycosylase | PIR:T36366 | 1,00E-60 |
| SEQ. ID. No.723 | ORF2428 | 1487191 | 1487937 | rRNA methylase | PIR:E70971 | 2,00E-40 |
| SEQ. ID. No.724 | ORF2432 | 1489611 | 1488250 | Unknown | PIRNEW:E81818 | 1,00E-161 |
| SEQ. ID. No.725 | ORF2434 | 1491352 | 1490105 | galactokinase | TREMBLNEW:SCE66_15 | 6,00E-77 |
| SEQ. ID. No.726 | ORF2436 | 1492631 | 1491372 | galactose-1-phosphate uridylyltransferase | TREMBLNEW:SCE66_17 | 5,00E-68 |
| SEQ. ID. No.727 | ORF2438 | 1493508 | 1492615 | Transcription rgulator DeoR family | TREMBLNEW:SCG20A_6 | 1,00E-49 |
| SEQ. ID. No.728 | ORF2441 | 1493917 | 1494930 | probable dihydroorotate oxidase (EC 1.3.3.1) - | PIR:S76039 | 2,00E-44 |
| SEQ.ID. No.729 | ORF2443 | 1496474 | 1495104 | NADH-dependent flavin oxidoreductase | PIR:H75303 | 5,00E-85 |
| SEQ.ID. No.730 | ORF2446 | 1498981 | 1496669 | penicillin-binding protein | PIR:T36565 | 2,00E-61 |
| SEQ.ID. No.731 | ORF2449 | 1499804 | 1499088 | transcription regulator | PIR:T36556 | 3,00E-50 |
| SEQ.ID. No.732 | ORF2450 | 1501128 | 1500046 | lipoate protein ligase | PIR:T46683 | 3,00E-67 |
| SEQ.ID. No.733 | ORF2452 | 1502217 | 1501189 | 3-ISOPROPYLMALATE DEHYDROGENASE | SWISSPROT:LEU3_CORGL | 1,00E-107 |
| SEQ. ID. No.734 | ORF2453 | 1504798 | 1502285 | peptidase | PIR:A70709 | 2,00E-93 |
| SEQ. ID. No.735 | ORF2459 | 1507988 | 1506699 | Unknown | PIR:E70951 | 1,00E-34 |
| SEQ. ID. No.736 | ORF2465 | 1509811 | 1510179 | thioredoxin | TREMBL:SCM1_18 | 9,00E-22 |
| SEQ. ID. No.737 | ORF2472 | 1514695 | 1513262 | capsular polysaccharide biosynthesis (epsB-like) | PIR:G70066 | 3,00E-27 |
| SEQ.ID. No.738 | ORF2479 | 1517945 | 1519261 | abortive phage resistance protein | TREMBL:LLU94520_1 | 2,00E-23 |
| SEQ.ID. No.739 | ORF2489 | 1524075 | 1523317 | transposase | PIR:T14971 | 2,00E-37 |
| SEQ.ID. No.740 | ORF2494 | 1525555 | 1524539 | transposase | TREMBLNEW:AP001520_222 | 5,00E-57 |
| SEQ.ID. No.741 | ORF2499 | 1529926 | 1528511 | dTDP-glucose-4,6-dehydratase | TREMBL:AF030360_3 | 1,00E-113 |
| SEQ.ID. No.742 | ORF2500 | 1529909 | 1531084 | UDP-galactopyranose mutase | PIR:E70888 | 1,00E-124 |
| SEQ.ID. No.743 | ORF2503 | 1532593 | 1533468 | 2-hydroxyhepta-2,4-diene-1,7-dioate isomerase | PIR:T29076 | 5,00E-53 |
| SEQ.ID. No.744 | ORF2505 | 1534592 | 1533720 | transcriptional regulator | TREMBLNEW:AE004771_5 | 8,00E-26 |
| SEQ.ID. No.745 | ORF2507 | 1534838 | 1536388 | histidine ammonia-lyase | PIR:JC1172 | 1,00E-149 |
| SEQ. ID. No.746 | ORF2509 | 1536610 | 1539276 | endopeptidase ClpB (ATP-binding chain B) | PIR:T36551 | 0 |
| SEQ. ID. No.747 | ORF2512 | 1539396 | 1540532 | glutamyl-tRNA synthetase-related protein | PIR:C81210 | 3,00E-34 |
| SEQ. ID. No.748 | ORF2515 | 1542345 | 1541869 | Unknown | PIR:H70090 | 1,00E-45 |
| SEQ. ID. No.749 | ORF2517 | 1543019 | 1542396 | uracil phosphoribosyltransferase | TREMBLNEW:AP001519_279 | 1,00E-52 |
| SEQ. ID. No.750 | ORF2519 | 1543338 | 1544645 | Unknown | TREMBL:AF179376_1 | 9,00E-57 |
| SEQ. ID. No.751 | ORF2523 | 1546932 | 1545721 | MutT 1-like | PIR:F70673 | 2,00E-27 |
| SEQ. ID. No.752 | ORF2528 | 1549312 | 1547078 | polyphosphate kinase | PIR:E70673 | 0 |
| SEQ. ID. No.753 | ORF2532 | 1552013 | 1551240 | transcription regulator | PIR:T29082 | 3,00E-82 |
| SEQ. ID. No. 754 | ORF2535 | 1552331 | 1553731 | 3-isopropylmalate dehydratase | PIR:T29083 | 1,00E-178 |
| SEQ. ID. No.755 | ORF2536 | 1553817 | 1554506 | 3-isopropylmalate dehydratase | PIR:T45426 | 9,00E-64 |
| SEQ. ID. No.756 | ORF2542 | 1556552 | 1557664 | Dihydroorotate oxidase | PIR:A23559 | 2,00E-41 |
| SEQ. ID. No.757 | ORF2544 | 1559219 | 1557876 | NADH oxidase | PIR:S26965 | 1,00E-114 |
| SEQ. ID. No.758 | ORF2546 | 1559468 | 1560790 | UDP-N-acetylglucosamine transferase | TREMBL:SCE59_8 | 1,00E-162 |
| SEQ. ID. No.759 | ORF2547 | 1562095 | 1560845 | Aminotransferase | PIR:D70849 | 4,00E-57 |
| SEQ. ID. No.760 | ORF2549 | 1563499 | 1562192 | lysR type transcription regulator | PIR:T35595 | 7,00E-31 |
| SEQ.ID. No.761 | ORF2552 | 1564473 | 1563211 | Multidrug resistance protein | PIR:D72388 | 7,00E-27 |
| SEQ. ID. No.762 | ORF2556 | 1565361 | 1567220 | arginine--tRNA ligase | PIR:H70772 | 1,00E-147 |
| SEQ. ID. No.763 | ORF2557 | 1567226 | 1568815 | Diaminopimelate decarboxylase | PIR:S03827 | 2,00E-56 |
| SEQ. ID. No.764 | ORF2559 | 1568979 | 1570292 | Homoserine dehydrogenase | PIR:B70773 | 1,00E-101 |
| SEQ.ID. No.765 | ORF2561 | 1570402 | 1571511 | HOMOSERINE KINASE | SWISSPROT:KHSE_BRELA | 1,00E-34 |
| SEQ.ID. No.766 | ORF2562 | 1571651 | 1573096 | maf-like protein | PIR:S76166 | 6,00E-29 |
| SEQ. ID. No.767 | ORF2565 | 1573295 | 1574365 | ABC transporter ATP-binding protein | TREMBLNEW:SCK7_8 | 8,00E-60 |
| SEQ.ID. No.768 | ORF2572 | 1576911 | 1578242 | succinyl-diaminopimelate desuccinylase | TREMBLNEW:SCP8_2 | 5,00E-87 |
| SEQ.ID. No.769 | ORF2575 | 1578582 | 1581590 | cytosolic axial filament protein | TREMBL:SCC88_10 | 1,00E-158 |
| SEQ.ID. No.770 | ORF2578 | 1582393 | 1584081 | GTP-binding protein obg | TREMBL:SGD916_3 | 1,00E-133 |
| SEQ.ID. No.771 | ORF2579 | 1584085 | 1585215 | Glutamate 5-kinase | TREMBL:SCC123_25 | 1,00E-78 |
| SEQ.ID. No.772 | ORF2581 | 1585309 | 1586511 | aspartate transaminase | PIR:T11786 | 1,00E-132 |
| SEQ.ID. No.773 | ORF2584 | 1587015 | 1587905 | Transcription antitermination factor | PIR.S54717 | 7,00E-48 |
| SEQ. ID. No.774 | ORF2587 | 1588618 | 1589307 | Ribosomal protein L1 | PIR:F70613 | 3,00E-71 |
| SEQ. ID. No.775 | ORF2605 | 1596209 | 1595385 | Dehydrogenase | PIR:D69988 | 2,00E-78 |
| SEQ. ID. No.776 | ORF2606 | 1596341 | 1597789 | Xylulokinase | PIR:S18562 | 1,00E-54 |
| SEQ. ID. No.777 | ORF2608 | 1598182 | 1599594 | Histidinol dehydrogenase | PIR:S26209 | 1,00E-122 |
| SEQ. ID. No.778 | ORF2609 | 1599594 | 1600751 | Histidinol-phosphate transaminase | PIR:JQ0637 | 1,00E-90 |
| SEQ. ID. No.779 | ORF2610 | 1600840 | 1601436 | Imidazole glycerol-phosphate dehydratase | PIR:C70544 | 4,00E-65 |
| SEQ. ID. No.780 | ORF2613 | 1602268 | 1602912 | Glutamine amidotransferase | PIR:JQ0640 | 3,00E-55 |
| SEQ. ID. No.781 | ORF2616 | 1602985 | 1603707 | N-(5'-phospho-D-ribosylformimino)-5-amino-1-(5"-phosphoribosyl)-4-imidazolecalboxamide isomerase | PIR:JQ0641 | 2,00E-79 |
| SEQ. ID. No.782 | ORF2617 | 1605571 | 1603817 | Unknown | PIR:T34645 | 6,00E-96 |
| SEQ. ID. No.783 | ORF2618 | 1605396 | 1606730 | Glutamine synthetase | TREMBL:SC1G2_3 | 1,00E-175 |
| SEQ. ID. No.784 | ORF2626 | 1612686 | 1608481 | ATP-dependent helicase | TREMBL:SCD25_28 | 0 |
| SEQ. ID. No.785 | ORF2628 | 1613260 | 1612607 | Unknown | PIR:F69751 | 4,00E-31 |
| SEQ. ID. No.786 | ORF2630 | 1613419 | 1614921 | ATP/GTP-binding protein | PIR:T35116 | 1,00E-117 |
| SEQ. ID. No.787 | ORF2631 | 1615084 | 1616064 | L-lactate dehydrogenase | PIR:JQ0183 | 0 |
| SEQ. ID. No.788 | ORF2632 | 1617147 | 1616212 | cation efflux system protein | PIR:T35276 | 7,00E-41 |
| SEQ. ID. No.789 | ORF2635 | 1618038 | 1617316 | SOS response regulator | TREMBL:SCAJ4870_2 | 3,00E-62 |
| SEQ. ID. No.790 | ORF2637 | 1618556 | 1619029 | Unknown | TREMBL:SCAJ4870_3 | 3,00E-52 |
| SEQ.ID. ID. No.791 | ORF2638 | 1620366 | 1619170 | D-3-phosphoglycerate dehydrogenase | PIRNEW:C82072 | 1,00E-111 |
| SEQ. ID. No.792 | ORF2639 | 1622656 | 1620380 | helicase protein | TREMBL:SC6A11_15 | 1,00E-113 |
| SEQ. ID. No.793 | ORF2641 | 1622989 | 1623507 | Unknow | TREMBLNEW:AP001516_5 | 2,00E-29 |
| SEQ. ID. No.794 | ORF2642 | 1623510 | 1624586 | Unknown | PIR:T34962 | 3,00E-79 |
| SEQ.ID. No.795 | ORF2647 | 1625042 | 1626841 | cell division protein | TREMBL:AF123319_2 | 9,00E-70 |
| SEQ. ID. No. 796 | ORF2652 | 1627792 | 1629240 | UDP-N-acetylmuramoylalanyl-D-glutamyl-2,6-diaminopimelate-D-alanyl-alanyl ligase | PIR:T34958 | 6,00E-75 |
| SEQ. ID. No.797 | ORF2653 | 1629288 | 1630391 | phospho-N-acetylmuramoyl-pentapeptide-transferase | PIR:T34957 | 3,00E-80 |
| SEQ. ID. No.798 | ORF2655 | 1630449 | 1631891 | UDP-N-acetylmuramoylalanine-D-glutamate ligase | PIR:T34956 | 1,00E-81 |
| SEQ. ID. No.799 | ORF2657 | 1631737 | 1633095 | cell division protein ftsW | PIR:T34955 | 6,00E-41 |
| SEQ. ID. No.800 | ORF2661 | 1633114 | 1634292 | UDP-N-acetylglucosamine-N-acetylmuramyl-(pentapeptide) pyrophosphoryl-undecaprenol N-acetylglucosamine transferase | PIR:T34954 | 1,00E-87 |
| SEQ. ID. No.801 | ORF2662 | 1634396 | 1635931 | UDP-N-acetylmuramoyl-L-alanine ligase | PIR:T35852 | 2,00E-78 |
| SEQ. ID. No.802 | ORF2671 | 1638807 | 1638253 | Unknown | PIRNEW:F82517 | 1,00E-20 |
| SEQ. ID. No.803 | ORF2674 | 1639338 | 1639811 | Unknown | TREMBLNEW:AF276071_1 | 3,00E-34 |
| SEQ. ID. No.804 | ORF2676 | 1639975 | 1643091 | alpha-mannosidase | TREMBL:SCM11_3 | 0 |
| SEQ.ID. No.805 | ORF2677 | 1642913 | 1646362 | alpha-mannosidase | TREMBL:SCM11_3 | 0 |
| SEQ. ID. No.806 | ORF2678 | 1646635 | 1649736 | alpha-mannosidase | TREMBL:SCM11_3 | 0 |
| SEQ. ID. No.807 | ORF2685 | 1649935 | 1651203 | solute-binding protein/sugar-binding protein | TREMBL:SCM11_7 | 3,00E-91 |
| SEQ. ID. No.808 | ORF2686 | 1651209 | 1652144 | lactose transport system permease protein | TREMBL:SCM11_6 | 6,00E-70 |
| SEQ. ID. No.809 | ORF2687 | 1652147 | 1653001 | sugar ABC transporter, permease protein | TREMBL:SCM11_5 | 5,00E-61 |
| SEQ. ID. No.810 | ORF2688 | 1653018 | 1654304 | Unknown | TREMBL:SCM11_4 | 1,00E-101 |
| SEQ.ID. No.811 | ORF2689 | 1654432 | 1657071 | alpha-xylosidase | TREMBLNEW:SSO251975_1 | 8,00E45 |
| SEQ.ID. No.812 | ORF2691 | 1657008 | 1659932 | cation-transporting ATPase | TREMBLNEW:AP001509_223 | 1,00E-177 |
| SEQ.ID. No.813 | ORF2693 | 1660231 | 1661334 | transcription regulator, LacI family | TREMBL:SCM11_8 | 2,00E-59 |
| SEQ.ID. No.814 | ORF2695 | 1662757 | 1661462 | Unknown | PIR:S40824 | 6,00E-83 |
| SEQ.ID. No.815 | ORF2697 | 1664059 | 1662764 | unknown | TREMBLNEW:AP001509_228 | 1,00E-108 |
| SEQ.ID. No.816 | ORF2701 | 1666453 | 1665242 | transcriptional regulator, ROK family | PIRNEW:A82130 | 3,00E-36 |
| SEQ. ID. No.817 | ORF2704 | 1666680 | 1667591 | glucose kinase | PIR:B69632 | 9,00E-26 |
| SEQ. ID. No.818 | ORF2706 | 1668868 | 1667747 | Transcriptional regulators of NagC/XylR family | TREMBLNEW:AP001516_187 | 4,00E-31 |
| SEQ. ID. No.819 | ORF2709 | 1669206 | 1670015 | glucosamine 6-phosphate isomerase | TREMBLNEW:SC7E4_33 | 6,00E-85 |
| SEQ. ID. No.820 | ORF2710 | 1669981 | 1671354 | N-acetylglucosamine-6-phosphate deacetylase | PIR:C70845 | 7,00E-55 |
| SEQ. ID. No.821 | ORF2713 | 1671637 | 1673268 | oligopeptide binding protein | TREMBL:SCI41_38 | 9,00E-86 |
| SEQ. ID. No.822 | ORF2715 | 1673436 | 1674524 | oligopeptide ABC transporter, permease protein | PIR:A72289 | 3,00E-48 |
| SEQ. ID. No.823 | ORF2719 | 1674646 | 1675695 | dipeptide ABC transporter, permease protein (dppC) | PIR:H69470 | 7,00E-33 |
| SEQ.ID. No. 824 | ORF2722 | 1675702 | 1677408 | ABC transporterATP-binding protein | TREMBL:ECD720_13 | 1,00E-116 |
| SEQ. ID. No.825 | ORF2724 | 1679626 | 1678034 | X-Pro aminopeptidase | PIR:JN0491 | 1,00E-108 |
| SEQ. ID. No.826 | ORF2729 | 1682035 | 1683621 | folylpolyglutamate synthase | TREMBL:SCC88_25 | 7,00E-99 |
| SEQ. ID. No.827 | ORF2730 | 1683685 | 1687359 | chromosome segregation SMC protein | PIR:T35661 | 0 |
| SEQ. ID. No.828 | ORF2734 | 1690151 | 1688601 | UDP-N-acetylmuramoylalanyl-D-glutamyl-2,6-diaminopimelate ligase | PIR:T34959 | 1,00E-28 |
| SEQ. ID. No.829 | ORF2736 | 1690269 | 1691075 | RNA polymerase sigma factor | PIR:T42015 | 1,00E-37 |
| SEQ.ID. No.830 | ORF2740 | 1692608 | 1691667 | aldose epimerase family protein | PIR:540823 | 3,00E-29 |
| SEQ.ID. No.831 | ORF2742 | 1693689 | 1692736 | Unknown | PIR:S40823 | 1,00E-22 |
| SEQ.ID. No.832 | ORF2744 | 1693914 | 1694969 | penicillin tolerance protein LytB | PIR:D70898 | 9,00E-89 |
| SEQ.ID. No.833 | ORF2745 | 1695449 | 1695015 | Unknown | PIR:T35087 | 4,00E-26 |
| SEQ. ID. No.834 | ORF2747 | 1696673 | 1695618 | glyceraldehyde-3-phosphatedehydrogenase | PIR:T09633 | 1,00E-116 |
| SEQ. ID. No.835 | ORF2753 | 1699247 | 1699879 | translation initiation factor IF-3 | PIR:T36835 | 3,00E-47 |
| SEQ. ID. No.836 | ORF2754 | 1700110 | 1700490 | ribosomal protein L20 | PIR:T36833 | 4,00E-50 |
| SEQ. ID. No.837 | ORF2755 | 1700608 | 1701531 | integrase/recombinase | PIR:D70503 | 4,00E-70 |
| SEQ.ID. No.838 | ORF2757 | 1701662 | 1704505 | ABC transporter ATP binding subunit | TREMBL:U73183_1 | 1,00E-106 |
| SEQ. ID. No.839 | ORF2759 | 1704710 | 1705606 | partitioning or sporulation protein | PIR:T36875 | 2,00E-82 |
| SEQ. ID. No.840 | ORF2761 | 1705628 | 1706539 | Unknown | PIR:T36873 | 1,00E-40 |
| SEQ. ID. No.841 | ORF2762 | 1706555 | 1707268 | Unknown | PIR:T36872 | 6,00E46 |
| SEQ.ID. No.842 | ORF2764 | 1707403 | 1708227 | MutT/nudix family protein | PIR:S75926 | 1,00E-30 |
| SEQ.ID. No.843 | ORF2767 | 1708291 | 1709568 | quinolinate synfhetase A | TREMBLNEW:AP001511_109 | 1,00E-104 |
| SEQ.ID. No.844 | ORF2769 | 1709659 | 1711287 | L-aspartate oxidase | PIR:T36393 | 4,00E-83 |
| SEQ.ID. No.845 | ORF2770 | 1711294 | 1712184 | nicotinate-nucleotide pyrophosphorylase | PIRNEW:T51326 | 2,00E-60 |
| SEQ.ID. No.846 | ORF2772 | 1712178 | 1713434 | cysteine desulfurase | TREMBLNEW:AF276772_1 | 3,00E-61 |
| SEQ.ID. No.847 | ORF2776 | 1713469 | 1714815 | transport protein | PIR:C69757 | 4,00E-70 |
| SEQ.ID. No.848 | ORF2778 | 1715133 | 1717061 | GTP-binding translation elongation factor | PIR:F70556 | 0 |
| SEQ. ID. No.849 | ORF2784 | 1718661 | 1719725 | oxidoreductase | PIR:C70800 | 1,00E-24 |
| SEQ. ID. No.850 | ORF2788 | 1720232 | 1721302 | integrase/recombinase | TREMBL:MLCB250_62 | 2,00E-61 |
| SEQ.ID. No.851 | ORF2790 | 1721558 | 1723195 | peptide transport system secreted peptide-binding protein | PIR:C70789 | 1,00E-111 |
| SEQ. ID. No.852 | ORF2793 | 1723499 | 1724227 | peptidetransport system permease - | PIR:B70789 | 5,00E-67 |
| SEQ.ID. No.853 | ORF2795 | 1724443 | 1725444 | oligopeptide ABC transporter (permease) | TREMBLNEW:SC9E12_4 | 9,00E-71 |
| SEQ. ID. No.854 | ORF2797 | 1725470 | 1727476 | peptidetransport system ABC-transporter | PIR:H70788 | 1,00E-119 |
| SEQ. ID. No.855 | ORF2798 | 1727584 | 1728441 | exodeoxyribonuclease | TREMBL:SCE87_25 | 2,00E-58 |
| SEQ. ID. No.856 | ORF2803 | 1729382 | 1730137 | Unknown | PIR:T34826 | 1,00E-21 |
| SEQ. ID. No.857 | ORF2806 | 1730091 | 1731395 | RNA methyltransferase | PIR:T34574 | 4,00E-43 |
| SEQ. ID. No.858 | ORF2810 | 1732196 | 1734706 | integral membrane ATPase | PIR:T36308 | 1,00E-144 |
| SEQ.ID. No.859 | ORF2811 | 1734830 | 1737526 | aconitate hydratase | PIR:F70873 | 0 |
| SEQ. ID. No.860 | ORF2820 | 1742368 | 1741469 | two-component response regulator | TREMBL:SCAH10_19 | 1,00E-33 |
| SEQ. ID. No.861 | ORF2826 | 1744819 | 1745715 | Unknown | PIR:B64815 | 1,00E-36 |
| SEQ. ID. No.862 | ORF2831 | 1745858 | 1746871 | Unknown | TREMBLNEW:AE004781_1 | 5,00E42 |
| SEQ.ID. No.863 | ORF2832 | 1747749 | 1746910 | GTP-pyrophosphokinase | TREMBLNEW:AP001513_158 | 2,00E-43 |
| SEQ.ID. No.864 | ORF2833 | 1747791 | 1749230 | Unknown | PIR:T35107 | 1,00E-148 |
| SEQ.ID. No.865 | ORF2834 | 1749061 | 1750227 | tRNA delta(2)-isopentenylpyrophosphate | PIR:T35111 | 2,00E-72 |
| SEQ.ID. No.866 | ORF2838 | 1751033 | 1754131 | ftsK homolog - DNA translocase | PIR:T35683 | 0 |
| SEQ. ID. No.867 | ORF2840 | 1754287 | 1754943 | CDPdiacylglycerol--glycerol-3-phosphate 3-phosphatidyltransferase | PIR:572934 | 1,00E-27 |
| SEQ. ID. No.868 | ORF2847 | 1756712 | 1757902 | RecA protein | TREMBL:AF094756_1 | 0 |
| SEQ. ID. No. 869 | ORF2852 | 1759378 | 1760037 | Unknown | PIR:C70592 | 2,00E-23 |
| SEQ. ID. No.870 | ORF2853 | 1760202 | 1763093 | preprotein translocase secA | PIR:S71922 | 0 |
| SEQ. ID. No.871 | ORF2858 | 1764350 | 1765393 | anthranilate phosphoribosyltransferase | PIR:T35529 | 5,00E-68 |
| SEQ. ID. No. 872 | ORF2864 | 1766541 | 1767242 | acyltransferase | TREMBL:SCL24_2 | 2,00E-48 |
| SEQ.ID. No. 873 | ORF2865 | 1769570 | 1767300 | serine/threonine-specific protein kinase | TREMBLNEW:AF233851_2 | 5,00E-61 |
| SEQ. ID. No.874 | ORF2868 | 1770832 | 1769717 | polyprenyl synthase - like protein | TREMBL:SGCRTB_1 | 4,00E-20 |
| SEQ.ID. No.875 | ORF2872 | 1771868 | 1773289 | transcription initiation factor sigma | PIR:T35194 | 1,00E-140 |
| SEQ. ID. No.876 | ORF2873 | 1773350 | 1775665 | DNA gyrase chain B | PIR:T35196 | 1,00E-143 |
| SEQ.ID. No.877 | ORF2876 | 1777135 | 1778094 | ribokinase | TREMBLNEW:AE004621_9 | 5,00E-50 |
| SEQ.ID. No.878 | ORF2877 | 1778113 | 1782843 | ATP-dependent helicase | PIR:G64922 | 0 |
| SEQ.ID. No.879 | ORF2879 | 1786819 | 1783793 | DNA gyrase | PIR:T35872 | 0 |
| SEQ.ID. No.880 | ORF2880 | 1786749 | 1788035 | Unknown | PIR:T35880 | 2,00E-48 |
| SEQ.ID. No.881 | ORF2886 | 1789591 | 1790064 | deoxyuridine 5'-triphosphate nucleotidohydrolase | PIR:T34820 | 3,00E-42 |
| SEQ. ID. No.882 | ORF2888 | 1790180 | 1792522 | GTP pyrophosphokinase | PIR:S70687 | 0 |
| SEQ.ID. No.883 | ORF2890 | 1793527 | 1792646 | Transposase | TREMBL:ECIS1397_2 | 2,00E-46 |
| SEQ.ID. No.884 | ORF2895 | 1794657 | 1794112 | peptidylprolyl isomerase | PIRNEW:T51359 | 3,00E-35 |
| SEQ.ID. No.885 | ORF2901 | 1797008 | 1796076 | Unknown | PIR:E71174 | 3,00E-23 |
| SEQ. ID. No.886 | ORF2905 | 1799082 | 1799924 | phosphoglycerate mutase | PIR:F70685 | 2,00E-22 |
| SEQ. ID. No.887 | ORF2908 | 1800042 | 1800989 | transport protein | PIR:A75272 | 2,00E-50 |
| SEQ. ID. No.888 | ORF2909 | 1801007 | 1801930 | glutamate-binding periplasmic protein | PIR:T35146 | 1,00E-32 |
| SEQ.ID. No.889 | ORF2911 | 1801976 | 1804936 | leucyl-tRNA synthetase | TREMBL:SCC123_9 | 0 |
| SEQ. ID. No.890 | ORF2914 | 1805942 | 1807627 | competence-like protein | TREMBL:SCC123_5 | 2,00E-22 |
| SEQ. ID. No.891 | ORF2916 | 1807770 | 1809113 | Unknown | TREMBLNEW:AP001517_57 | 2,00E-37 |
| SEQ. ID. No.892 | ORF2920 | 1809279 | 1810151 | Unknown | TREMBL:SCC123_2 | 2,00E-35 |
| SEQ. ID. No.893 | ORF2921 | 1810175 | 1810738 | Unknown | PIR:T35576 | 1,00E-20 |
| SEQ.ID. No.894 | ORF2927 | 1812252 | 1813292 | O-sialoglycoprotein endopeptidase - | PIR:T35581 | 1,00E-105 |
| SEQ. ID. No.895 | ORF2930 | 1813962 | 1814894 | integrase/recombinase | PIR:B70965 | 3,00E-25 |
| SEQ. ID. No.896 | ORF2951 | 1824277 | 1822820 | Unknown | TREMBLNEW:SCK7_2 | 2,00E-22 |
| SEQ. ID. No.897 | ORF2957 | 1825368 | 1828001 | DNA topoisomerase III topB | PIR:H69724 | 9,00E-86 |
| SEQ.ID. No.898 | ORF2965 | 1830496 | 1829231 | type II site-specific deoxyribonuclease | PIR:A34919 | 2,00E-34 |
| SEQ.ID. No.899 | ORF2968 | 1831513 | 1830545 | site-specific DNA-methyltransferase (cytosine-specific) | PIR:XYECR2 | 2,00E-88 |
| SEQ.ID. No.900 | ORF2975 | 1835344 | 1833872 | Unknown | SWISSPROT:Y4ME_RHISN | 4,00E-33 |
| SEQ.ID. No.901 | ORF2984 | 1839447 | 1837642 | Unknown | PIR:T36167 | 7,00E-58 |
| SEQ.ID. No.902 | ORF3031 | 1863238 | 1858430 | aggregation protein precursor | PIR:H41662 | 5,00E-28 |
| SEQ.ID. No.903 | ORF3041 | 1866693 | 1866103 | plasmid partition protein | TREMBL:AF121000_6 | 1,00E-24 |
| SEQ.ID. No.904 | ORF3055 | 1871346 | 1872032 | Unknown | PIR:F70925 | 3,00E-20 |
| SEQ.ID. No.905 | ORF3059 | 1875342 | 1873996 | isocitrate dehydrogenase | PIR:B70846 | 1,00E-179 |
| SEQ.ID. No.906 | ORF3060 | 1875134 | 1876462 | IMP dehydrogenase | TREMBL:AB003154_2 | 1,00E-91 |
| SEQ.ID. No.907 | ORF3062 | 1877209 | 1879293 | long-chain-fatty-acid-CoA ligase | PIR:E70937 | 1,00E-111 |
| SEQ. ID. No.908 | ORF3063 | 1879303 | 1879788 | polypetide deformylase | PIR:C70631 | 2,00E-24 |
| SEQ. ID. No.909 | ORF3066 | 1880103 | 1880990 | ribosomal protein S2 | PIR:T34809 | 2,00E-87 |
| SEQ. ID. No.910 | ORF3067 | 1881072 | 1881920 | translation elongation factor EF-Ts | PIR:D70925 | 8,00E-53 |
| SEQ.ID. No.911 | ORF3069 | 1882097 | 1882834 | uridylate kinase | PIR:T34811 | 7,00E-74 |
| SEQ.ID. No.912 | ORF3071 | 1882914 | 1883462 | ribosome recycling factor | PIR:T35471 | 5,00E-55 |
| SEQ. ID. No.913 | ORF3072 | 1883386 | 1884471 | phosphatidate cytidylyltransferase | PIR:T35470 | 6,00E-27 |
| SEQ.ID. No.914 | ORF3074 | 1884685 | 1885851 | Unknown | PIR:T35453 | 1,00E-113 |
| SEQ. ID. No.915 | ORF3075 | 1886403 | 1885858 | pfpI like protease | TREMBLNEW:AE004833_9 | 3,00E-37 |
| SEQ. ID. No.916 | ORF3078 | 1886612 | 1887793 | ransposase | TREMBLNEW:AB032203_2 | 1,00E-48 |
| SEQ. ID. No.917 | ORF3083 | 1890162 | 1889584 | dCTP deaminase | PIR:T36613 | 4,00E-72 |
| SEQ. ID. No.918 | ORF3091 | 1895286 | 1893217 | Na+/H+ antiporter | PIR:E70040 | 2,00E-55 |
| SEQ. ID. No.919 | ORF3095 | 1895982 | 1898285 | alpha-galactosidase | TREMBL:AF124596_1 | 0 |
| SEQ. ID. No.920 | ORF3097 | 1899661 | 1898456 | transcriptional repressor | TREMBL:SCG11A_2 | 1,00E-21 |
| SEQ.ID. No.921 | ORF3098 | 1899592 | 1901121 | sugar transport system (sugar-binding protein) | PIR:B42400 | 4,00E-28 |
| SEQ. ID. No.922 | ORF3101 | 1901146 | 1902072 | membrane protein | PIR:C42400 | 2,00E-50 |
| SEQ. ID. No.923 | ORF3103 | 1902094 | 1902957 | multiple sugar-binding transport system permease protein | PIR:D42400 | 6,00E-48 |
| SEQ. ID. No.924 | ORF3107 | 1904376 | 1906193 | oligo-1,6-glucosidase | PIR:A41707 | 1,00E-163 |
| SEQ.ID. No.925 | ORF3108 | 1906482 | 1907726 | threonine dehydratase | PIR:D72386 | 8,00E-76 |
| SEQ.ID. No.926 | ORF3109 | 1908525 | 1907878 | SIR2 family transcription regulator | PIR:A72370 | 4,00E-31 |
| SEQ. ID. No.927 | ORF3128 | 1915770 | 1916519 | transcriptional regulator | TREMBL:CZA382_19 | 2,00E-20 |
| SEQ.ID. No.928 | ORF3129 | 1917495 | 1916632 | Unknown | TREMBL:SC4A7_14 | 6,00E-24 |
| SEQ.ID. No.929 | ORF3139 | 1921801 | 1923762 | acyl-CoA carboxylase complex A chain | TREMBL:AF126429_1 | 1,00E-129 |
| SEQ.ID. No.930 | ORF3140 | 1923758 | 1925377 | propionyl-CoA carboxylase complex B chain - | PIR:T42208 | 1,00E-150 |
| SEQ. ID. No.931 | ORF3141 | 1925419 | 1934934 | fatty-acid synthase | PIR:S55505 | 0 |
| SEQ. ID. No.932 | ORF3162 | 1944074 | 1942971 | gene: "tnpA ; product: "transposase"; | TREMBL:AF052750_3 | 1,00E-41 |
| SEQ. ID. No.933 | ORF3168 | 1944695 | 1947220 | endo-1,4-beta-xylanase xynD | PIR:H69735 | 1,00E-120 |
| SEQ. ID. No.934 | ORF3172 | 1947446 | 1950319 | endo-1,4-beta-xylanase | PIR:T30909 | 9,00E-95 |
| SEQ.ID. No.935 | ORF3181 | 1952286 | 1955024 | polyribonucleotide nucleotidyltransferase | PIR:T10932 | 0 |
| SEQ. ID. No.936 | ORF3184 | 1956848 | 1955457 | Unknown | TREMBLNEW:AP001509_46 | 2,00E-81 |
| SEQ. ID. No.937 | ORF3189 | 1958007 | 1958720 | ribosomal protein | PIR:S32238 | 6,00E-48 |
| SEQ. ID. No.938 | ORF3205 | 1966792 | 1966046 | 5-formyltetrahydrofolate cyclo-ligase | TREMBL:SCE87_34 | 2,00E-23 |
| SEQ. ID. No.939 | ORF3206 | 1966819 | 1967511 | Unknown | PIR:F70601 | 9,00E-24 |
| SEQ.ID. No.940 | ORF3211 | 1969192 | 1969482 | chaperonin groES | TREMBL:AF071828_1 | 1,00E-30 |
| SEQ.ID. No.941 | ORF3214 | 1971682 | 1970162 | cystathionine gamma-synthase homolog | PIR:T21246 | 1,00E-65 |
| SEQ.ID. No.942 | ORF3221 | 1972559 | 1973656 | UDP-N-acetylpyruvoylglucosamine reductase | PIR:E70743 | 2,00E-42 |
| SEQ.ID. No.943 | ORF3222 | 1973811 | 1975334 | cationic amino acid transporter- | PIR:G70593 | 1,00E-119 |
| SEQ. ID. No.944 | ORF3225 | 1975833 | 1977065 | aminotransferase | PIR:B70876 | 6,00E-55 |
| SEQ.ID. No.945 | ORF3226 | 1978441 | 1977098 | DNA-DAMAGE-INDUCIBLE PROTEIN PimpB/mucB/samB family protein | PIRNEW:A81861 | 8,00E-47 |
| SEQ.ID. No.946 | ORF3231 | 1980080 | 1981420 | Unknown | TREMBL:SCM10_29 | 2,00E-41 |
| SEQ.ID. No.947 | ORF3234 | 1981497 | 1982510 | dehydrogenase | PIRNEW:F82068 | 8,00E-30 |
| SEQ. ID. No.948 | ORF3243 | 1984135 | 1986297 | 4-ALPHA-GLUCANOTRANSFERASE | PIR:G70928 | 1,00E-139 |
| SEQ.ID. No.949 | ORF3247 | 1987170 | 1987658 | ribosomal protein | PIR:T35564 | 1,00E44 |
| SEQ.ID. No.950 | ORF3248 | 1990292 | 1987755 | glycosyl hydrolase | TREMBL:SC3D11_13 | 1,00E-137 |
| SEQ.ID. No.951 | ORF3250 | 1991482 | 1990292 | N-acetylglucosamine repressor | TREMBL:AB009593_3 | 5,00E-20 |
| SEQ.ID. No.952 | ORF3254 | 1991997 | 1994723 | ALCOHOL DEHYDROGENASE 2 | SWISSPROT:ADH2_ENTHI | 0 |
| SEQ.ID. No.953 | ORF3259 | 1996645 | 1997283 | ribosomal protein L3 | PIR:H70641 | 4,00E-51 |
| SEQ.ID. No.954 | ORF3260 | 1997293 | 1997946 | ribosomal protein L4 | PIR:A70642 | 3,00E-56 |
| SEQ.ID. No.955 | ORF3261 | 1997955 | 1998248 | ribosomal protein L23 | PIR:T45366 | 5,00E-25 |
| SEQ.ID. No.956 | ORF3262 | 1998288 | 1999115 | ribosomal protein L2 | PIR:C70642 | 1,00E-116 |
| SEQ. ID. No.957 | ORF3263 | 1999134 | 1999409 | ribosomal protein | PIR:T45368 | 2,00E-42 |
| SEQ. ID. No.958 | ORF3265 | 1999791 | 2000591 | ribosomal protein S3 | PIR:F70642 | 4,00E-74 |
| SEQ.ID. No.959 | ORF3266 | 2000601 | 2001017 | ribosomal protein L16 rplP | PIR:G70642 | 5,00E-48 |
| SEQ.ID. No.960 | ORF3270 | 2002331 | 2002900 | Ribosomal protein L5 | PIR:S29884 | 7,00E-68 |
| SEQ. ID. No.961 | ORF3273 | 2003596 | 2004132 | ribosomal protein L6 | PIR:S50001 | 1,00E-46 |
| SEQ. ID. No.962 | ORF3274 | 2004137 | 2004505 | ribosomal protein L18 | PIR:S29887 | 1,00E-37 |
| SEQ. ID. No.963 | ORF3276 | 2004535 | 2005233 | 30S RIBOSOMAL PROTEIN S5. | SWISSPROT:RS5_STRCO | 4,00E-50 |
| SEQ. ID. No.964 | ORF3279 | 2005430 | 2005879 | Ribosomal protein | PIR:S29890 | 8,00E43 |
| SEQ. ID. No.965 | ORF3280 | 2006156 | 2007490 | preprotein translocase | PIR:JC4288 | 1,00E-137 |
| SEQ. ID. No.966 | ORF3282 | 2007663 | 2008220 | adenylate kinase | PIR:S17070 | 2,00E46 |
| SEQ.ID. No.967 | ORF3288 | 2009848 | 2010840 | DNA-directed RNA polymerase alpha chain | PIR:F70565 | 1,00E-134 |
| SEQ.ID. No.968 | ORF3289 | 2010943 | 2011473 | ribosomal protein L17 | PIR:T35559 | 8,00E-36 |
| SEQ.ID. No.969. | ORF3290 | 2012469 | 2011561 | pseudouridylate synthase | PIR:T35560 | 6,00E-54 |
| SEQ. ID. No.970 | ORF3292 | 2012670 | 2014922 | Unknown | PIR:C75323 | 1,00E-102 |
| SEQ. ID. No.971 | ORF3294 | 2015132 | 2015929 | Unknown | PIR:F75328 | 4,00E-21 |
| SEQ. ID. No.972 | ORF3298 | 2016508 | 2018307 | alpha-L-arabinofuranosidase | PIR:S55274 | 1,00E-158 |
| SEQ.ID. No.973 | ORF3301 | 2018843 | 2019883 | Transcriptional regulators of the LacI family | TREMBLNEW:SCG22_12 | 1,00E-39 |
| SEQ. ID. No.974 | ORF3304 | 2020863 | 2021927 | transcription termination-antitermination factor | PIR:D70588 | 5,00E-78 |
| SEQ.ID. No.975 | ORF3305 | 2022200 | 2025061 | translation initiation factor IF-2 | PIR:B70694 | 0 |
| SEQ.ID. No.976 | ORF3306 | 2025217 | 2025687 | ribosome-binding factor A | PIR:T35987 | 1,00E-24 |
| SEQ. ID. No.977 | ORF3308 | 2025578 | 2026852 | tRNA pseudouridine synthase | PIR:T35986 | 4,00E-53 |
| SEQ. ID. No.978 | ORF3310 | 2026953 | 2028074 | probable riboflavin kinase (FAD synthetase) | PIR:T35984 | 8,00E-35 |
| SEQ. ID. No.979 | ORF3312 | 2029630 | 2028095 | DNA repair protein | PIR:T36362 | 1,00E-111 |
| SEQ. ID. No.980 | ORF3316 | 2031522 | 2030827 | ribose 5-phosphate isomerase | PIR:G69180 | 2,00E-35 |
| SEQ.ID. No.981 | ORF3320 | 2034810 | 2033179 | mercury(II) reductase | PIR:H64756 | 1,00E-57 |
| SEQ. ID. No.982 | ORF3327 | 2039100 | 2037427 | phosphoglucomutase | TREMBL:SC6D11_39 | 1,00E-180 |
| SEQ.ID. No.983 | ORF3328 | 2040740 | 2039190 | D-xylose proton-symporter | TREMBL:AF045552_3 | 1,00E-96 |
| SEQ. ID. No.984 | ORF3330 | 2041240 | 2043612 | glucose permease | TREMBL:AF045481_1 | 1,00E-120 |
| SEQ.ID. No.985 | ORF3331 | 2043633 | 2044469 | transcription antiterminator | PIR:S47216 | 9,00E-38 |
| SEQ. ID. No.986 | ORF3335 | 2045875 | 2047194 | seryl-tRNA synthase | PIR:T36067 | 1,00E-129 |
| SEQ. ID. No.987 | ORF3339 | 2048203 | 2049516 | sugar-binding protein | TREMBL:SCF91_20 | 3,00E-35 |
| SEQ. ID. No.988 | ORF3341 | 2049659 | 2050678 | ABC transporter sugar permease | TREMBL:SC6D11_6 | 2,00E-50 |
| SEQ.ID. No.989 | ORF3343 | 2050681 | 2051628 | ABC transporter sugar permease | TREMBL:SC6D11_5 | 3,00E-53 |
| SEQ. ID. No.990 | ORF3347 | 2054134 | 2055210 | Unknown | PIR:S77134 | 7,00E-53 |
| SEQ. ID. No.991 | ORF3351 | 2055260 | 2056804 | galactose-1-phosphate uridylyltransferase | TREMBL:AF082008_2 | 6,00E-72 |
| SEQ.ID. No.992 | ORF3354 | 2056876 | 2057895 | UDPglucose 4-epimerase | PIR:D69628 | 1,00E-102 |
| SEQ. ID. No.993 | ORF3355 | 2058641 | 2057949 | two-component system regulator | PIR:T35501 | 2,00E-27 |
| SEQ.ID. No.994 | ORF3368 | 2068722 | 2067535 | chloramphenicol resistance protein homolog | TREMBL:ECU73857_115 | 3,00E-66 |
| SEQ. ID. No.995 | ORF3372 | 2069223 | 2070902 | lysine--tRNA ligase | PIR:G70954 | 1,00E-148 |
| SEQ. ID. No.996 | ORF3373 | 2070967 | 2071929 | 1,4-dihydroxy-2-naphthoate octaprenyltransferase | PIR:A70546 | 1,00E-26 |
| SEQ. ID. No.997 | ORF3374 | 2071962 | 2072729 | phosphoglycerate mutase | PIR:S30886 | 9,00E-84 |
| SEQ. ID. No.998 | ORF3376 | 2073765 | 2073094 | phosphate transport system regulatory protein PhoU-like | PIR:G70533 | 2,00E-26 |
| SEQ.ID. No.999 | ORF3378 | 2073780 | 2075147 | Sensory transduction histidine kinases | TREMBLNEW.SCD8A_2 | 1,00E-46 |
| SEQ. ID. No.1000 | ORF3381 | 2076802 | 2075663 | phosphoserine aminotransferase | PIR:T45349 | 1,00E-110 |
| SEQ. ID. No.1001 | ORF3383 | 2077196 | 2078149 | Unknown | TREMBL:STH243106_4 | 3,00E-20 |
| SEQ. ID. No.1002 | ORF3392 | 2081808 | 2082683 | thymidylate synthase | PIR:C70881 | 1,00E-109 |
| SEQ. ID. No.1003 | ORF3393 | 2082514 | 2083455 | dihydrofolate reductase | TREMBL:AF006616_1 | 3,00E-26 |
| SEQ. ID. No.1004 | ORF3395 | 2083582 | 2084097 | protein-tyrosine-phosphatase | PIR:T37174 | 8,00E-22 |
| SEQ. ID. No.1005 | ORF3397 | 2085624 | 2084611 | branched-chain amino acid transport protein | PIR:G69592 | 5,00E-74 |
| SEQ. ID. No.1006 | ORF3400 | 2086942 | 2085851 | Unknown | PIR:G65081 | 1,00E-30 |
| SEQ.ID. No.1007 | ORF3403 | 2087036 | 2088046 | UDP-glucose 4-epimerase | TREMBLNEW:AP001510_283 | 1,00E-61 |
| SEQ. ID. No.1008 | ORF3408 | 2090294 | 2088993 | cyclopropane-fatty-acyl-phospholipid synthase | TREMBL:MLCB2407_16 | 3,00E-98 |
| SEQ. ID. No.1009 | ORF3410 | 2090734 | 2091882 | lactaldehyde reductase | PIR:RDECLA | 1,00E-120 |
| SEQ. ID. No.1010 | ORF3413 | 2092840 | 2094621 | Unknown | PIR:T35137 | 1,00E-45 |
| SEQ. ID. No.1011 | ORF3419 | 2094624 | 2095844 | glycosyltransferase | TREMBL:PPRRMP_7 | 2,00E-25 |
| SEQ. ID. No.1012 | ORF3420 | 2095942 | 2096895 | ABC transporter, ATP-binding protein | TREMBLNEW:SCD10_29 | 2,00E-69 |
| SEQ. ID. No.1013 | ORF3423 | 2098591 | 2100078 | Unknown | PIR:T36334 | 4,00E-32 |
| SEQ. ID. No.1014 | ORF3426 | 2100171 | 2101334 | Unknown | PIR:T36332 | 1,00E-45 |
| SEQ.ID. No.1015 | ORF3428 | 2101324 | 2101884 | hypoxanthine phosphoribosyltransferase | PIR:T36331 | 5,00E-40 |
| SEQ. ID. No.1016 | ORF3429 | 2101884 | 2103971 | cell division protein ftsH2 | PIR:T36330 | 0 |
| SEQ. ID. No.1017 | ORF3431 | 2104069 | 2104665 | GTP cyclohydrolase I | PIR:T36329 | 3,00E-64 |
| SEQ. ID. No.1018 | ORF3434 | 2104731 | 2105603 | Dihydropteroate synthase - Streptomyces | PIR:T36324 | 3,00E-49 |
| SEQ. ID. No.1019 | ORF3437 | 2105717 | 2107126 | hydroxymethyldihydropteridine | PIR:T36327 | 1,00E-30 |
| SEQ. ID. No.1020 | ORF3441 | 2108830 | 2107934 | acyl-CoA thioesterase II | PIRNEW:A82248 | 6,00E-39 |
| SEQ. ID. No.1021 | ORF3443 | 2110821 | 2109145 | ABC transporter ATP-binding protein | PIR:D70867 | 0 |
| SEQ.ID. No.1022 | ORF3448 | 2111654 | 2112601 | glucose kinase | PIR:S26208 | 9,00E-67 |
| SEQ.ID. No. 1023 | ORF3449 | 2113495 | 2112632 | ABC-transport system ATP binding protein | PIR:T35675 | 4,00E-57 |
| SEQ. ID. No.1024 | ORF3450 | 2114702 | 2113536 | transcription repressor | PIR:T35673 | 4,000-74 |
| SEQ.ID. No.1025 | ORF3454 | 2114954 | 2116108 | multiple sugar-binding protein | TREMBL:SC4A7_32 | 1,00E-68 |
| SEQ.ID. No.1026 | ORF3455 | 2116212 | 2117762 | L-arabinose transport ATP binding protein | TREMBL:AF160811_1 | 1,00E-168 |
| SEQ.ID. No.1027 | ORF3457 | 2117765 | 2118979 | L-arabinose membrane permease | TREMBL:AF160811_2 | 1,00E-71 |
| SEQ.ID. No.1028 | ORF3460 | 2120072 | 2120491 | permease | TREMBL:OOE250422_3 | 9,00E-20 |
| SEQ.ID. No.1029 | ORF3461 | 2120502 | 2120918 | Polypeptide deformylase | TREMBL:CBFMS_1 | 7,00E-32 |
| SEQ. ID. No.1030 | ORF3466 | 2123521 | 2124867 | xylose isomerase | PIR:JC1137 | 0 |
| SEQ. ID. No.1031 | ORF3468 | 2125136 | 2126410 | transposase | PIR:H70582 | 1,00E-153 |
| SEQ. ID. No.1032 | ORF3473 | 2129364 | 2127847 | xylulokinase | PIR:A41339 | 2,00E-78 |
| SEQ.ID. No.1033 | ORF3476 | 2129584 | 2130810 | Transcriptional regulators of NagC/XylR family | TREMBLNEW:SCG22_6 | 1,00E-39 |
| SEQ. ID. No.1034 | ORF3479 | 2131508 | 2132593 | translation releasing factor RF-1 | TREMBLNEW:SC26G5_4 | 1,00E-105 |
| SEQ. ID. No. 1035 | ORF3481 | 2132683 | 2133564 | Methylase | TREMBLNEW:SC26G5_5 | 3,00E49 |
| SEQ. ID. No. 1036 | ORF3483 | 2133840 | 2135024 | branched chain amino acid ABC transporter, periplasmic | PIR:C72290 | 9,00E-41 |
| SEQ. ID. No. 1037 | ORF3484 | 2135269 | 2136192 | high-affinity branched-chain amino acid transport protein | PIR:JH0668 | 4,00E-51 |
| SEQ. ID. No.1038 | ORF3485 | 2136212 | 2137285 | branched-chain amino acid transport protein | TREMBLNEW:AE004904_4 | 6,00E-35 |
| SEQ. ID. No.1039 | ORF3487 | 2137285 | 2138142 | branched chain amino acid ABC transporter, ATP-binding protein | PIR:F72290 | 6,00E-71 |
| SEQ. ID. No. 1040 | ORF3488 | 2138145 | 2138846 | branched chain amino acid ABC transporter, ATP-binding protein | PIR:T35757 | 7,00E-76 |
| SEQ. ID. No.1041 | ORF3490 | 2139658 | 2138984 | galactoside O-acetyltransferase | PIR:T37905 | 4,00E-26 |
| SEQ.ID. No.1042 | ORF3493 | 2139833 | 2140504 | Unknown | PIR:T44498 | 2,00E-23 |
| SEQ.ID. No.1043 | ORF3495 | 2140621 | 2141784 | UNDECAPRENYL-PHOSPHATE ALPHA-N-ACETYLGLUCOSAMINYLTRANSFERASE | PIR:T09982 | 7,00E-49 |
| SEQ. ID. No.1044 | ORF3497 | 2141756 | 2143390 | inosine-5'-monophosphate dehydrogenase | PIR:H70736 | 1,00E-164 |
| SEQ.ID. No.1045 | ORF3499 | 2143561 | 2144208 | oligoribonuclease | TREMBLNEW:AB036424_2 | 6,00E-51 |
| SEQ.ID. No.1046 | ORF3500 | 2144260 | 2145675 | helicase | PIRNEW:T47241 | 9,00E-53 |
| SEQ. ID. No.1047 | ORF3502 | 2145719 | 2147515 | Unknown | PIR:D70376 | 2.00E-20 |
| SEQ. ID. No.1048 | ORF3505 | 2147505 | 2148674 | pyruvate formate-lyase activating enzyme | PIR:C70646 | 1,00E-108 |
| SEQ. ID. No.1049 | ORF3509 | 2149420 | 2151261 | prolyl-tRNA synthetase | PIR:H70588 | 1,00E-175 |
| SEQ. ID. No.1050 | ORF3513 | 2154603 | 2152369 | zinc metalloproteinase | PIR:C70838 | 1,00E-165 |
| SEQ. ID. No.1051 | ORF3516 | 2155887 | 2156666 | methionine aminopeptidase | PIR:T34903 | 1,00E-65 |
| SEQ. ID. No.1052 | ORF3517 | 2156943 | 2158232 | citrate synthase | PIR:E70782 | 1,00E-124 |
| SEQ. ID. No.1053 | ORF3519 | 2159468 | 2158452 | tetrahydrodipicolinate succinylase | PIR:G70608 | 1,00E-72 |
| SEQ.ID. No.1054 | ORF3522 | 2160865 | 2165046 | helicase | TREMBL:BCX98455_2 | 1,00E-105 |
| SEQ.ID. No.1055 | ORF3527 | 2166299 | 2166811 | patch repair protein | PIRNEW:H81959 | 3,00E-34 |
| SEQ.ID. No.1056 | ORF3528 | 2168122 | 2166848 | oxidoreductase | PIRNEW:T50594 | 3,00E-46 |
| SEQ. ID. No.1057 | ORF3530 | 2167994 | 2169214 | aspartate aminotransferase | TREMBLNEW:AE004885_13 | 3,00E-78 |
| SEQ.ID. No.1058 | ORF3532 | 2169395 | 2170360 | exopolyphosphatase | PIR:T36297 | 1,00E-80 |
| SEQ.ID. No.1059 | ORF3539 | 2173536 | 2175398 | long chain fatty acid coA ligase | PIR:T35513 | 1,00E-129 |
| SEQ. ID. No.1060 | ORF3540 | 2176064 | 2175543 | NAD(P)H oxidoreductase | PIR:B69977 | 2,00E-35 |
| SEQ.ID. No.1061 | ORF3542 | 2177910 | 2176543 | exonuclease VII large subunit | PIR:B70898 | 4,00E-68 |
| SEQ.ID. No.1062 | ORF3545 | 2178378 | 2180783 | anaerobic ribonucleotide | TREMBL:U73336_1 | 0 |
| SEQ.ID. No.1063 | ORF3546 | 2180853 | 2181659 | anaerobic ribonucleotide reductase activase | TREMBL:U73336_2 | 6,00E-64 |
| SEQ.ID. No.1064 | ORF3548 | 2181890 | 2187025 | (R)-hydroxyglutaryl-CoA dehydratase activator | PIR:F70325 | 2,00E-73 |
| SEQ.ID. No.1065 | ORF3556 | 2187334 | 2188467 | gamma-glutamylcysteinyl synthetase precursor | TREMBL:AF128454_1 | 3,00E-33 |
| SEQ.ID. No.1066 | ORF3559 | 2189457 | 2191817 | beta-glucosidase | TREMBL:BFAF6658_1 | 1,00E-116 |
| SEQ. ID. No.1067 | ORF3564 | 2192945 | 2194156 | exo-1,3-beta-glucanase | TREMBLNEW:PB26160_1 | 3,00E-34 |
| SEQ. ID. No.1068 | ORF3567 | 2194826 | 2195962 | beta-glucosidase | TREMBL:U92808_1 | 6,00E-54 |
| SEQ. ID. No.1069 | ORF3571 | 2197101 | 2199056 | ABC transporter, ATP-binding protein | PIR:E72396 | 1,00E-104 |
| SEQ. ID. No.1070 | ORF3572 | 2199056 | 2201068 | ABC transporter | TREMBLNEW:AP001509_253 | 1,00E-98 |
| SEQ. ID. No.1071 | ORF3573 | 2201251 | 2202273 | banscriptional regulator LacI family | TREMBL:SCC57A_16 | 3,00E-43 |
| SEQ.ID. No.1072 | ORF3575 | 2202990 | 2203907 | Glycosidase | PIR:JE0404 | 1,00E-73 |
| SEQ.ID. No.1073 | ORF3576 | 2204012 | 2205439 | C4-dicarboxylate transporter | PIRNEW:G82431 | 8,00E-56 |
| SEQ.ID. No.1074 | ORF3579 | 2205668 | 2206633 | carbohydrate kinase | TREMBL:SCC57A_19 | 8,00E-44 |
| SEQ.ID. No.1075 | ORF3581 | 2207166 | 2208170 | transcription repressor of beta-galactosidase gene | TREMBLNEW:AP001514_24 | 2,00E-58 |
| SEQ.ID. No.1076 | ORF3582 | 2208838 | 2208239 | beta-galactosidase | TREMBL:BLO242596_1 | 3,00E-62 |
| SEQ.ID. No.1077 | ORF3585 | 2210107 | 2208899 | Aminotransferase | PIR:S32934 | 4,00E-54 |
| SEQ.ID. No.1078 | ORF3590 | 2210701 | 2214009 | isoleucyl-tRNA synthetase | PIR:E70760 | 0 |
| SEQ.ID. No.1079 | ORF3592 | 2215780 | 2214854 | Integrase | PIR:T13262 | 1,000-51 |
| SEQ.ID. No.1080 | ORF3591 | 2214208 | 2214855 | type I site-specific deoxyribonuclease | PIR:S02167 | 7,00E-25 |
| SEQ. ID. No.1081 | ORF3593 | 2216442 | 2215774 | type I restriction-modification enzyme 2, S subunit | TREMBL:AF153410_1 | 1,00E-54 |
| SEQ. ID. No.1082 | ORF3594 | 2216374 | 2217306 | Mrr restriction endonuclease | PIR:D70657 | 2,00E-50 |
| SEQ. ID. No.1083 | ORF3595 | 2217341 | 2219905 | type I site-specific deoxyribonuclease | TREMBLNEW:AF243383_16 | 1,00E-103 |
| SEQ.ID. No.1084 | ORF3596 | 2219908 | 2221125 | type I site-specific deoxyribonuclease | TREMBL:AF027167_1 | 1,00E-66 |
| SEQ. ID. No.1085 | ORF3598 | 2222569 | 2225991 | type I restriction enzyme R protein | TREMBL:AF013165_2 | 6,00E-93 |
| SEQ. ID. No. 1086 | ORF3602 | 2227282 | 2226065 | methionine adenosyltransferase | TREMBLNEW:CGL290443_2 | 1,00E-130 |
| SEQ. ID. No. 1087 | ORF3605 | 2228047 | 2229906 | dihydroxy-acid dehydratase | PIR:T36294 | 0 |
| SEQ. ID. No.1088 | ORF3607 | 2230986 | 2230003 | methionyl-tRNA formyltransferase | PIR:C70901 | 2,00E-68 |
| SEQ. ID. No. 1089 | ORF3611 | 2234085 | 2231776 | primosomal protein | PIR:G70900 | 5,00E-60 |
| SEQ. ID. No. 1090 | ORF3614 | 2234877 | 2234128 | Phosphoserine phosphatase | PIR:T36772 | 3,00E-49 |
| SEQ. ID. No.1091 | ORF3618 | 2234898 | 2235593 | Unknown | PIR:S76640 | 1,00E-37 |
| SEQ. ID. No. 1092 | ORF3619 | 2235657 | 2237219 | H+-transporting ATP synthase | PIR:F70512 | 1,00E-112 |
| SEQ. ID. No.1093 | ORF3620 | 2237244 | 2238908 | Unknown | PIR:C70512 | 9.00E-91 |
| SEQ.ID. No. 1094 | ORF3623 | 2240113 | 2241570 | Unknown | TREMBL:MLCB2533_24 | 4,00E-88 |
| SEQ. ID. No.1095 | ORF3626 | 2244677 | 2242119 | Unknown | TREMBLNEW:SC23B6_5 | 4,00E-49 |
| SEQ.ID. No.1096 | ORF3630 | 2246254 | 2244812 | adenylosuccinate lyase | PIRNEW:A82237 | 1,00E-111 |
| SEQ. ID. No.1097 | ORF3644 | 2255246 | 2254161 | methanol dehydrogenase regulatory protein | TREMBLNEW:AE004848_9 | 1,00E-64 |
| SEQ. ID. No. 1098 | ORF3646 | 2255918 | 2255280 | uracil-DNA glycosylase | PIR:E70672 | 2,00E-77 |

The ORFs corresponding to SEQ. ID. NO. 2 to SEQ. ID. NO. 1098 nucleotide sequences are defined in table 1, supra, and are repesented by their position in sequence SEQ. ID. No. 1. For example, the ORF3 sequence is defined by the nucleotide sequence between the nucleotides at position 785 and 2455 on the sequence SEQI No.1, ends included.

The open reading frames have been identified via homology analyses as well as via analyses of potential ORF start sites. It is to be understood that each identified ORF of the invention comprises a nucleotide sequence that spans the contiguous nucleotide sequence from the codon immediately 3' to the stop codon of the preceding ORF and through the 5' codon to the next stop codon of SEQ. ID. No. 1 inframe to the ORF nucleotide sequence.

Table 1 also depicts the results of homology searches that compared the sequences of the polypeptides encoded by each of the ORFs to sequences present in public published databases. It is understood that in one embodiment, those polypeptides listed in Table 1 as exhibiting greater than about 99 % identity to a polypeptide present in a publicly disclosed database are not considered part of the present invention. Likewise in this embodiment, those nucleotide sequences encoding such polypeptides are not considered part of the invention.

As regards the homology with the ORF nucleotide sequences, the homologous sequences exhibiting a percentage identity with the bases of one of the ORF nucleotide sequences of at least 80%, preferably 90% and 95%, are preferred. Such homologous sequences are identified routinely via, for example, the algorithms described above and in the examples below. The said homologous sequences correspond to the homologous sequences as defined above and may comprise, for example, the sequences corresponding to the ORF sequences of a bacterium belonging to the Bifidobacterium family.

These homologous sequences may likewise correspond to variations linked to mutations within the same species or between species and may correspond in particular to truncations, substitutions, deletions and/or additions of at least one nucleotide. The said homologous sequences may also correspond to variations linked to the degeneracy of the genetic code or to a bias in the genetic code which is specific to the family, to the species or to the variant and which are likely to be present in Bifidobacterium.

According to a preferred embodiment the polynucleotide is a nucleotide sequence, which encodes the following polypeptides or fragments thereof:

(a) SEQ. ID. No.5 (ORF13)

MPTGRVRWFDAAKGYGFITSEEGKDVFLPAQALPTGVTTLRKGAKVEYSV
VDGRRGPQAMDVRLIASAPSLVKATRPKADDMAAICEDLIKMLDAAGNTL
RRHRYPSAADSKKLATLLRAVADQFDVQD

(b) SEQ. ID. No.525 (ORF1827)

MTTAAAQAPAPGKLEFKDDYTPDEAERVIRNSKGLPVGVRPKMVWTWKKA
LLWAAIAIVCACGWAILAVSRGEQISAIWFLVVALSSYAIAYRFYAYYIQ
IKIMRTDDANATPAERVHDGANFERTDRRVLFGQHFAGISGAGPLVGPIL
AAQMGYLPSTLWIILGVIFAGAVQDMLVLWISAKRRGRSLGQMATDEMGK
FGGMILSIFLVVMTAIAMAFLALVAIKAMAASPWAVFSIGMTIPIALIMG
CYQRFLRPGRVIETTLLGFVLLVLDIVAGGWIASIPAVAAVFTLDAKQLV
IALVIYSFAAAALPHWLLVTPRDYLSTLMKIGTLVLLVIGIIIANPSVKV
PGLTELASTSTGPTFSGNLFPFLFITIACGALSGFHGAVSSGLTPKAVEK
ENQIRMIGYGSMLVESFTAVIALIAAITISQGVYFSTNMSAAQITAASGV
SISATSTPGEQADAAVKAVESMKVSDIEGNQMQVTWDSVDENGAAKTYEG
AAALEQAAADIGETSIVSRTGGATTFAMGMANFLKSYLGGHDSMAFWYHF
AIMFEALFILTTVDNGTRVARYQIGEMLGNVRKLKKFADPTWKPGN
IITTLIATALWGGLLWMGVSDANGGINAMVPIFGISNQLLAAACFV
LITVCVAKMGYWKHLWIPVVPLVWDIAVTFTADFQKIFGPLSYFTT
ASKYQAQIDSGELTGEALTNAKAALSNAYLDGVLSVFFLVMMGVFV
VVGIVVVARTFAAGKYGAETTSEEPFVESQWFAPSSLVATALEKKV
QREYSAKLHELVRNGQVAA

c) SEQ. ID. No.424 (ORF1473)

MTQSRRMLVLRAVVEDYIRSQEPVGSTSLTRDHDLGVSSATIRNDMAALE
DEGYLIQPHTSAGRVPTEKGYRYFVDRLATVVPLSEAQRRGINSFLSGSV
SLKDALQRSARLLSEITGQVAVVASPSLAKATLRHVEMVPVAMTTLLAVV
ITDTGRVAQHGLTIASMPAVDEINRLSNTVNEQCDGLSLSKSAETVRSIA
ASAGYESVRGVADTLADAFESMALDERANELYMSGTSHLAHSRSLADLAP
LFDALEEQVVLMKLMSNLSEETNASGVGVAIGSEMHTPGLLHASVVSSGY
GRSGAAGEPAGNDPVGEPETESETESQTNDTEPIAFVGSIGPTHMDYAAT
MAAVRAVARYLTAFLSEGRTQD

d) SEQ. ID. No.548 (ORF1905)

MYFKDGNDNAQRGGSTVRRSRQRRIMGRVVSYNEDVPRCTFCGKTEHQVR
KLVAGPNASICDECIALCVDIISEERVKDAEVNSLSLPKPAQIFDYLNRY
VIGQENAKRALSVAVYNHYKRVNMELQESAEQLDGNNGHSGQTSKQAKQS
VPTQTRATRRSNDPLADVEVAKSNILLLGPTGVGKTYLAQALARVMNVPF
VITDATTLTEAGYVGDDVETVLQRLLQAADGDVSRAQHGIIYIDEIDKIA
RKSGENTSITRDVSGEGVQQALLKILEGTIASVPLEGTRKHKEQDVAQMD
TRGILFICGGAFVGLTDIVRKRLGRRETGFGANWHDADMKDEELLEQVNA
DDLAEFGLLPEFIGRLPVTSVLKELTVDDLTAILTQPANALIKQYRKLFA
VDGVDLQFTEQAIRAIADIAIKQGTGARGLRSIIERTLQDTMFQLPSLDD
VRQVIVDKASVEGSSTPKLLREAVDVPQGRLKVAQSVLLDRVRTHEA

e) SEQ. ID. No.74 (ORF219)

MSEQLMEQYRLRGQRKCRNACIAAIVTVVLVLAVAGGVWWTAGDGSALVR
NMFKPKATPATQPVVNSTATFAYRTAPEFLAMEAGDRGTGNVNYSPASMW
MALAIAAQGANGTTRSQLNELLGSGSLTDSDYQSLLSSINGQYSGAKSEM
SAANSLWIDDDYSLASDYQSTVKKMFEAEVTTLPFDDQAAAKMSDWIAKH
TNGSLKPKITLRDREVLSIINTVYADGRWKDPFEEQSTGNGTFHGEAGDA
QVPMMHQTFSQMAYGHDEYNTWQRVEIPFDNGGNLAIVLPAEGHFDELAG
DAEKLSWAFGTCSTASLGEGAMGCAADSMPGWGVSVNSVMVNVTLPRFTI
DSMFDSEATIKAFEKLGVTDAFSAGDADFTKMIDTGSHGENLYIGSILQG
TRIEVNEAGAKAMSFTKVGADSVSAPVDNVEFTVDRPFLYSYVTPDGIPL
FIGAVRNLGGVGGEN

f) SEQ. ID. No.576 (ORF1972)

MQIRPGSMYPLGASYDGAGVNFALYSQVAQKVELCLFDEHDVETRIEMTE
RNSYVWHNYIPGLHPGQRYGYRVYGPYDPVHGLRCNPNKLLLDPYAKAIE
GNIDGDESLFSYWFKSPDDNSAMNDLDSAAHTMKSAVINPYFDWGNDQHP
YISYHDSVIYEAHVRGMTNLNMDVPPDIRGTYAGLAYPSVIEYLKKLGIT
AIELMPIHQFVNDSFLQEKGLSNYWGYNTIGFFAPHNAYSSSGERGEQVN
EFKSMVKAYHRAGMEVILDVVYNHTAEGNHMGPTLSFKGIDNASYYRLVE
GDQQHYFDTTGTGNSLLMRSPHALQLITDSLRYWVTEMHVDGFRFDLAAT
LARQFQEVDKLSAFFDIVEQDPIISRVKLIAEPWDLGSGGYQVGGFPSSW
SEWNGRYRDTVRDFWRSQPSTLPEFASRLMGSSDLYQVNGRRPVASVNFI

TAHDGFTMNDLVSYNEKHNEANGEGNRDGESNNRSWNCGVEGPTNIPDVN
DLRQRQMRNMFATLLFSQGIPMICGGDEVARTQQGNNNAYCQDNEISWTN
WHLDKGRKELLAFVSKLIHLRLDHPVLHRRRFFTGREPGDDSNTIPQVEW
FDHTGSIMDMDDWQNTHAFSMMIYLNGSDIPEVDWYGNRMVDNDFILIFN
AHYEPIMFTLPDERYGRKWQLVVDTHNPNEPALSYEAGFMITAQSRSFLM
LMSAKKPKKPMGL

g) SEQ. ID. No.403 (ORF1403)

MEIRARPHMASGSYRELFSARMMYGMQYIQQTIVGIDGSEARFFGYVADN
SEEMEPDRIRPAILILPGGGYAMTSDREAEPVALQFLAKGFAVFVLRYSV
QPSRYPVALLEAAEAMRLIRANVDQWHVNPAQVAVLGFSAGGHLAANLAT
SVGDEDIREQGGMDPDAVRPNALMLSYPVITAGKYAHRGSFQCLLGDQAH
NQALLDKFSIEKHIDAKTPPVFVWHTMTDDAVPVENTLMLIQACRAAGVS
IEAHLFPEGSHGLSLANAETAGNGFYAHIVECVQCWPDLAEAWLRRLF

(h) SEQ. ID. No.804 (ORF2676)

MFLKPEQQLERCRRIVRQRVDPHIHPSIAQLTVESYDIPGEPMPSDEFFA
KLDRGDIDFKPFMLGSEWGTTWGTVWFRLTGTVPAGYPKGKPLELILDLG
WYPHSCGGHIEGLVYRADGTAIKAVHPLNYWVPFMDAEGNAQVPVAEDGS
FTLYLEAASNPLLLGVPPFIETELGDHATGKPDEPYVFKSADLAEFDERY
ENYSVDLDVVSSLMEFADKQSPRYWQLAKALQRSLNAYDERNPESVEAAR
AVLAGVLAKPANASAMNVSAIGHAHIDSAWLWPVRETRRKVARTVSNALA
LMDADPDFKYAMSSAQQYAWLEEDHPDIFKRMKRRIEEGRFIPVGGMWVE
ADGMLPAGESLIRQIAYGRKYFKEHLGVEPKGVWLPDSFGYTGAWPQIAR
RAGYEWFLTQKISWNDTTKFPHHSFMWEGIDGSRIFTHFPPADTYAAWCK
VQELDYAEKNFQDKDLSDRSLLLFGFGDGGGGPTRNMMEHLHRYENLEGV
SKVSIEEPNDFFDKAHQQLAENAGPEMPVWKGELYLELHRGTLTSQQDMK
RGCRQEESLLRTVEYLGAAAVLSDPEYVYPREELDRIWKTLLLNQFHDIL
PGSAIAWVHREAREDYRRDLKRLAEIAQDMCAVLRKANPQADLLAEARIS
QFRNDGASWHANRINEPTDALSVLTQTLDNGRVLLANGVLSVTIEADGTI
SSLLDEEHGRELVPAGTRLGQYELLRDEPAVWDAWEIERESLLMANAVTG
SIESVNTENGAAQVHVHTADGDTVITTTITLRPGSHTLDFHADIDWHERE
RFLKVDLPLGIVADQATYDCQYGLIRRPIVKNTASDEAKYESSTNRFAII
GDAGYAAAVINGSVYGSDASPIAGNAAEGRDSGTMFRLSLLSAPTFPDPR
TDIGSHEFDWSVVADATVDRALDAAGVLNAPVLHDVPDITPLASIESVNG
TVVLDWMKLADDGSGDLIVRAYEAAGGQADAMLHVCPALAGASVHETNVL
EGDDLAADLPVALQDGRQNAEGATLHFGPFQLATLRITR

(i) SEQ. ID. No.313 (ORF1077)

MISRDGWAVIDDSAANIIIETDTVNGKANPFGTWVSPRATAETDLYFFGY
GHRYIEAVRDFYRLTGPTPLLPRFAMGNWWSRYYRYTQDGYLALMDRFKR
EGIPFTTSVIDMDWHRVDDVDPKYGSGWTGYSWNRELFPDPPAFLADLHR
RGLRTTLNVHPRDGVRAFEDAYPEVAKRVGIDPATEENVEFDLTNPDFVD
AYFDMHHRMEAEGVDFWWLDWQQGGVTRQKGLDPLWMLNHMHYLDSGRGG

NWPLTFSRYAGPGSHRYPVGFSGDTIVTWESLAFQPQFTATASNIGYGWW
SHDIGGHMFGYRNEELEARWYQLGAFSPINRLHSSNSPFSGKEPWNFNRD
VSAAMVDALRLRHAMMPYLYTMNYRAAEAGRPLVEPMYWQNPDTPDAYEV
PDEFRFGTELVVAPIVSPDDAAACRGRADAWLPQGEWFDFFDGRRYVSSD
AAGRRLEVWRSLDRTPVFAKAGAIVPLQDVAESGEAINSIANPQALRVLV
FPGADGSFVMREDRGTWGAPSADTAIAFTWGGADASPSAFTVAPVTGDTS
AVPELRDWTVVFRGVAPVDAASGVRAWSGEAPVEATVAYDEATMSLTVSV
TGISSAASLRIEIPGGLRIADNPVESDAMDLLLHAQMLYRTKELALQAVH
KLGIGAIGALRTMNRGPRYANDFWITDMPDAVAGALEEILLRS

According to another preferred embodiment the present invention comprises a polynucleotide encoding a fusion protein, comprising any one of SEQ. ID. NO. 2 to SEQ. ID. NO. 1098 ligated in frame to a polynucleotide encoding a heterologous polypeptide. The skilled person is well aquainted with techniques performing such a ligation and expressing the corresponding fusion-polypeptide in an appropriate cell.

The present invention also relates to recombinant vectors for the cloning and/or the expression of a nucleotide sequence according to the present invention. The vectors comprise elements necessary to enable expression and/or secretion of the nucleotide sequences in a given host cell, such as a promoter, signals for initiation and for termination of translation, as well as appropriate regions for regulation of transcription. For example, expression of a protein or peptide may be controlled by any promoter/enhancer element known in the art. Exemplary promotors are the CMV promoter, the SV40 early promoter region, the promoter contained in the 3' long terminal repeat of the rous sarcoma virus, the herpes thymidine kinase promoter, the regulatory sequences of the metallothionein gene, or, for prokaryotic expression systems, the β-lactamase promoter, the tac promoter or the T7 promoter.

The vector should be capable of being stably maintained in the host cell and may optionally possess particular signals specifying the secretion of the translated protein. These different elements are chosen according to the host cell utilized. To this effect the nucleotide sequences according to the invention may be inserted into autonomously-replicating vectors within the chosen host, or integrative vectors in the chosen host, such as e.g yeast artificial chromosomes, plasmids or viral vectors. It will be appreciated that the vector may well be the plasmid accordig to SEQ. ID. No 1099 or a recombinant form thereof, which has been supplemented by particular ori's that enable a high copy number.

Any of the standard methods known to those skilled in the art for inserting DNA fragments into a vector may be used to construct expression vectors containing a chimeric gene consisting of appropriate transcriptional/translational control signals and the protein coding sequences. These methods may include in vitro recombinant DNA and synthetic techniques and in vivo recombinants (genetic recombination).

The vector may be used for transcripton and/or translation of a nucleic acid comprised by SEQ. ID. NO. 1 to produce RNA or antisense RNA, respectively. Such a vector can remain episomal or become chromosomally integrated, as long as it can be transcribed to produce the desired transcript.

The antisense nucleic acids of the invention comprise a sequence complementary to at least a portion of a RNA transcript of a polynucleotide sequence in SEQ. ID. No. 1, designating a sequence having sufficient complementarity to be able to hybridize with the RNA, forming a stable duplex. In the case of double-stranded antisense nucleic acid sequence, a single strand of the duplex DNA may thus be tested, or triplex formation may be assayed.

The invention also encompasses host cells transformed with a nucleic acid or a vector according to the present invention and as described above. These cells may be obtained by introducing into an appropriate cell a nucleotide sequence or a vector as defined above, and then culturing the said cell under conditions allowing the replication and/or the expression of the transformed/transfected nucleotide sequence.

The host cell may be chosen from eukaryotic or prokaryotic system, such as for example bacterial cells, yeast cells, animal cells as well as plant cells. In the context of this invention a cell shall be understood to comprise higher biological systems. Such as animals, whole plants or parts thereof. Furthermore, a host cell strain may be chosen which modulates the expression of the inserted sequences, or modifies and processes the gene product in the specific fashion desired.

A preferred host cell for the expression of the proteins of the invention consists of prokaryotic cells, such as gram negative or gram positive bacteria. A further preferred host cell according to the invention is a bacterium belonging to the Bifidobacterium family, more preferably belonging to the species Bifidobacterium longum or chosen from a microorganism associated with the species Bifidobacterium longum.

The transformed/transfected cells according to the invention may advantageously serve as a model and may be used in methods for studying, identifying and/or selecting compounds capable of being responsible for any of the beneficial effects brought about by the present Bifidobacterium strain.

The invention further provides polypeptides encoded by the Bifidobacterium longum ORFs, in particular those listed in table 1 and identified in the sequence listings. In the present description, the terms polypeptide, peptide and protein are used interchangeably. Furthermore the present invention also pertains to method for preparing such polypeptides by recombinant means comprising the steps of (a) culturing a host cell according to the present invention under conditions suitable to produce the polypeptide encoded by the polynucleotide; and (b) recovering the polypeptide from the culture.

It will be appreciated that the above polypeptides may also be obtained using combinatory chemistry, wherein the polypeptide is modified at some locations before testing them in model systems, so as to select the compounds which are the most active or which exhibit the desired properties.

In this context, chemical synthesis has the advantage of being able to use non-natural amino acids or nonpeptide bonds. Accordingly, in order to e.g. extend the life of the polypeptides according to the invention, it may be advantageous to use such non-natural amino acids, for example in the D form, or alternatively amino acid analogues, preferably sulphur-containing forms.

Finally, the structure of the polypeptides according to the invention, its homologous or modified forms, as well as the corresponding fragments may be integrated into chemical structures of the polypeptide type and the like. Accordingly, in order to preserve the polypeptide in an in vivo environment it will be preferred to provide at the N- and C-terminal ends compounds which convey a resistance to degradation to proteases.

It will also be appreciated that the different polypeptides according to the present invention and produced by the above method may represent antigens to the immune system of a host animal, so that antibodies may be produced directed against said polypeptides. These antibodies may be used for the detection of a polypeptide of interest in a mixture or generically of a strain of Bifidobacterium in a sample. In addition they may be used as research tools by e.g. producing antibodies against cellular surface epitopes and determining the effect of blocking certains polypeptides on the bacterial cell wall. Therefore, according to another aspect, the invention provides antibodies directed to epitopes on the various polypeptides provided by this invention.

According to another aspect the present invention also provides a method for the detection and/or identification of Bifidobacterium longum in a biological sample. This method may comprise several techniques known in the art, such as PCR or simply hybridisation with a suitable probe. Alternatively, an antibody raised against a cell wall epitope of Bifidobacterium longum may be used for said purpose. It will be appreciated that the above method may also be reversed and the presence of antibodies against Bifidobacterium may be determined by contacting the sample to be tested with a polypeptide of Bifidobacterium under conditions to allow formation of immune complexes.

The polypeptides according to the invention, the antibodies according to the invention described below and the nucleotide sequences according to the invention may be used in in vitro and/or in vivo methods for the detection and/or the identification of bacteria belonging to the species Bifidobacterium in a biological sample (biological tissue or fluid) which is likely to contain them. These methods, depending on the specificity of the polypeptides, of the antibodies and of the nucleotide sequences according to the invention which will be used, may detect and/or identify the bacterial variants belonging to the species Bifidobacterium as well as associated microorganisms capable of being detected by the polypeptides, the antibodies and the nucleotide sequences according to the invention which will be chosen. It may, for example, be advantageous to choose a polypeptide, an antibody or a nucleotide sequence according to the invention, which is capable of detecting any bacterium of the Bifidobacterium family by choosing a polypeptide, an antibody and/or a nucleotide sequence according to the invention which is specific to the family.

All the sequences referred to herein (SEQ ID. NO. 1 to SEQ ID: NO. 1099) are listed in the attached sequence listings which is to be considered as part of the specification.

The invention also comprises the nucleotide sequences or polypeptides according to the invention covalently or noncovalently immobilized on a solid support. In the first case such a support may serve to capture, through specific hybridization, the target nucleic acid obtained from a biological sample to be tested. If necessary, the solid support is separated from the sample and the hybridization complex formed between the capture probe and the target nucleic acid is then detected by means of a second probe, called detection probe, labelled with an easily detectable element.

Such support may take the form of so-called DNA array or DNA chips, a multitude of molecular probes precisely organized or arrayed on a solid support, which will allow sequencing genes, studies of mutations contained therein and the expression of genes, and which are currently of interest given their very small size and their high capacity in terms of number of analyses.

The function of these arrays/chips is based on molecular probes, mainly oligonucleotides which are attached to a carrier having a size of generally a few square centimetres or more as desired. For an analysis the carrier (DNA array/chip) is coated with probes that are arranged at a predetermined location of the carrier. A sample containing fragments of a target nucleic acid to be analysed, for example DNA or RNA or cDNA, that has been labelled beforehand, is subsequently contacted with the DNA array/chip leading to the formation, through hybridization, of a duplex. After a washing step, analysis of the surface of the chip allows the effective hybridizations to be located by means of the signals emitted by the labels tagging the target. A hybridization fingerprint results from this analysis which, by appropriate computer processing, allows to retrieve information such as the expression of genes, the presence of specific fragments in the sample, the determination of sequences and the presence of mutations.

The hybridization between the probes of the invention, deposited or synthesized in situ on the DNA chips, and the sample to be analysed, may, e.g. be determined by means of fluorescence, radioactivity or by electronic detection.

The nucleotide sequences according to the invention may be used in DNA arrays/chips to carry out analyses of the expression of the Bifidobacterium genes. This analysis is based on DNA arrays/chips on which probes, chosen for their specificity to characterize a given gene, are present. The target sequences to be analysed are labelled before being hybridized onto the chip. After washing the labelled compounds are detected and quantified, with the hybridizations being carried out at least in duplicate. Comparative analyses of the signal intensities obtained with respect to the same probe for different samples and/or for different probes with the same sample, determine a differential transcription of RNA derived from the sample.

The DNA arrays/chips according to the present invention may also contain nucleotide probes specific for other microorganisms, which will enable a serial testing allowing rapid identification of the presence of a microorganism in a sample.

The principle of the DNA chip, as detailed above may also be used to produce protein chips on which the support has been coated with a polypeptide or an antibody according to the invention, or arrays thereof, in place of the DNA. These protein chips make it possible to analyse the biomolecular interactions (BIA) induced by the affinity capture of target analytes onto a support coated e.g. with proteins, by surface plasma resonance (SPR). The polypeptides or antibodies according to the invention, capable of specifically binding antibodies or polypeptides derived from the sample to be analysed, may thus be used in protein chips for the detection and/or the identification of proteins in samples.

The present invention also relates to a computer readable medium having recorded thereon one or more nucleotide and/or a polypeptide sequences according to the invention. This medium may also comprise additional information extracted from the present invention, such as e.g. analogies with already known sequences and/or information relating to the nucleotide and/or polypeptide sequences of other microorganisms so as to facilitate the comparative analysis and the exploitation of the results obtained. Preferred media are e.g. magnetic, optical, electrical and hybrid media such as, for example, floppy disks, CD-ROMs or recording cassettes.

The invention also relates to kits or sets for the detection and/or the identification of bacteria belonging to the species Bifidobacterium longum or to associated microorganisms, which comprises, a polypeptide according to the invention, where appropriate, the reagents for constituting the medium appropriate for the immunological or specific reaction, the reagents allowing the detection of the antigen-antibody complexes produced by the immunological reaction between the polypeptide (s) of the invention and the antibodies which may be present in the biological sample, it being possible for these reagents also to carry a label, or to be capable of being recognized in turn by a labelled reagent, more particularly in the case where the polypeptide according to the invention is not labelled, a reference biological sample (negative control) free of antibodies recognized by a polypeptide according to the invention, a reference biological sample (positive control) containing a predetermined quantity of antibodies recognized by a polypeptide according to the invention.

The invention also relates to a kit or set for the detection and/or the identification of bacteria belonging to the species Bifidobacterium longum or to an associated microorganism, or for the detection and/or the identification of a microorganism, wherein the kit comprises a protein chip according to the invention.

The present invention also pertains to a novel microorganism termed NCC2705, which has been deposited according to the Budapest Treaty with the Institute Pasteur on January 29^{th}, 2001 and received the deposit no. CNCM I-2618. This micro-organism belongs to the genus Bifidobacterium, species Bifidobacterium longum and is a probiotc micro-organism, i.e. it may pass the gastro-intestinal tract in an essentially live and viable form and has the capability of preventing colonization of the intestine with pathogenic bacteria causing diarrhea and in addition may prevent or reduce the occurence of infection of intestinal cells by rotaviruses.

The microorganism is gram positive, catalase negative and CO₂ production negative, it produces L(+) lactic acid and essentially prevents colonization of intestinal cells by bacteria bringing about diarrhea, such as pathogenic E. coli, e.g. enteropathogenic E.coli (EPEC), or salmonella, e.g. Salmonella typhimurium and prevents infection of intestinal cells by rotaviruses.

The novel microorganism may be used for the preparation of a variety of carrier materials, such as e.g. milk, yogurt, curd, fermented milks, milk based fermented products, fermented cereal based products, milk based powders, infant formulae and may be included in the support in an amount of from about 10⁵ cfu / g to about 10¹¹ cfu / g. For the purpose of the present invention the abbreviation cfu shall designate a "colony forming unit" that is defined as number of bacterial cells as revealed by microbiological counts on agar plates.

The present invention also provides a food or a pharmaceutical composition containing at least the Bifidobacterium NCC 2705 and/or containing a supernatant, in which the microorganisms have been grown or an active fraction/metabolite thereof, respectively.

For preparing a food composition according to the present invention at least one of the Bifidobacteria of the present invention is incorporated in a suitable support, in an amount of from about 10⁵ cfu / g to about 10¹² cfu / g, preferably from about 10⁶ cfu / g to about 10¹⁰ cfu / g, more preferably from about 10⁷ cfu / g to about 10⁹ cfu / g.

In case of a pharmaceutical preparation the product may be prepared in form of tablets, liquid bacterial suspensions, dried oral supplements, wet oral supplements, dry tube feeding or a wet tube feeding with the amount of the Bifidobacterium/Bifidobacteria to be incorporated therein being in the range of up to about 10¹² cfu / g, preferably from about 10⁷ cfu / g to about 10¹¹ cfu / g, more preferably from about 10⁷ cfu / g to about 10¹⁰ cfu / g.

The activity of the novel microorganism in the individual's intestine is of course dose dependent. That is, the more the novel microorganism or an active component thereof is incorporated by means of ingesting the above food material or the pharmaceutical composition the higher the protective and/or curing activity. Since the novel microorganism is not detrimental to mankind and animals and has eventually been isolated from baby feces a high amount thereof may be incorporated so that essentially a high proportion of the individual's intestine will be colonized by the novel microorganisms.

Yet, according to another preferred embodiment the supernatant of a culture of the Bifidobacterium of the present invention, or an active fraction thereof, may be used for preparing the carrier. The supernatant may be used as such or may be dried under conditions that do not destroy the metabolic compounds secreted by the microrganisms into the liquid medium, such as e.g. freeze drying, and may be included in the carrier. In order to minimize the number of unknown compounds in the supernatant the Bifidobacteria will preferably be grown in a defined media, the composition of which is known and does not negatively affect the host incorporating it. Further, the skilled person will, based on his general knowledge optionally deplete the supernatant from unwanted products, such as e.g. by means of chromatography.

The present inventors have investigated baby feces and isolated a variety of different bacterial strains therefrom. These strains were subsequently examined for their capability to prevent prevent colonization and/or invasion of epithelial cells with bacteria that are known to cause diarrhea, such as E.coli, Sigella, Klebsiella, Yersinia, Pseudomonas aeruginosa Listeria, Streptococcus, Staphilococcus, Clostridium difficile, H. pyori and also Candida albicans.

Several bacterial genera comprising Bifidobacterium, Lactococcus and Streptococcus were screened for their diarrhea inhibitory properties. The tests for the inhibitory property were performed with pathogenic microorganisms, such as E.coli, Klebsiella, Yersinia, Pseudomonas aeruginosa, H. pyori, and Salmonella typhimurium as representatives for pathogenic micro-organisms causing diarrhea in affected individuals.

The various bacteria were grown in a suitable medium, such as MRS, Hugo-Jago or M17 medium at temperatures of from about 30 to 40°C corresponding to their optimal growth temperature. After reaching stationary growth the bacteria were collected by centrifugation and resuspended in physiological NaCl solution. Between the different tests the bacterial cells were stored frozen (-20°C).

For assessing anti-bacterial properties the following approaches were chosen.

According to one protocol the cultured Bifidobacterium of the present invention was examined for its capability to decrease the viability of the different pathogenic micro-organisms. To this end, a culture of pathogenic bacteria was contacted with a concentrated supernatant of a Bifidobacterium culture and the growth potential of the pathogenic bacteria was assessed.

According to a second protocol the adhesion capability of the Bifidobacteria of the present invention to T₈₄ cells, a cell culture model for the intestine, was determined by culturing the Bifidobacterium with T₈₄ cells and the rate of adhesion was assessed.

According to another protocol the potential of the Bifidobacterium of the present invention to prevent infection of intestinal cells by Salmonella, using the cell line Caco-2 as a model for the intestine, was determined. In this respect, the supernatant of a cell culture of the Bifidobacteria of the present invention was added together with the pathogenic microorganism to the intestinal cells and the rate of adhesion, or invasion, respectively, was assessed.

Thus, it could be shown that the cultured Bifidobacterium and the supernatant proofed to be extremely effective in preventing both adhesion to and invasion into the intestinal cells indicating that one or more metabolic compounds secreted by the microorganism is/are likely to be responsible for the anti-diarrhea activity.

According to yet another protocol it was further assessed, whether NCC2705 would be capable to prevent invasion of epithelial cells by rotaviruses. Two different protocols were applied. According to one protocol the various bacterial strains were examined for their direct interaction with the rotavirus strain while in the second protocol the bacteria were screened for those strains that interact with cellular rotavirus receptors.

The first protocol involved contacting the respective bacterial suspension each with a different rotavirus strain and incubating in suitable media. Subsequently, the virus-bacteria mixture was applied to a monolayer of cells of the human undifferentiated colon adenoma cells HT-29 (intestinal epithelial cell line) and incubation was continued. Virus replication was then assayed.

The second protocol involved incubating the respective bacterial suspension first together with a monolayer of cells of the human undifferentiated colon adenoma cells HT-29 and adding the virus subsequently. After continued incubation virus replication was assayed.

Rotavirus replication was assessed by histo-immunological staining of rotavirus proteins in infected cells. A rotavirus inhibitory effect was attributed to a given bacterium when the number of infected cells was reduced by 90% in the cell culture inoculated with rotavirus plus the indicated bacteria in comparison with cells inoculated only with rotavirus.

The present invention will now be described by way of examples without limiting the same thereto.

Media and solutions:
MRS (Difco)
Hugo-Jago (tryptone 30g / 1(Difco), yeast extract 10 g / 1(Difco), lactose 5 g / 1
(Difco), KH₂PO₄ 6 g / 1, beef extract 2 g / 1(Difco), agar 2 g / 1(Difco))
M17 (Difco)
Eugon Tomato Agar (Canned tomato juice 400 ml, Eugon agar BBL 45.5 g, Maltose Difco 10 g, Hemin Sigma 5mg, Agar Difco 5 g, distilled water 600 ml)
DMEM (Dulbecco's modified Eagle medium)
CFA (according to Ghosh et al. Journal of Clinical Microbiology, 1993 31 2163-6)
Müller Hinton agar (Oxoid)
LB (Luria Bertami, Maniatis, A Laboratory Handbook, Cold Spring Harbor, 1992)
C¹⁴-acetate (53,4 Ci/mMol, Amersham International PLC)
PBS (NaCl 8g/l, KCl 0.2 g/l, Na₂HPO₄ 1.15 g/l, KH₂PO₄ 0.2 g/l))
Trypsin-EDTA solution (Seromed)
FCS Fetal calf serum (Gibco)

E. coli DAEC C 1845 was obtained from Washington University, Seattle and E. coli JPN15 was obtained from the Center for Vaccine Development of the University of Maryland, USA). The Salmonella typhimurium strain SL1344 was obtained from the department of Microbiology, Stanford University, CA, USA. This strain acts as a pathogen on mice and is resistant to Streptomycin. It adheres to Caco-2 colon cells (Finlay and Falkow, 1990). The Klebsiella was obtained from stock clinical isolates from the microbiological laboratory of the Faculté de Pharmacie Paris XI, Châtenay-Malabry, France. The Yersinia was obtained from INSERM Unit 411, Hôpital Necker, Paris, France. The Pseudomonas aeruginosa was obtained from stock clinical isolates from the microbiological laboratory of the Faculté de Pharmacie Paris XI, Châtenay-Malabry, France.

The H. pylori was obtained from Institute of Microbiology, Lausanne University, Lausanne, Switzerland.

Human rotavirus Wa (G1 serotype) and simian rotavirus SA-11 (G3 serotype) were obtained from P.A. Offit, Children's Hospital of Philadelphia, U.S.A. The DS-lxRRV reassortant virus was obtained from A. Kapikian, NIH Bethesda, U.S.A. The serotype 4 human rotavirus Hochi was obtained from P. Bachmann, University of Munich, Germany.

### Example 1

### Isolation of Bifidobacteria

Fresh feces were harvested from diapers of 16 healthy babies 15 to 27 days old. 1 g of fresh feces was placed under anaerobic conditions for transportation to the laboratory and microbiological analyses were run within 2 hours from sampling by serial dilutions in Ringer solution and plating on selective media. Eugon Tomato Agar (Canned tomato juice 400 ml, Eugon agar BBL 45.5 g, Maltose Difco 10 g, Hemin Sigma 5mg, Agar Difco 5 g, distilled water 600 ml) incubated anaerobically at 37°C for 48 hours was used to isolate bifidobacteria. Colonies were randomly picked up and purified. Physiological and genetic characterisation was performed on the isolates.

### Example 2

### Cultivating cell lines

### Caco-2 cells:

For the inhibition assays the cell line Caco-2 was utilized as a model of mature enterocytes of the small intestine. This cell line presents characteristic of intestinal cells such as e.g. polarization, expression of intestinal enzymes, production of particular structural polypeptides etc.. The cells were grown on different supports, namely on plastic dishes (25 cm², Corning) for growth and propagation, on defatted and sterilized 6 well glass plates (22 x 22 mm, Corning) for the adhesion and the inhibition tests. After the second day in culture the medium (DMEM) was changed on a daily basis. Before use the medium was supplemented with 100 U/ml penicilline/streptomycine, 1 µg/ml amphoterine, 20 % FCS inactivated at 56 °C for 30 min and 1 % of a solution containing non-essential amino acids (10 mM) (Eurobio, Paris, France). Culturing was performed at 37 °C in an atmosphere comprising 90% air and 10% CO₂. The cells were splitted every six days. The cells were detached from the walls of the well by treatment in PBS with 0.015 % trypsine and 3 mM EDTA at pH 7.2. For neutralizing the effect of trypsine an equal volume of the culture medium containing FCS was added to the cell suspension obtained, the mixture was centrifuged (10 min at 1000 rpm) and the pellet was again dissolved in culture medium. Living cells (not dyed with trypane blue) were counted. About 3.5 x 10⁵ living cells were transferred to a new culture bottle and about 1.4 x 10⁵ cells per well and cultivated until a confluent monolayer was obtained.

### T₈₄ cells:

For the adhesion assays the cell line T₈₄ was utilized as a model of colon cells from the intestine. This cell line presents characteristics of intestinal cells such as e.g. polarisation, expression of intestinal enzymes, production of particular structural polypeptides etc.. T₈₄ cells were obtained from University of California, San Diego, CA. Cells were grown in DMEM (50%) and Ham's F12 (50%) supplemented with 2 mM glutamine, 50 mM HEPES, 1% non-essential amino acids and 10% inactivated (30 min, 56°C) fetal calf serum (Boehringer, Mannheim, Germany) at 37°C in a 10% CO₂/90% air atmosphere. Cells were seeded at a concentration of 10⁶ cells per cm². Cells were used for adherence assays at late post-confluence, i.e., after 10 days.

All strains except Bifidobacteria were kept at -80°C in their culture medium containing 15% glycerol. As the number of transfers into new media has an influence on the adhesion factors, the Salmonella strain was only transferred twice within a period of 24 hours, the first transfer taking place when the strain was frozen. All cultures were raised aerobically.

### Bifidobacteria

The bacterial strain (Bifidobacterium longum CNCM I-2618 (NCC2705) was stored at -20 °C in MRS medium containing 15 % glycerol. The strain was grown under anaerobic conditions in MRS and transferred twice to new media at intervals of 24 hours before use in the inhibition assays. For the assay a concentration of 2 x 10⁹ cfu/ml was utilized. The supernatant was collected by centrifugation for 1 hour at 20.000 rpm and the supernatant obtained was subsequently checked for the presence of bacteria. The strains of Bifidobacterium were cultivated anaerobically in MRS during 18 hours at 37°C. The cultures were then centrifuged (20 min. at 4°C), the supernatant was collected, lyophilized, returned to the solution and then concentrated ten times (10x). The pH of the supernatant was finally adjusted to 4.5.

### E. coli C 1845:

The first passage after thawing was effected on a CFA - Müller Hinton agar, which is suitable to effect expression of adhesion factors by the bacterium. Before each experiment the bacterial cells were incubated at 37 °C with a transfer to a new medium being effected twice after 24 hours each.

### Klebsiella:

Bacteria were grown overnight for 18 hrs at 37°C in Luria broth.

### Yersinia:

Bacteria were grown overnight for 18 hrs at 37°C in Luria broth .

### Pseudomonas aeruginosa:

Bacteria were grown overnight for 18 hrs at 37°C in Luria broth.

### H. pylori:

Bacteria were grown on Brain-Heart Infusion (BHI)-agar plates containing 0.25% yeast extract (Difco Laboratories, Detroit, MI), 10% horse serum and 0.4% Campylobacter selective complement (Skirrow supplement, SR 69 ; Oxoid Ltd, Basingstoke, England).

### Example 4

The Caco-2 and T₈₄ monolayers, prepared on glass coverslips which were placed in six-well Corning tissue culture plates (Corning Glass Works, Corning, NY), were washed twice with phosphate-buffered saline (PBS). Bifidobacteria (1 ml, 4x10⁸ bacteria/ml in spent culture supernatant, treated-supernatant or fresh MRS broth) were added to 1 ml of the cell line culture medium. This suspension (2 ml) was added to each well of the tissue culture plate and the plate incubated at 37 °C in 10% CO₂/90% air. After 1 hour of incubation, the monolayers were washed five times with sterile PBS, fixed with methanol, stained with Gram stain, and examined microscopically. Each adherence assay was conducted in duplicate over three successive passages of intestinal cells. For each monolayer on a glass coverslip, the number of adherent bacteria was evaluated in 20 random microscopic areas. Adhesion was evaluated by two different technicians to eliminate bias.

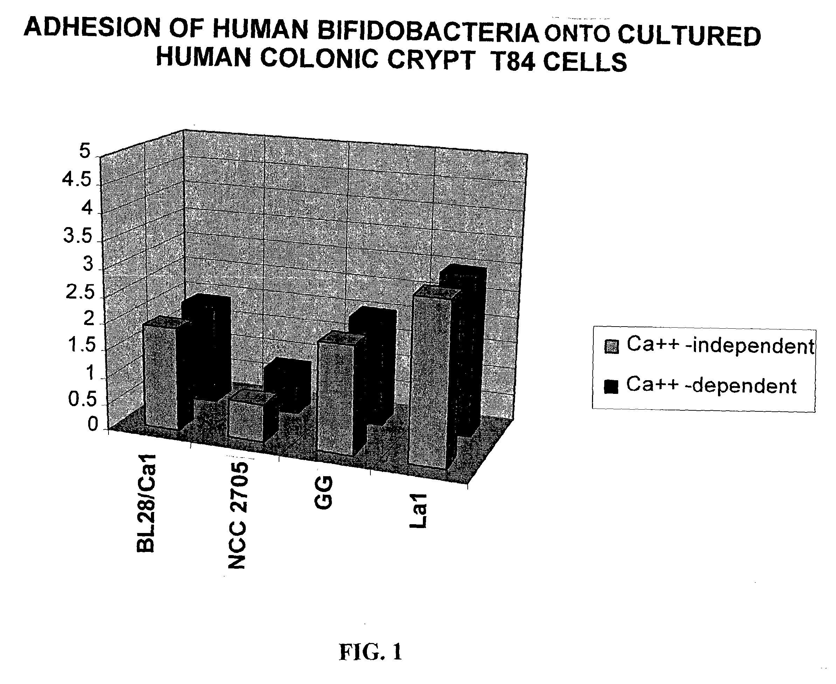

The results are shown in Fig. 1 from which it becomes obvious that NCC2705 is capable to adhere to intestinal cells as compared to to the known cell line GG (WO 97/00078), Lal (EP 0 577 903) or another Bifido strain (BL28/Cal).

### Example 5

As candidates for pathogenic bacteria E.coli, Klebsiella, Yersinia, Pseudomonas aeruginosa and H. pyori were used.

Based on a culture of NCC2705 kept in MRS medium for 18 hours, an exponentially growing culture was produced (3 hours at 37°C). 2 ml of this solution were removed and centrifuged for 5 min. at 5500 g, +4 °C. After collection of the supernatant the cell pellet was washed in sterile PBS. After centrifuging, the pellet was collected and 2 ml of sterile PBS were added. The bacteria were counted and the suspension was adapted in such a way that between 1 and 5 x 10⁶ bacteria / ml were produced.

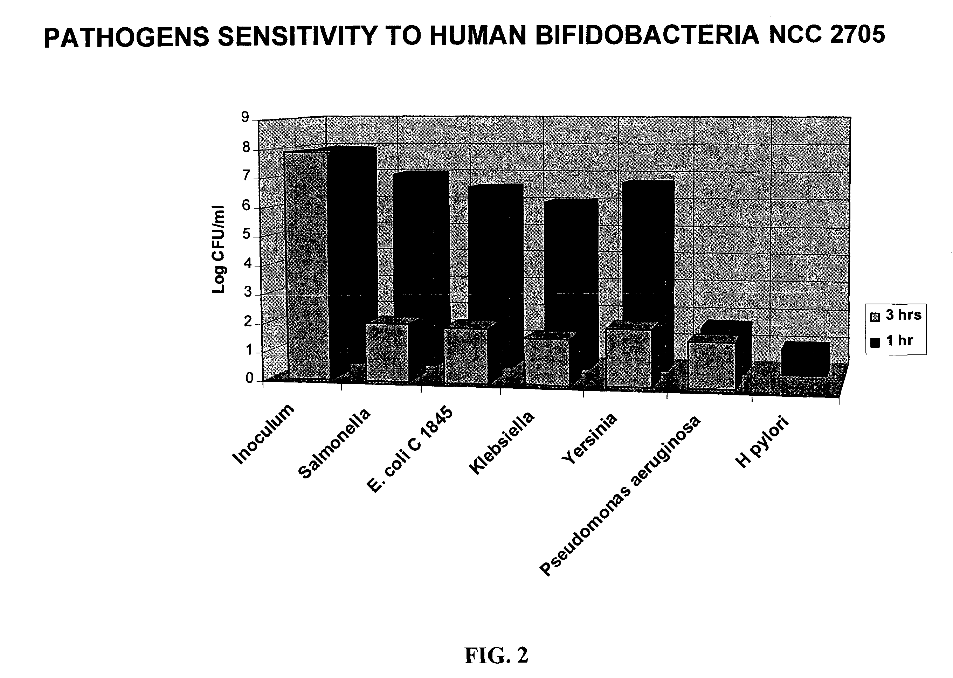

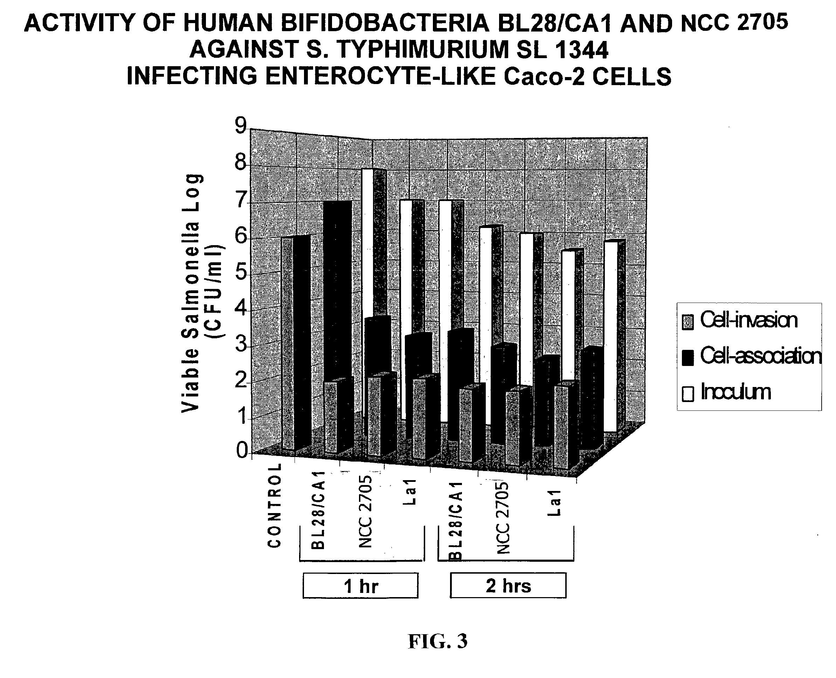

The assessment of the antimicrobial effect exerted by the Bifidobacteria of the present invention was carried out according to the Lehrer method described in Lehrer et al., J. Imunol. Methods 137 (1991), 167-173, which document is incorporated here by way of reference. The results thereof are shown in Fig. 2 and 3.

From the above results it may be seen that the Bifidobacterium of the present invention may effectively inhibit growth of the various pathogenic bacteria.

### Example 6

### Inhibition assay for salmonella

Salmonella are bacteria that invade epithelial cells and multiply therein. For determining the inhibitory activity of the Bifidobacteria of the present invention towards Salmonella typhimurium the strain SL1344 and following procedure was used.

The pathogenic cells were cultivated in LB-medium. After the second passage to new medium the bacterial strains were marked with radioisotopes using C¹⁴-acetate at 10 µCi/ml in LB-medium. Incubation of the strains in this medium was performed for 18 hours at 37 °C.

The bacterial suspension was subsequently subjected to centrifugation (1041 rpm, 15 min) so as to eliminate the remaining C¹⁴-acetate from the supernatant. The pellet was suspended and washed in PBS and the cells were suspended at a concentration of about 10⁸ cells / ml in 1 % sterile mannose. Mannose is known to inhibit non specific adhesion. The bacterial solution was then adjusted to 2 x 10⁸ cells/ml.

The pathogen (1 ml; 2 x 10⁸ cells) and an aliquot of a supernatant (1 ml) of a Bifidobacterium culture are pre-incubated for 2 hours at 37°C. The suspension is subsequently centrifuged, the resulting supernatant is removed and the pellet is again suspended in 0.5 ml PBS. This pathogen solution (0,5 ml) is then brought in contact with human intestine cells in culture. The culture was washed with sterile PBS twice and 0,5 ml adhesion medium (DMEM) was added. The cells are then incubated for 1 hour at 37°C under 10% CO₂.

After incubation the number of bacteria in the incubation medium and on/in the intestinal cells are counted. In order to determine the amount of cells adhering on or having invaded into the intestinal cells the following approaches have been chosen.

For determining the number of adhering bacteria the medium was decanted and the cells were washed once with culture medium and once with sterile PBS. Subsequently, 1 ml of sterile H₂O was added per compartment, to lyse the cells and to form a cell solution which was incubated for 1-2 hours at 37°C, after which successive dilutions were carried out. In order to count the number of adhering and invasive bacteria, the cell solution was centrifuged to remove cell debris and the radioactivity was measured.

According to another protocol 10 aliquots were each put on TSA medium. The media were then incubated for 18-24 hours at 37°C.

For determining the amount of invaded bacteria the Caco-2 cells were washed with PBS so as to eliminate all non-adhering cells. Subsequently, a medium containing gentamycin (20 µg/ml) was added and incubation was continued for 1 hour at 37 °C. Gentamycin is an antibiotic not penetrating intestinal cells so that all extracellular microorganisms were killed, while bacteria having already invaded intestinal cells will survive. The cells were then incubated for another hour at 37°C and were then washed twice with PBS. The cells were lysed by addition of and incubation in sterile distilled water for for 1-2 hours at 37°C. After removing the cell debris radioactivity was determined. According to another protocol successive dilutions were carried out, which were put on TSA medium. Incubation: 18-24 hours at 37°C.

It may be seen that cultured cells and the culture supernatant were extremely effective in preventing adhesion of and invasion into intestinal cells by Salmonella.

### Example 7

### Infection of mice by the strain S. typhimurium C5

Adult, 7-8 weeks old, axenic, female mice (C3H/He/oujco conventional, Iffa Credo, France), raised under sterile conditions, were orally infected with a fixed concentration of S. typhimurium (0,2 ml, 10⁸ cfu/mouse). Some mice were rendered monoxenic by the implantation of a range of Bifidobacteria strains. With some mice, the Bifidobacteria in segments of the intestine were counted after its removal and mincing of the organs in PBS. With other mice, the protection against infection was assessed in such a way that they were continuously kept in a sterile environment and the days of survival were compared to the control group.

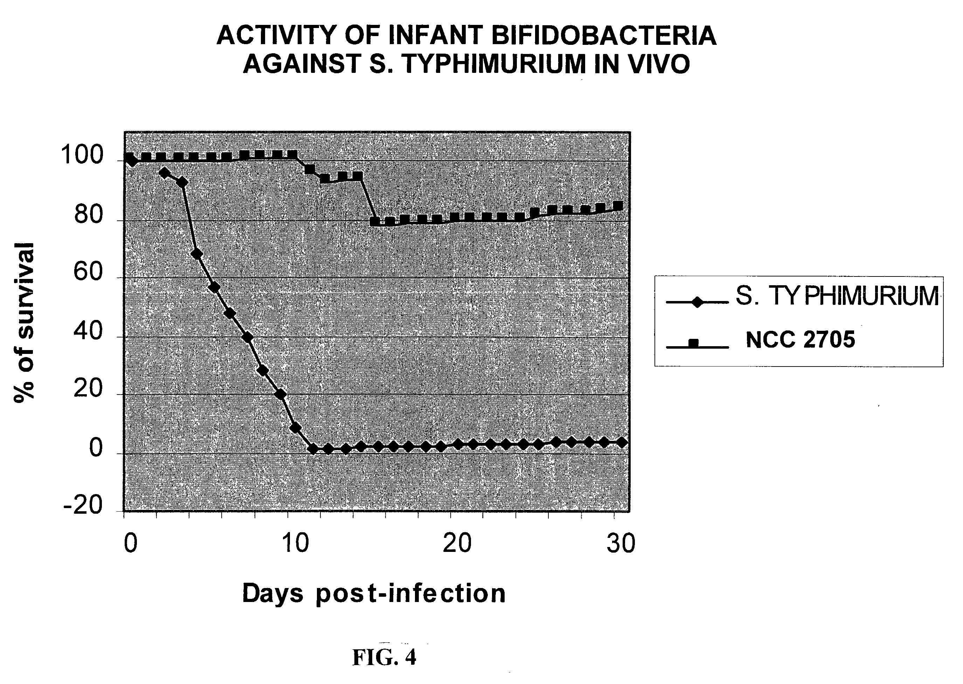

The results are shown in Fig. 4. As may be derived therefrom in the control group nearly all mice died after a time period of about 10 days. In contrast thereto, all mice treated with NCC2705 were alive after 10 days with only 20 % dying from the detrimental effect exerted by Salmonella after a period of 30 days.

### Example 8

### 1^{st} protocol:

30 µl of the respective bacterial suspension (containing on average 3x10⁶ bacteria) were mixed with 70 µl M199 medium supplemented with 10% tryptose phosphate broth (Flow) and 5% trypsin-EDTA solution (Seromed) to which were added 100 µl of virus in supplemented M199 medium. The virus-bacteria mixture thus obtained was incubated for 1 hour at 4°C and for 1 hour at 37°C. Separately, cells of the human undifferentiated colon adenoma cells HT-29 growing as a confluent monolayer in 96-well microtiter plates (in M199 medium supplemented with 10% tryptose phosphate broth (Flow) and 5% trypsin-EDTA solution (Seromed) 1 : 4 diluted with PBS) were washed three times with phosphate-buffered saline (PBS ; pH 7.2). The virus-bacteria mixture processed as indicated above was transferred to the cells and the microtiter plates were incubated for 18 h in a CO₂ incubator (Heraeus). Virus replication was assayed as described below.

### 2^{nd} protocol:

30 µl of the bacterial suspension (supra) were mixed with 70 µl M199 medium supplemented with 10% tryptose phosphate broth (Flow) and 5% trypsin-EDTA solution (Seromed ) and applied directly on HT-29 cells grown and pretreated as described in the 1^{st} protocol in the microtiter plates. After one hour incubation at 37°C 100 µl of virus in supplemented M199 medium were added to the cells in the microtiter plates. The incubation was continued for 18 h in a CO₂ incubator (Heraeus). Virus replication was assayed as described below.

The rotavirus replication was assessed by histo-immunological staining of rotavirus proteins in infected cells as described hereafter.

One day after infection, the cell culture medium was removed from the microtiter plates and the cells were fixed with absolute ethanol for 10 min. Ethanol was discarded, and the plates were washed three times in PBS buffer. Then 50 µl of an anti-rotavirus serum (mainly directed against VP6 protein), produced in rabbits (obtained from the ISREC University of Lausanne) and diluted 1 :2000 in PBS was added to each well and incubated for 1 h at 37°C with a cover slip to prevent desiccation of the wells. The anti-serum was discarded afterwards and the plates were washed three times with PBS. Then 50 µl of anti-rabbit immunoglobulin G (IgG) antiserum produced in goats and coupled to peroxidase (GAR-IgG-PO; Nordic) were added at a dilution of 1 : 500 in PBS to each well and the plates were incubated for 1 hour at 37 °C. The serum was discarded and the plates were again washed three times with PBS. Then 100 µl of the following substrate mixture was added to each well: 10 ml of 0.05 M Tris-hydrochloride (pH 7.8), 1 ml of H₂O₂ (30% suprapur, diluted 1 :600 in H₂O ; Merck) and 200 µl of 3-amino-9-ethylcarbazole (0.1 g/10 ml of ethanol stored in 200 µl aliquots at -80 °C ; A-5754 ; Sigma). The plates were incubated for at least 30 min at room temperature. The substrate was discarded and the wells were filled with 200 µl of H₂O to stop the reaction. Infected cell foci were counted with an inverted microscope (Diavert; Leitz).

Only very few bacterial strains interacted with rotaviruses. Merely 4 out of the 260 bacterial cells primarily selected inhibited rotavirus replication in at least one protocol. Bifidobacterium adolescentis CNCM I-2618 (NCC2705) showed an extremely high activity against Serotype 1 Rotavirus, Serotype 3 rotavirus SA-11 and Serotype 4 rotavirus Hochi.

NCC2705 is gram positive and catalase negative, it does not produce CO₂ during fermentation and produces just L (+) lactic acid according to methods disclosed in the "Genera of lactic acid bacteria", Ed. B.J.B. Wood and W.H. Holzapfel, Blackie A&P.

These results show the extreme superior properties of the Bifidobacterium of the present invention.

## Claims

1. A polynucleotide having a nucleotide sequence of the Bifidobacterium genome, comprising
(a) the nucleotide sequence of SEQ. ID. No. 1;
(b) a nucleotide sequence exhibiting at least 90% identity with the sequence of SEQ. ID. No. 1; or
(c) a nucleotide sequence that hybridizes to SEQ ID. No. 1 under stringent conditions.

2. A polynucleotide having a nucleotide sequence selected from the group:
(a) a nucleotide sequence as identifed by SEQ ID. NO. 1099; or
(b) a nucleotide sequence exhibiting at least 95 % identity with SEQ. ID. NO. 1099; or
(c) a polynucleotide which hybridizes to SEQ. ID. NO. 1099 under conditions of high stringency.

3. A polynucleotide having a nucleotide sequence of an open reading frame (ORF) of a Bifidobacterium longum genome, comprising:
(a) a nucleotide sequence chosen from any one of Seq ID. No. 2 to SEQ. ID. NO. 1098; or
(b) a nucleotide sequence exhibiting at least 95 % identity with any one of SEQ ID. NO. 2 to SEQ. ID. NO. 1098; or
(c) a polynucleotide which hybridizes to any one of SEQ. ID. NO. 2 to SEQ. ID. NO. 1098 under conditions of high stringency.

4. The polynucleotide of any of the claims 1 or 2, which encodes the following polypeptides or fragments thereof:

(a) SEQ. ID. No.5 (ORF13)

MPTGRVRWFDAAKGYGFITSEEGKDVFLPAQALPTGVTTLRKGAKVEYSV
VDGRRGPQAMDVRLIASAPSLVKATRPKADDMAAICEDLIKMLDAAGNTL
RRHRYPSAADSKKLATLLRAVADQFDVQD

(b) SEQ. ID. No.525 (ORF1827)

MTTAAAQAPAPGKLEFKDDYTPDEAERVIRNSKGLPVGVRPKMVWTWKKA
LLWAAIAIVCACGWAILAVSRGEQISAIWFLVVALSSYAIAYRFYAYYIQ
IKIMRTDDANATPAERVHDGANFERTDRRVLFGQHFAGISGAGPLVGPIL
AAQMGYLPSTLWIILGVIFAGAVQDMLVLWISAKRRGRSLGQMATDEMGK
FGGMILSIFLVVMTAIAMAFLALVAIKAMAASPWAVFSIGMTIPIALIMG
CYQRFLRPGRVIETTLLGFVLLVLDIVAGGWIASIPAVAAVFTLDAKQLV
IALVIYSFAAAALPHWLLVTPRDYLSTLMKIGTLVLLVIGIIIANPSVKV
PGLTELASTSTGPTFSGNLFPFLFITIACGALSGFHGAVSSGLTPKAVEK
ENQIRMIGYGSMLVESFTAVIALIAAITISQGVYFSTNMSAAQITAASGV
SISATSTPGEQADAAVKAVESMKVSDIEGNQMQVTWDSVDENGAAKTYEG
AAALEQAAADIGETSIVSRTGGATTFAMGMANFLKSYLGGHDSMAFWYHF
AIMFEALFILTTVDNGTRVARYQIGEMLGNVRKLKKFADPTWKPGNIITT
LIATALWGGLLWMGVSDANGGINAMVPIFGISNQLLAAACFVLITVCVAK
MGYWKHLWIPVVPLVWDIAVTFTADFQKIFGPLSYFTTASKYQAQIDSGE
LTGEALTNAKAALSNAYLDGVLSVFFLVMMGVFVVVGIVVVARTFAAGKY
GAETTSEEPFVESQWFAPSSLVATALEKKVQREYSAKLHELVRNGQVAA

c) SEQ. ID. No.424 (ORF1473)

MTQSRRMLVLRAVVEDYIRSQEPVGSTSLTRDHDLGVSSATIRNDMAALE
DEGYLIQPHTSAGRVPTEKGYRYFVDRLATVVPLSEAQRRGINSFLSGSV
SLKDALQRSARLLSEITGQVAVVASPSLAKATLRHVEMVPVAMTTLLAVV
ITDTGRVAQHGLTIASMPAVDEINRLSNTVNEQCDGLSLSKSAETVRSIA
ASAGYESVRGVADTLADAFESMALDERANELYMSGTSHLAHSRSLADLAP
LFDALEEQVVLMKLMSNLSEETNASGVGVAIGSEMHTPGLLHASVVSSGY
GRSGAAGEPAGNDPVGEPETESETESQTNDTEPIAFVGSIGPTHMDYAAT
MAAVRAVARYLTAFLSEGRTQD

d) SEQ. ID. No.548 (ORF1905)

MYFKDGNDNAQRGGSTVRRSRQRRIMGRVVSYNEDVPRCTFCGKTEHQVR

KLVAGPNASICDECIALCVDIISEERVKDAEVNSLSLPKPAQIFDYLNRY
VIGQENAKRALSVAVYNHYKRVNMELQESAEQLDGNNGHSGQTSKQAKQS
VPTQTRATRRSNDPLADVEVAKSNILLLGPTGVGKTYLAQALARVMNVPF
VITDATTLTEAGYVGDDVETVLQRLLQAADGDVSRAQHGIIYIDEIDKIA
RKSGENTSITRDVSGEGVQQALLKILEGTIASVPLEGTRKHKEQDVAQMD
TRGILFICGGAFVGLTDIVRKRLGRRETGFGANWHDADMKDEELLEQVNA
DDLAEFGLLPEFIGRLPVTSVLKELTVDDLTAILTQPANALIKQYRKLFA
VDGVDLQFTEQAIRAIADIAIKQGTGARGLRSIIERTLQDTMFQLPSLDD
VRQVIVDKASVEGSSTPKLLREAVDVPQGRLKVAQSVLLDRVRTHEA

e) SEQ. ID. No.74 (ORF219)

MSEQLMEQYRLRGQRKCRNACIAAIVTVVLVLAVAGGVWWTAGDGSALVR

NMFKPKATPATQPVVNSTATFAYRTAPEFLAMEAGDRGTGNVNYSPASMW

MALAIAAQGANGTTRSQLNELLGSGSLTDSDYQSLLSSINGQYSGAKSEM

SAANSLWIDDDYSLASDYQSTVKKMFEAEVTTLPFDDQAAAKMSDWIAKH

TNGSLKPKITLRDREVLSIINTVYADGRWKDPFEEQSTGNGTFHGEAGDA

QVPMMHQTFSQMAYGHDEYNTWQRVEIPFDNGGNLAIVLPAEGHFDELAG

DAEKLSWAFGTCSTASLGEGAMGCAADSMPGWGVSVNSVMVNVTLPRFTI

DSMFDSEATIKAFEKLGVTDAFSAGDADFTKMIDTGSHGENLYIGSILQG

TRIEVNEAGAKAMSFTKVGADSVSAPVDNVEFTVDRPFLYSYVTPDGIPL

FIGAVRNLGGVGGEN

f) SEQ. ID. No.576 (ORF1972)

MQIRPGSMYPLGASYDGAGVNFALYSQVAQKVELCLFDEHDVETRIEMTE
RNSYVWHNYIPGLHPGQRYGYRVYGPYDPVHGLRCNPNKLLLDPYAKAIE
GNIDGDESLFSYWFKSPDDNSAMNDLDSAAHTMKSAVINPYFDWGNDQHP
YISYHDSVIYEAHVRGMTNLNMDVPPDIRGTYAGLAYPSVIEYLKKLGIT
AIELMPIHQFVNDSFLQEKGLSNYWGYNTIGFFAPHNAYSSSGERGEQVN
EFKSMVKAYHRAGMEVILDVVYNHTAEGNHMGPTLSFKGIDNASYYRLVE
GDQQHYFDTTGTGNSLLMRSPHALQLITDSLRYWVTEMHVDGFRFDLAAT
LARQFQEVDKLSAFFDIVEQDPIISRVKLIAEPWDLGSGGYQVGGFPSSW

SEWNGRYRDTVRDFWRSQPSTLPEFASRLMGSSDLYQVNGRRPVASVNFI
TAHDGFTMNDLVSYNEKHNEANGEGNRDGESNNRSWNCGVEGPTNIPDVN
DLRQRQMRNMFATLLFSQGIPMICGGDEVARTQQGNNNAYCQDNEISWTN
WHLDKGRKELLAFVSKLIHLRLDHPVLHRRRFFTGREPGDDSNTIPQVEW
FDHTGSIMDMDDWQNTHAFSMMIYLNGSDIPEVDWYGNRMVDNDFILIFN
AHYEPIMFTLPDERYGRKWQLVVDTHNPNEPALSYEAGFMITAQSRSFLM
LMSAKKPKKPMGL

g) SEQ. ID. No.403 (ORF1403)

MEIRARPHMASGSYRELFSARMMYGMQYIQQTIVGIDGSEARFFGYVADN

SEEMEPDRIRPAILILPGGGYAMTSDREAEPVALQFLAKGFAVFVLRYSV

QPSRYPVALLEAAEAMRLIRANVDQWHVNPAQVAVLGFSAGGHLAANLAT

SVGDEDIREQGGMDPDAVRPNALMLSYPVITAGKYAHRGSFQCLLGDQAH

NQALLDKFSIEKHIDAKTPPVFVWHTMTDDAVPVENTLMLIQACRAAGVS

IEAHLFPEGSHGLSLANAETAGNGFYAHIVECVQCWPDLAEAWLRRLF

(h) SEQ. ID. No.804 (ORF2676)

MFLKPEQQLERCRRIVRQRVDPHIHPSIAQLTVESYDIPGEPMPSDEFFA

KLDRGDIDFKPFMLGSEWGTTWGTVWFRLTGTVPAGYPKGKPLELILDLG

WYPHSCGGHIEGLVYRADGTAIKAVHPLNYWVPFMDAEGNAQVPVAEDGS

FTLYLEAASNPLLLGVPPFIETELGDHATGKPDEPYVFKSADLAEFDERY

ENYSVDLDVVSSLMEFADKQSPRYWQLAKALQRSLNAYDERNPESVEAAR

AVLAGVLAKPANASAMNVSAIGHAHIDSAWLWPVRETRRKVARTVSNALA

LMDADPDFKYAMSSAQQYAWLEEDHPDIFKRMKRRIEEGRFIPVGGMWVE

ADGMLPAGESLIRQIAYGRKYFKEHLGVEPKGVWLPDSFGYTGAWPQIAR

RAGYEWFLTQKISWNDTTKFPHHSFMWEGIDGSRIFTHFPPADTYAAWCK

VQELDYAEKNFQDKDLSDRSLLLFGFGDGGGGPTRNMMEHLHRYENLEGV

SKVSIEEPNDFFDKAHQQLAENAGPEMPVWKGELYLELHRGTLTSQQDMK

RGCRQEESLLRTVEYLGAAAVLSDPEYVYPREELDRIWKTLLLNQFHDIL

PGSAIAWVHREAREDYRRDLKRLAEIAQDMCAVLRKANPQADLLAEARIS

QFRNDGASWHANRINEPTDALSVLTQTLDNGRVLLANGVLSVTIEADGTI

SSLLDEEHGRELVPAGTRLGQYELLRDEPAVWDAWEIERESLLMANAVTG

SIESVNTENGAAQVHVHTADGDTVITTTITLRPGSHTLDFHADIDWHERE

RFLKVDLPLGIVADQATYDCQYGLIRRPIVKNTASDEAKYESSTNRFAII

GDAGYAAAVINGSVYGSDASPIAGNAAEGRDSGTMFRLSLLSAPTFPDPR

TDIGSHEFDWSVVADATVDRALDAAGVLNAPVLHDVPDITPLASIESVNG

TVVLDWMKLADDGSGDLIVRAYEAAGGQADAMLHVCPALAGASVHETNVL

EGDDLAADLPVALQDGRQNAEGATLHFGPFQLATLRITR

(i) SEQ. ID. No.313 (ORF1077)

MISRDGWAVIDDSAANIIIETDTVNGKANPFGTWVSPRATAETDLYFFGY
GHRYIEAVRDFYRLTGPTPLLPRFAMGNWWSRYYRYTQDGYLALMDRFKR
EGIPFTTSVIDMDWHRVDDVDPKYGSGWTGYSWNRELFPDPPAFLADLHR
RGLRTTLNVHPRDGVRAFEDAYPEVAKRVGIDPATEENVEFDLTNPDFVD
AYFDMHHRMEAEGVDFWWLDWQQGGVTRQKGLDPLWMLNHMHYLDSGRGG
NWPLTFSRYAGPGSHRYPVGFSGDTIVTWESLAFQPQFTATASNIGYGWW
SHDIGGHMFGYRNEELEARWYQLGAFSPINRLHSSNSPFSGKEPWNFNRD
VSAAMVDALRLRHAMMPYLYTMNYRAAEAGRPLVEPMYWQNPDTPDAYEV
PDEFRFGTELVVAPIVSPDDAAACRGRADAWLPQGEWFDFFDGRRYVSSD
AAGRRLEVWRSLDRTPVFAKAGAIVPLQDVAESGEAINSIANPQALRVLV
FPGADGSFVMREDRGTWGAPSADTAIAFTWGGADASPSAFTVAPVTGDTS
AVPELRDWTVVFRGVAPVDAASGVRAWSGEAPVEATVAYDEATMSLTVSV
TGISSAASLRIEIPGGLRIADNPVESDAMDLLLHAQMLYRTKELALQAVH
KLGIGAIGALRTMNRGPRYANDFWITDMPDAVAGALEEILLRS

5. A polynucleotide encoding a fusion protein, comprising any one of SEQ. ID. NO. 2 to SEQ. ID. NO. 1098 ligated in frame to a polynucleotide encoding a heterologous polypeptide.

6. A recombinant vector that contains the polynucleotide of any of the preceding claims.

7. A recombinant vector according to claim 6, that contains a polynucleotide of Claim 4.

8. A recombinant vector according to any of the claims 6 or 7, wherein the polynucleotide is operatively associated with a regulatory sequence that controls gene expression.

9. A host that contains a polynucleotide according to any of the Claim 1 to 5 or a recombinant vector according to any of the claims 6 to 8.

10. A host according to claim 9, that contains a polynucleotide of Claim 4 or the vector according to claim 7.

11. A host according to any of the claims 9 or 10, which is a procaryotic cell, an eucaryotic or a plant or a a non-human animal.

12. A method for producing a polypeptide of Bifidobacteruim longum, comprising:
(a) culturing a host cell according to claim 9 or claim 10 under conditions suitable to produce the polypeptide encoded by the polynucleotide; and
(b) recovering the polypeptide from the culture.

13. A method for producing a fusion protein, comprising:
(a) culturing a host cell according to Claim 9 or claim 10 under conditions suitable to produce the fusion protein encoded by the polynucleotide; and
(b) recovering the fusion protein from the culture.

14. A polypeptide encoded by a polynucleotide according to any of the claims 3 to 5.

15. A fusion protein encoded by a polynucleotide of Claim 5.

16. An antibody that immunospecifically binds to a polypeptide of any of the claims 14 or 15.

17. An antibody that immunospecifically binds to the fusion protein of claim 5.

18. A method for the detection and/or identification of Bifidobacterium longum in a biological sample, comprising:
(a) contacting the sample with a polynucleotide primer derived fom a sequence according to any of the claims 1 to 4 in the presence of a polymerase enzyme and nucleotides under conditions which permit primer extension; and
(b) detecting the presence of primer extension products in the sample in which the detection of primer extension products indicates the presence of Bifidobacteruim longum in the sample.

19. A method for the detection and/or identification of Bifidobacterium in a biological sample, comprising:
(a) contacting the sample with a probe derived from a polynucleotide according to any of the claims 1 to 8 under conditions which permit hybridization of complementary base pairs; and
(b) detecting the presence of hybridization complexes in the sample in which the detection of hybridization complexes indicates the presence of Bifodobacterium longum in the sample.

20. A method for the detection and/or identification of Bifodobacterium in a biological sample, comprising:
(a) contacting the sample with an antibody according to Claim 16 or 17 under conditions suitable for the formation of immune complexes; and
(b) detecting the presence of immune complexes in the sample, in which the detection of immune complexes indicates the presence of Bifodobacterium longum in the sample.

21. A method for the detection and/or identification of antibodies to Bifodobacterium longum in a biological sample, comprising:
(a) contacting the sample with a polypeptide according to any of the claims 14 or 15 under conditions suitable for the formation of immune complexes; and
(b) detecting the presence of immune complexes in the sample, in which the detection of immune complexes indicates the presence of Bifodobacterium longum in the sample.

22. A DNA array/chip containing an array of polynucleotides comprising at least a polynucleotide according to any of the claims 1 to 4 or a vector according to any of the claims 6 to 8.

23. A protein array/chip containing an array of polypeptides comprising at least one of the polypeptides according to any of the claims 14 or 15.

24. An immunogenic composition comprising a polypeptide ccording to any of the claims 14 or 15 and a pharmaceutically acceptable carrier.

25. An immunogeneic composition comprising a polypeptide according to any of the claims 14 or 15 and a pharmaceutically acceptable carrier.

26. A screening assay, comprising:
(a) contacting a test compound with a polynucleotide according to any of the claims 1 to 4 or a vector according to any of the claims 6 to 8; and
(b) detecting whether binding occurs.

27. A screening assay, comprising:
(a) contacting a test compound with a polypeptide according to any of the claims 14 or 15; and
(b) detecting whether binding occurs.

28. A kit comprising a container containing a polynucleotide according to any of the claims 1 to 4.

29. The kit according to claim 28, wherein the polynucleotide is a primer or a probe and wherein the kit optionally contains a polymerase and deoxynucleotide triphosphates.

30. A kit comprising a container containing an antibody that immunospecifically binds to a polypeptide according to any of the claims 14 and 15.

31. A computer readable medium having recorded thereon a nucleic acid sequence according to any of the claims 1 to 8.

32. A computer readable medium having recorded thereon a polypeptide sequence according to any of the claims 14 or 15.

33. The computer readable medium according to any of the claims 31 or 33, wherein said medium is selected from the group consisting of:
(a) a floppy disc;
(b) a hard disc;
(c) random access memory (RAM);
(d) read only memory (ROM); and
(e) CD-ROM.

34. A computer-based system for identifying fragments of the Bifidobacterium longum genome comprising the following elements:
(a) a data storage means comprising a nucleic acid sequence according to any of the claims 1 to 4;
(b) search means for comparing a target sequence to the nucleotide sequence of the data storage means of step (a) to identify homologous sequence (s); and
(c) retrieval means for obtaining said homologous sequence (s) of step (b).

35. Lactic acid bacterium, which is Bifidobacterium NCC2705 (CNCM I-2618).

36. Use of the Bifidobacterium according to claim 35, or a culture supernatant thereof, or a metabolite thereof for the preparation of a carrier.

37. The use according to claim 36, wherein the Bifidobacterium is contained in the carrier in an amount of from about 10⁵ cfu / g to about 10¹² cfu / g carrier material.

38. The use according to any of the claims 36 and 37, wherein the carrier is for the prevention and/or treatment of diearrohea brought about by pathogenic bacteria and/or rotaviruses.

39. The use according to any of the claims 36 to 38, wherein the carrier is a food composition selected from milk, yogurt, curd, cheese, fermented milks, milk based fermented products, ice-creams, fermented cereal based products, milk based powders, infant formulae, pet food or a pharmaceutical composition selected from tablets, liquid bacterial suspensions, dried oral supplement, wet oral supplement, dry tube feeding or wet tubefeeding.
